# EUROPEAN PATENT APPLICATION

(11) **EP 2 333 511 A1**
(43) Date of publication of application: **15.06.2011**
(21) Application number: 09809697.7
(22) Date of filing: 03.07.2009
(51) Int. Cl.: G01N 1/00, G01N 1/04, G01N 33/48

(54) **SPECIMEN CONTAINER**

(30) Priority: 29.08.2008 JP 2008221755; 10.03.2009 JP 2009056797
(71) Applicant: Olympus Corporation, Shibuya-ku Tokyo 151-0072 (JP)
(72) Inventor: OKADA, Yoshihiro, Tokyo 192-8512 (JP); TODA, Masaya, Tokyo 192-8512 (JP); KOMIYAMA, Shigeru, Tokyo 192-8512 (JP)
(74) Representative: Schicker, Silvia
(86) International application number: PCT/JP2009/062200
(87) International publication number: WO 2010/024042

(57) **Abstract**

A container for specimen of the present invention comprises: a container body one end of which is open and which can hold a liquid for dispersing or preserving a specimen and can be mounted in a general-purpose apparatus for physical and chemical analysis; a specimen-picking rod; a cover member which connects with the specimen-picking rod; and an intermediary unit 4. The both ends of the intermediary unit are open, and the intermediary unit includes a penetration wall in the middle portion of the intermediary unit. The one open end of the intermediary unit can be removably fitted to the open end of the container body, and the other open end of the intermediary unit can be removably fitted to the cover member. The penetration wall separates a space in the intermediary unit into two divisions on the upper and lower sides in the state of the specimen-picking rod penetrating through a through hole that is formed in the penetration wall. A predetermined specimen can be collected by the specimen-picking rod and the collected specimen which is contained by the container body can be dispersed.

## Description

### Technical Field

This invention relates to a container for specimen with which a specimen can be collected and/or in which a specimen can be preserved.

### Background Art

For example, specimens of human origin, such as stool, mouth mucosa, and saliva, are favorable because it is possible to perform a minimally invasive test using these specimens like urine. These specimens are currently used as a sample in a test. And, a burden to a human body is low in collecting these specimens of human origin, so that these specimens are used for a test and/or diagnosis of disease. For example, a fecal occult blood test is so important as to be necessary for a test and/or diagnosis of digestive system diseases, in particular, such as colorectal cancer or the like.
Also, as far as testing techniques are concerned, automatic inspection apparatuses have been developed in accordance with test items, by the development of apparatus technologies. For example, in fecal occult blood test, it is possible to perform automatic testing and/or automatic measurement after a stool-collecting and -preserving container is set to an inspection apparatus as it is.

In recent years, because of rapid progress in genetic testing techniques, researches and developments for genetic test with specimens of human origin including stool have been conducted for the purpose of making it possible to quickly and simply perform measurement using a small amount of specimen. At present, researches on and developments of techniques for genetic testing methods have progressed and make it possible to extract, purify, and measure nucleic acids using a general-purpose apparatus for physical and chemical analysis. A general-purpose container which can be mounted in the general-purpose apparatus for physical and chemical analysis is used in these operations.

Conventional containers which are used for detecting fecal occult blood in the above-described fecal occult blood test include a stool-collecting and -preserving container which is described in Japanese Utility Model Jitukou No. Hei 05-017652. In fecal occult blood testing, this stool-collecting-and-preserving container containing a stool of specimen can be mounted in an automatic inspection apparatus for fecal occult blood test as it is.

By the way, for example, a genetic test with stool requires a process of extracting nucleic acids from a stool with a general-purpose apparatus for physical and chemical analysis or a nucleic acid-extracting apparatus, like centrifuge.
However, the fecal occult blood-testing container which is described in Japanese Utility Model Jitukou No. Hei 05-017652 cannot be directly mounted in a general-purpose apparatus for physical and chemical analysis or a nucleic acid-extracting apparatus, like centrifuge.
Accordingly, in the case where a collected stool is held by and preserved in the fecal occult blood container which is described in Japanese Utility Model Jitukou No. Hei 05-017652, a process of extracting nucleic acids from the collected stool requires the operation of transferring the collected stool to a highly versatile container which can be mounted in a nucleic acid-extracting apparatus, or the like. However, the transfer of the collected stool to another container is liable to cause: the problem of contamination easily causing; the problem that as a result of the adhesion of an indefinite amount of stool to the original container or a tool with which the collected stool is transferred in transferring the collected stool, or the like, it becomes difficult to quantify the amount of the specimen sample which is removed to the container that is mounted in a nucleic acid-extracting apparatus, within a given permissible range; the problem of the specimen sample confused with another; and so on.

### Disclosure of Invention

The present invention is made in view of such conventional problems. The first object of the present invention is to offer a container for specimen: which can be mounted in a general-purpose apparatus for physical and chemical analysis while the container holds the collected and preserved specimen without transferring the collected and preserved specimen to another container, even in a test requiring extraction of nucleic acids or the like with the general-purpose apparatus for physical and chemical analysis like centrifuge, or the like; and, in addition, which makes it possible to use for a test the amount of a collected specimen which is quantified within a given permissible range.
Also, the second object of the present invention is to offer a container for specimen: which is easy to put into a specimen like stool without applying large force and makes it possible to quantify and collect enough amount of specimen for a test; which can be mounted in a general-purpose apparatus for physical and chemical analysis while the container holds the collected and preserved specimen without transferring the collected and preserved specimen to another container, even in a test requiring extraction of nucleic acids or the like with the general-purpose apparatus for physical and chemical analysis like centrifuge, or the like; and, in addition, which makes it possible to use for a test an enough amount of collected specimen which is quantified within a given permissible range.

In order to achieve the above first object, a container for specimen according to the present invention is characterized in that the container comprises: a container body one end of which is open, and which can hold a liquid for dispersing or preserving a specimen and is formed in the shape of a tube so that the container body can be mounted in a general-purpose apparatus for physical and chemical analysis; a specimen-picking rod for picking a specimen; a cover member which connects with the rear end portion of the specimen-picking rod; and an intermediary unit which connects the container body with the cover member, wherein the intermediary unit includes: a connection portion the both ends of which are open, the one end of which can be removably fitted to the open end of the container body, the other end of which can be removably fitted to the cover member, and which is formed in the shape of a tube; and a penetration wall which is provided on the inside of the connection portion, includes a through hole that can be penetrated by the specimen-picking rod, and separates a space into two divisions on the upper and lower sides in the state of the specimen-picking rod penetrating through the through hole.

Also, in a container for specimen according to the present invention, it is preferred that the penetration wall is made of elastic material.

Also, in a container for specimen according to the present invention, it is preferred that the penetration wall is provided with an elastic member in the vicinity of the through hole.

Also, in a container for specimen according to the present invention, it is preferred that the penetration wall includes an annular groove which is formed on the surface forming the through hole, and an annular elastic member which is fitted to the annular groove and the inside diameter of which is smaller than the diameter of the through hole.

Also, in a container for specimen according to the present invention, it is preferred that the penetration wall is provided with an elastic member which is given a radial slash that passes through the center of the through hole, covers the through hole, and deforms to come into close contact with the specimen-picking rod when the specimen-picking rod is inserted into the through hole.

Also, in a container for specimen according to the present invention, it is preferred that the penetration wall is provided with a plurality of annular grooves which are formed on the surface forming the through hole and along the direction of the penetration.

Also, in a container for specimen according to the present invention, it is preferred that the cross sectional shapes of a plurality of the annular grooves which are formed on the surface forming the through hole of the penetration wall are formed in the shape of one of the letter "U", the letter "V", and an arc.

Also, in a container for specimen according to the present invention, it is preferred that the specimen-picking rod includes a concave picking portion in the front end of the specimen-picking rod and the container body includes a convex removing portion which is placed on the bottom of the container body and can be inserted into the concave picking portion.

Also, in a container for specimen according to the present invention, it is preferred that the concave picking portion and the convex removing portion are formed in such a way that the concave picking portion and the convex removing portion can be fitted to each other and be separated from each other from the state of the concave picking portion fitted to the convex removing portion.

Also, in a container for specimen according to the present invention, it is preferred that the specimen-picking rod includes a path which links the concave picking portion and the external peripheral surface of the specimen-picking rod and a specimen passes through the path to be extruded to the outside when the convex removing portion is inserted into the concave picking portion.

Also, in a container for specimen according to the present invention, it is preferred that the specimen-picking rod includes a plurality of slits which link the lateral side of the concave picking portion and the external peripheral surface of the specimen-picking rod.

Also, in a container for specimen according to the present invention, it is preferred that the specimen-picking rod includes a plurality of extrusion holes which link the bottom surface of the concave picking portion and the external peripheral surface of the specimen-picking rod.

Also, in a container for specimen according to the present invention, it is preferred that the specimen-picking rod includes a picking portion which is placed on the external peripheral surface of the front end of the specimen-picking rod and is composed of a lattice-shaped groove.

Also, in a container for specimen according to the present invention, it is preferred that the specimen-picking rod includes a picking portion which is placed at the front end of the specimen-picking rod and is shaped like a brush.

Also, in a container for specimen according to the present invention, it is preferred that the container for specimen includes a connection means which connects the intermediary unit with the convex picking portion.

Also, in a container for specimen according to the present invention, it is preferred that the connection means includes: an engaging member which is fixed on the inside of the connection portion; and a frame member which is formed in the shape of a tube so as to be capable of being held inside the container body, the bottom portion of which the convex picking portion is fixed on, and the upper portion of which can engage with the engaging member.

Also, in a container for specimen according to the present invention, it is preferred that the specimen-picking rod includes: an absorbing portion which is placed on the front end of the specimen-picking rod and is spongy; a tube member which connects with the absorbing portion; and a rod member which moves inside the tube member, the front end portion of which can push the absorbing portion, and the other end of which connects with the cover member.

Also, in a container for specimen according to the present invention, it is preferred that threads are formed in the internal peripheral portion of the one end of the connection portion in the intermediary unit and in the external peripheral portion of the open end of the container body and the open end of the container body is screwed into the one end of the connection portion of the intermediary unit through the threads.

Also, in a container for specimen according to the present invention, it is preferred that the external peripheral portion of the one end of the connection portion in the intermediary unit is provided with: at least one annular convex portion which is formed along the penetration direction; an annular stopper portion the diameter of which is larger than the internal peripheral portion of the open end of the container body; and an O-ring which is placed on the container-body side of the stopper member, and the internal peripheral portion of the open end of the container body is provided with an annular concave portion which can be fitted to the annular convex portion.

Also, in a container for specimen according to the present invention, it is preferred that the edge portion of the one end of the connection portion in the intermediary unit is provided with: an annular groove which is formed so as to be capable of being fitted to the open end of the container body; an O-ring which is placed on the bottom of the annular groove; and a plurality of opening portions which are provided on the circumference of the connection portion of the intermediary unit so as to link the annular groove and the external peripheral portion of the one end of the connection portion of the intermediary unit, and the external peripheral portion of the open end of the container body is provided with a plurality of catch portions which can be inserted into the opening portions of the connection portion of the intermediary unit and are formed in such a way that the catch portions break off at the bases when the catch portions are inserted into the opening portions and force is applied in a set direction crossing the insertion direction.

Also, in a container for specimen according to the present invention, it is preferred that the edge portion of the one end of the connection portion in the intermediary unit is provided with: an annular groove which is formed so as to be capable of being fitted to the open end of the container body; an O-ring which is placed on the bottom of the annular groove; and a plurality of opening portions which are provided on the circumference of the connection portion of the intermediary unit so as to link the annular groove and the external peripheral portion of the one end of the connection portion of the intermediary unit, and the external peripheral portion of the open end of the container body is provided with a plurality of catch portions which can be inserted into the opening portions of the connection portion of the intermediary unit and are formed in such a way that the catch portions have sloped surfaces so that the catch portions come out of the opening portions when force is applied so that the intermediary unit is moved in the direction opposite to the direction toward the container body with the catch portions inserted into the opening portions.

Also, in a container for specimen according to the present invention, it is preferred that the connection portion of the intermediary unit, the cover member, and the container body are made of elastic material, the elastic modulus of the connection portion of the intermediary unit is smaller than those of the penetration wall and the cover member and is larger than that of the container body, and the elastic modulus of the cover member is smaller than that of the penetration wall.

Also, in a container for specimen according to the present invention, it is preferred that the connection portion of the intermediary unit, the cover member, and the container body are made of elastic material, and the elastic modulus of the connection portion of the intermediary unit is smaller than that of the penetration wall and is larger than those of the cover member and the container body.

Also, in a container for specimen according to the present invention, it is preferred that threads are provided on the external peripheral portion of the other end of the connection portion of the intermediary unit and on the internal peripheral portion of the cover member, and the other end of the connection portion of the intermediary unit is screwed into the container body through the threads.

Also, in a container for specimen according to the present invention, it is preferred that torque by which the other end of the connection portion of the intermediary unit is screwed into the cover member is smaller than torque by which the open end of the container body is screwed into the one end of the connection portion of the intermediary unit.

Also, in a container for specimen according to the present invention, it is preferred that the specimen-picking rod is formed so that the specimen-picking rod can be removably fitted to the cover member.

Also, in a container for specimen according to the present invention, it is preferred that the threads are formed in the internal peripheral portion of the one end of the connection portion of the intermediary unit, in the external peripheral portion of the open end of the container body, in the external peripheral portion of the other end of the connection portion of the intermediary unit, and in the internal peripheral portion of the cover member respectively in such a way that the opening and closing directions in rotating the intermediary unit in a set direction through the threads that are formed in the internal peripheral portion of the one end of the connection portion of the intermediary unit and in the external peripheral portion of the open end of the container body are opposite to the opening and closing directions in rotating the intermediary unit in a set direction through the threads that are formed in the external peripheral potion of the other end of the connection portion of the intermediary unit and in the internal peripheral portion of the cover member, respectively.

Also, in a container for specimen according to the present invention, it is preferred that the cover member is provided with a grasping portion.
Besides, in the present invention, the grasping portion means a portion which is formed so as to be capable of being grasped in order to make the cover member easy to rotate. For example, it is preferred that the grasping portion is formed in the shape of a rectangle or polygon, in the D-Cut shape, or the like so that at least a part of the cover member has an approximately plane surface, or is formed in the shape by which at least a part of the cover member has a convex or concave portion.

Also, in a container for specimen according to the present invention, it is preferred that the outer diameter of the cover member is larger than that of the connection portion of the intermediary unit and the outer diameter of the connection portion of the intermediary unit is larger than that of the container body.

Also, in a container for specimen according to the present invention, it is preferred that the cover member is different from the connection portion of the intermediary unit in material.

Also, in a container for specimen according to the present invention, it is preferred that the container body is different from the connection portion of the intermediary unit in material.

Also, in a container for specimen according to the present invention, it is preferred that: the specimen-picking rod includes a separation portion which can open and close at least in the front end of the specimen-picking rod and round almost one part of the specimen-picking rod and which holds a set amount of specimen when the separation portion is closed; and the specimen-picking rod slides on the through hole to penetrate through the through hole while the separation portion is closed.

Also, a container for specimen according to the present invention is characterized in that the container comprises: a container body one end of which is open, and which can hold a liquid for dispersing or preserving a specimen and is formed in the shape of a tube; a specimen-picking rod for picking a specimen; a cover member which connects with the rear end portion of the specimen-picking rod; and an intermediary unit which connects the container body with the cover member, wherein the intermediary unit includes: a connection portion the both ends of which are open, one end of which can be removably fitted to the open end of the container body, the other end of which can be removably fitted to the cover member, and which is formed in the shape of a tube; and a penetration wall which is provided on the inside of the connection portion, includes a through hole that can be penetrated by the specimen-picking rod, and separates a space into two divisions on the upper and lower sides in the state of the specimen-picking rod penetrating through the through hole, the specimen-picking rod includes a forked portion which can open and close at least in the front end of the specimen-picking rod and which holds a set amount of a specimen with the specimen fragmented when the fork portion is closed, and the specimen-picking rod slides on the through hole to penetrate through the through hole while the fork portion is closed.

Also, in a container for specimen according to the present invention, it is preferred that at least one branching portion of the forked portion includes at least one specimen-holding portion which is provided with: a specimen-introducing inlet that is formed on the side on which the one branching portion touches the other branching portion of the forked portion when the forked portion is closed; and a specimen-exhausting outlet that is formed on the side opposite to the side on which the specimen-introducing inlet is formed.

Also, in a container for specimen according to the present invention, it is preferred that the front end of one branching portion of the forked portion includes at least one groove which is formed by placing at least two thin plate-shaped plane portions along the direction of the forked portion opening or closing, and the front end of the other branching portion of the forked portion includes a shield portion which is composed of a thin plate-shaped plane portion that protrudes toward the one-branching-portion side so that the shield portion covers the opening side of the groove along the direction of the forked portion opening or closing when the forked portion is closed.

Also, in a container for specimen according to the present invention, it is preferred that: the front end of one branching portion of the forked portion includes at least one groove which is formed by placing at least two thin plate-shaped plane portions along the direction of the forked portion opening or closing; the other branching portion of the forked portion includes a specimen-holding portion which is provided with a specimen-introducing inlet that is formed on the side on which the one branching portion touches the other branching portion of the forked portion when the forked portion is closed and a specimen-exhausting outlet that is formed on the side opposite to the side on which the specimen-introducing inlet is formed; and the front end of the other branching portion of the forked portion includes a shield portion which is composed of a thin plate-shaped plane portion that protrudes toward the one-branching-portion side so that the shield portion covers the opening side of the groove along the direction of the forked portion opening or closing when the forked portion is closed.

Also, in a container for specimen according to the present invention, it is preferred that, in the at least one groove, at least one of the thin plate-shaped plane portions which form the groove protrudes toward the the-other-branching-portion side so as to enter the specimen-holding portion when the forked portion is closed.

Also, in a container for specimen according to the present invention, it is preferred that the thin plate-shaped plane portions which form the groove become thinner and thinner towards the side on which the one branching portion of the forked portion touches the other branching portion of the forked portion when the forked portion is closed.

Also, in a container for specimen according to the present invention, it is preferred that the forked portion of the specimen-picking rod is formed so as to be opened with elastic force biased, and the specimen-picking rod is provided with an annular member which is placed on the outer circumference of the specimen-picking rod, can move in the longitudinal direction, is moved towards the front end by a predetermined distance to make the forked portion close, and is moved towards the rear end by a predetermined distance to release the forked portion that is in a closed state.

Also, in a container for specimen according to the present invention, it is preferred that the specimen-picking rod includes an annular member-fixing means for fixing the annular member at a predetermined position at which the forked portion is opened.

Also, in a container for specimen according to the present invention, it is preferred that: the specimen-picking rod includes a flange at the rear end of the specimen-picking rod; the trunk portion of the specimen-picking rod which runs from the base of the forked portion to the rear end is formed as a hollow part and, in addition, the specimen-picking rod includes a pair of guide grooves including a groove with a T-shaped cross section which is provided for each of the branching portions approximately along the longitudinal direction of the specimen-picking rod on the side on which the one branching portion of the forked portion touches the other branching portion of the forked portion in closing the forked portion and a second groove which continues to the one edge of the T-shaped groove; the cover member includes a holding portion by which the flange at the rear end of the specimen-picking rod is rotatably held, a shaft-shaped male screw which is fixed in the middle of the cover member and runs towards the front end of the specimen-picking rod with the male screw having a predetermined length, a female screw into which the male screw is screwed and the diameter of which has a size that enables the second grooves of the both guide grooves to hold the female screw when the male screw is screwed into the female screw up to a predetermined position in the male screw and the forked portion is closed, and T-shaped portions which are provided on the external peripheral surface of the female screw with the T-shaped portions opposite to each other and are freely fitted to the T-shaped grooves of the guide grooves in the forked portion respectively; and the rotation of the cover member relative to the specimen-picking rod makes the forked portion open and close.

Also, in a container for specimen according to the present invention, it is preferred that the pair of the guide grooves is provided so as to incline relative to the longitudinal direction of the specimen-picking rod so that the T-shaped grooves and the bottom surfaces of the second grooves become shallower and shallower towards the base of the forked portion.

Also, in a container for specimen according to the present invention, it is preferred that: the specimen-picking rod is connected with at least one headed pin-shaped connection member at the middle portion of the specimen-picking rod and can open and close with the shaft of the headed pin-shaped connection member as a fulcrum; the portion of the specimen-picking rod which runs from the front end of the specimen-picking rod to the fulcrum is composed of two members which form the forked portion, a first member of the two members includes a first nick portion which runs from the rear end to the vicinity of the fulcrum, and a second member of the two members includes a second nick portion which runs from the rear end to the vicinity of the fulcrum and into which the portion of the first member running from the rear end of the first member to the vicinity of the fulcrum can be slidably fitted; and the cover member includes a shaft-shaped supporting member which is fixed in the middle of the cover member, has a diameter and a length that enable the supporting portion to be slidably fitted into the first nick portion of the first member, and includes an annular groove that can hold the head of the headed pin-shaped connection member connecting the two members in the circumferential direction, and a swirl groove which is formed in the internal surface of the cover member in such a way that the rear ends of the two members are slidably fitted to the swirl groove and is formed in the shape of a swirl converging to the center of the cover member.

Also, in a container for specimen according to the present invention, it is preferred that the internal surface of the cover member which is provided with the swirl groove is formed in the shape of a cone.

Also, in a container for specimen according to the present invention, it is preferred that: the internal surface of the cover member is formed in the shape of a cone and is provided with a groove which is formed in the shape of a spiral converging to the center of the cover member and has a T-shaped cross section; the specimen-picking rod is connected with at least one headed pin-shaped connection member at the middle portion of the specimen-picking rod and can open and close with the shaft of the headed pin-shaped connection member as a fulcrum; and the portion of the specimen-picking rod which runs from the front end of the specimen-picking rod to the fulcrum is composed of two members which form the forked portion, the two members are formed in the shapes that enable the two members to overlap with each other over the range from the rear end to the vicinity of the fulcrum, the inside surfaces of the forked portion are formed in the shapes that enable the inside surfaces of the forked portion to abut each other, and the rear ends of the two members are provided with T-shaped portions which are freely fitted to the T-shaped groove of the cover member; and the rotation of the cover member relative to the specimen-picking rod makes the forked portion open and close.

Also, a container for specimen according to the present invention is characterized in that the container comprises: a container body one end of which is open, and which can hold a liquid for dispersing or preserving a specimen and is formed in the shape of a tube; a specimen-picking rod for picking a specimen; a cover member which connects with the rear end portion of the specimen-picking rod; and an intermediary unit which connects the container body with the cover member, wherein the intermediary unit includes: a connection portion the both ends of which are open, one end of which can be removably fitted to the open end of the container body, the other end of which can be removably fitted to the cover member, and which is formed in the shape of a tube; and a penetration wall which is provided on the inside of the connection portion, includes a through hole that can be penetrated by the specimen-picking rod, and separates a space into two divisions on the upper and lower sides in the state of the specimen-picking rod penetrating through the through hole, the specimen-picking rod includes a plurality of vertical divisions which can open and close at least on the front end of the specimen-picking rod and hold a set amount of specimen when the vertical divisions are in a closed state, a plurality of the vertical divisions are formed in such a way that at least a part of each of the vertical divisions expands towards the outside more and more towards the front end with the parts of the vertical divisions curving, and the specimen-picking rod slides on the through hole to penetrate through the through hole with a plurality of the vertical divisions closed.

Also, in a container for specimen according to the present invention, it is preferred that the front end of each of a plurality of the vertical divisions bends inward so that the vertical divisions can hold a set amount of specimen when the vertical divisions are in a closed state.

Also, in a container for specimen according to the present invention, it is preferred that the specimen-picking rod includes a tube portion which connects with the cover member and a cylinder portion which is inserted into the tube portion, and a plurality of the vertical divisions are placed on the front end of the cylinder portion respectively and are formed so as to expand towards the outside more and more towards the front end with the vertical divisions curving when the vertical divisions protrude from the tube portion towards the outside.

Also, in a container for specimen according to the present invention, it is preferred that the specimen-picking rod is formed in such a way that a plurality of the vertical divisions can slide with the air-tightness kept when the vertical divisions are contained in the tube portion.

Also, in a container for specimen according to the present invention, it is preferred that the specimen-picking rod includes a reagent-holding portion which is placed in the cylinder portion.

Also, in a container for specimen according to the present invention, it is preferred that the cylinder portion is composed of a rear end portion, a front end portion the front end of which is provided with a plurality of the vertical divisions, and a support portion which connects the rear end portion and the front end portion.

Also, in a container for specimen according to the present invention, it is preferred that: the support portion is formed in such a way that the support portion has a small diameter; the reagent-holding portion is formed by the space which is surrounded by the tube portion, the rear end portion of the cylinder portion, the support portion of the cylinder portion, and the front end portion of the cylinder portion; the front end portion of the cylinder portion includes a plurality of the vertical divisions and a base portion which holds a plurality of the vertical divisions; and the base portion plays a role as a stopper for the reagent-holding portion for the tube portion.

Also, in a container for specimen according to the present invention, it is preferred that the support portion is formed as a hollow part, the reagent-holding portion is formed by the space which is surrounded by the rear end portion of the cylinder portion, the hollow connection portion of the cylinder portion, and the front end portion of the cylinder portion, and a part of the hollow connection portion is provided with an opening for exhausting a reagent.

Also, in a container for specimen according to the present invention, it is preferred that the rear end portion of the cylinder portion is provided with a stopper portion which is formed in such a way that the stopper portion restrains the movement of the cylinder portion towards the front end in the tube portion in order to keep the air-tightness of the reagent-holding portion and the restraint of the movement of the cylinder portion can be released by applying a predetermined amount or more of pressing force to the stopper portion towards the front end.

Also, in a container for specimen according to the present invention, it is preferred that: the container for specimen includes a removable outer cover which is placed on the outside of the intermediary unit and on the cover-member side with the intermediary unit connecting the container body with the cover member; the intermediary unit includes a first thread portion which is provided on the external peripheral portion of the intermediary unit on the cover-member side and can be threadedly engaged with the outer cover; and the outer cover includes a second thread portion which is provided on the internal peripheral portion of the outer cover and can be threadedly engaged with the intermediary unit, and a restraint-releasing portion which applies a predetermined amount or more of pressing force to the stopper portion to release restraint of the movement of the cylinder portion towards the front end in the tube portion by the stopper portion when the second thread portion of the outer cover is threadedly engaged with the first thread portion of the intermediary unit.

According to the present invention, it is possible to obtain a container for specimen which: can be attached to a general-purpose apparatus for physical or chemical analysis as it is without transferring a collected and preserved specimen to another container even in a test that requires extracting a nucleic acid or the like using general-purpose apparatuses for physical or chemical analysis such as centrifuge; and makes it possible to use for test a specimen the collection amount of which is quantified within a predetermined acceptable range.
Also according to the present invention, it is possible to obtain a container for specimen which: can be easily inserted into specimens such as stool without applying large force; makes it possible to quantify and collect a sufficient amount of a specimen for test; can be attached to a general-purpose apparatus for physical or chemical analysis as it is without transferring a collected and preserved specimen to another container even in a test that requires extracting a nucleic acid or the like using general-purpose apparatuses for physical or chemical analysis such as centrifuge; and makes it possible to use for test a specimen the sufficient collection amount of which is quantified within a predetermined acceptable range.

### Brief Description of Drawings

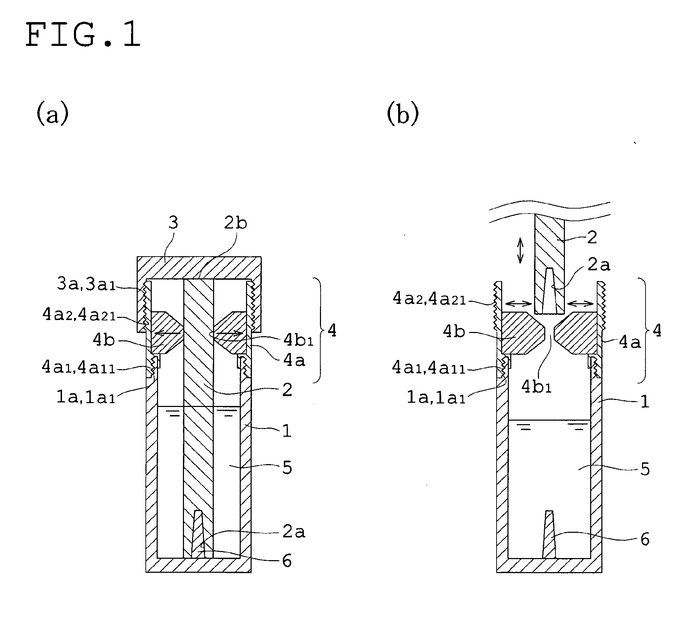

[Fig. 1] Fig. 1 is a view schematically showing the constitution of a container for specimen according to a first embodiment of the present invention, (a) is a sectional view showing a state in which the cover member connecting with the specimen-picking rod is connected with the container body through the intermediary unit, and (b) is a sectional view showing a state in which the specimen-picking rod is separated from the intermediary unit that connects with the container body.

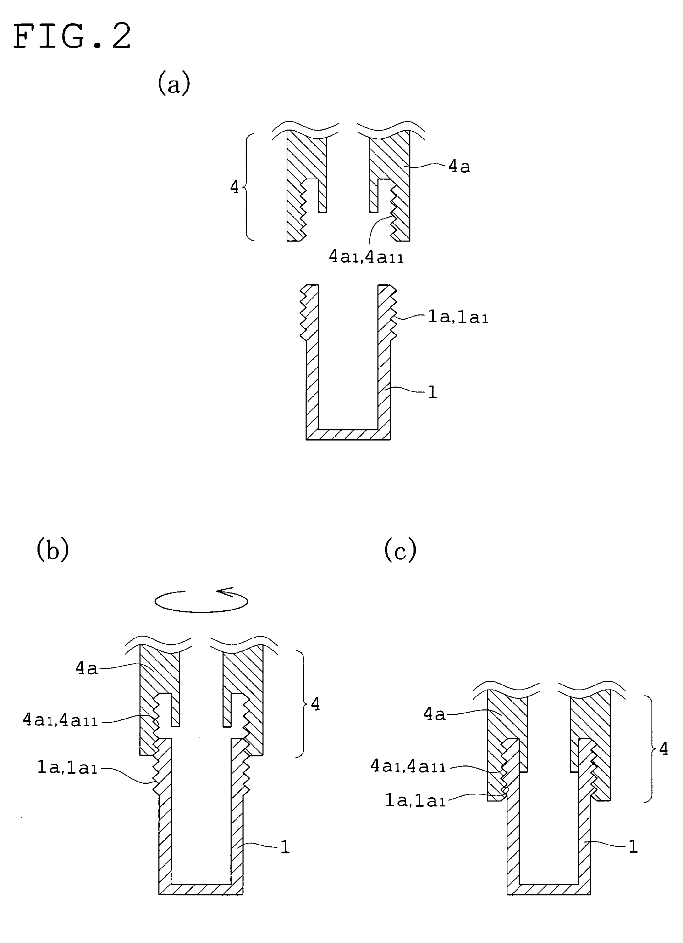

[Fig. 2] Fig. 2 is a view showing one example of the constitution of a main part of the container for specimen which is shown in Fig. 1, (a) is a view showing a state in which the connection portion of the intermediary unit is separated from the container body, (b) is a view showing a state of the middle of fitting the connection portion of the intermediary unit to the container body, and (c) is a view showing a state in which the intermediary unit is completely fitted to the container body.

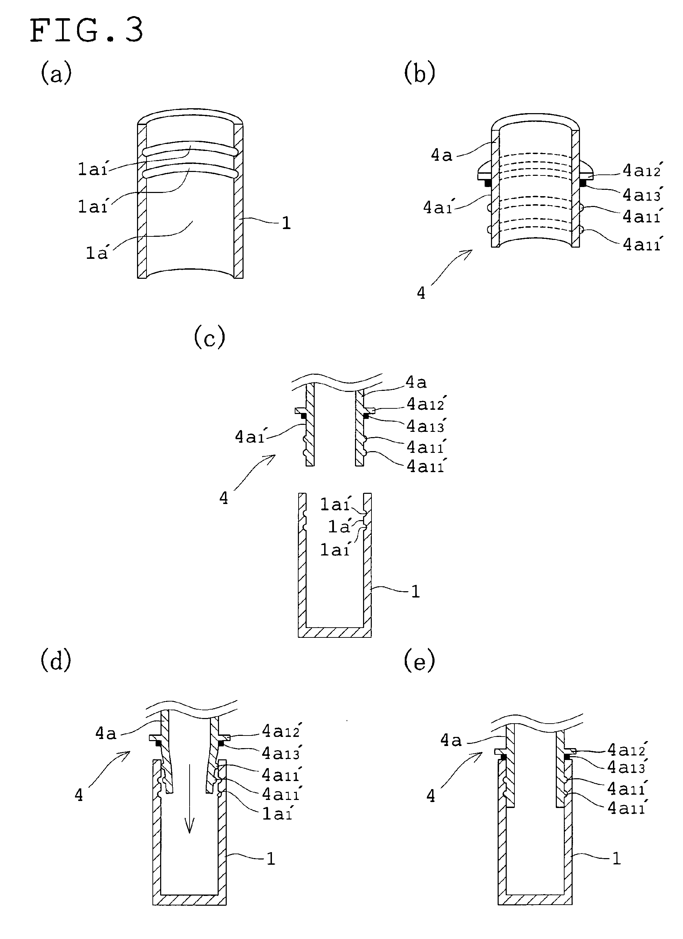

[Fig. 3]Fig. 3 is an explanatory view showing one example of the constitution of main parts of a container for specimen according to a second embodiment of the present invention, (a) is an explanatory view showing the main part in the container body which is cut along the longitudinal direction of the container body, (b) is an explanatory view showing the main part in the intermediary unit which is cut along the longitudinal direction of the intermediary unit, (c) is a sectional view showing a state in which the connection portion of the intermediary unit is separated from the container body, (d) is a sectional view showing a state of the middle of fitting the intermediary unit to the container body, and (e) is a sectional view showing a state in which the intermediary unit is completely fitted to the container body.

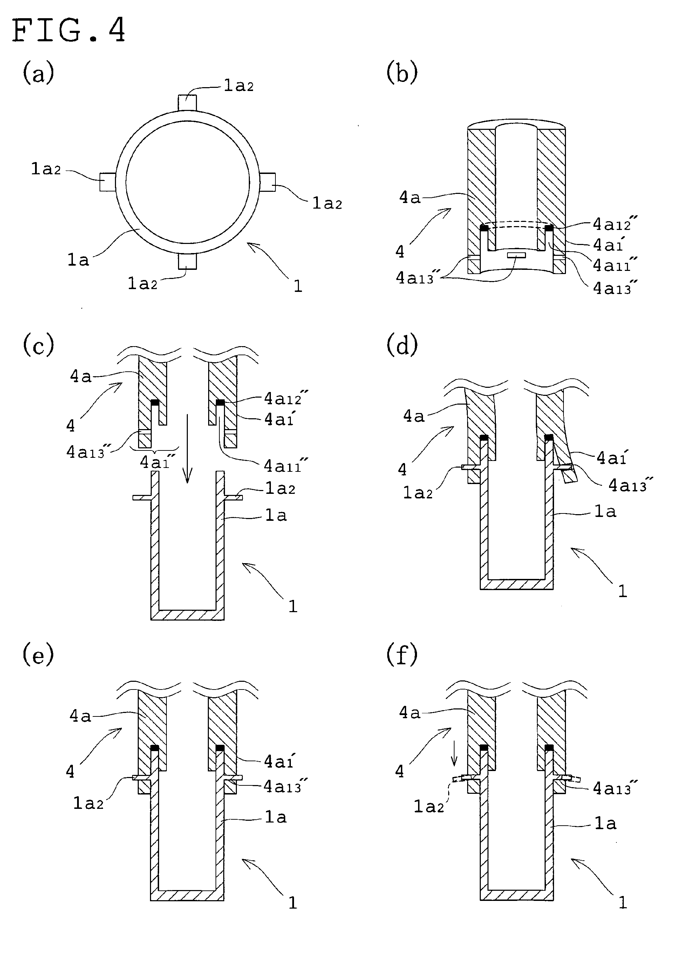

[Fig. 4] Fig. 4 is an explanatory view showing one example of variations of main parts of the container for specimen according to the second embodiment of the present invention, (a) is a top view of the main part in the container body, (b) is an explanatory view showing the main part in the intermediary unit which is cut along the longitudinal direction of the intermediary unit, (c) is a sectional view showing a state in which the intermediary unit is separated from the container body, (d) is a sectional view showing a state of the middle of fitting the intermediary unit to the container body, (e) is a sectional view showing a state in which the intermediary unit is completely fitted to the container body, and (f) is an explanatory view showing a state of the middle of removing the intermediary unit from the container body.

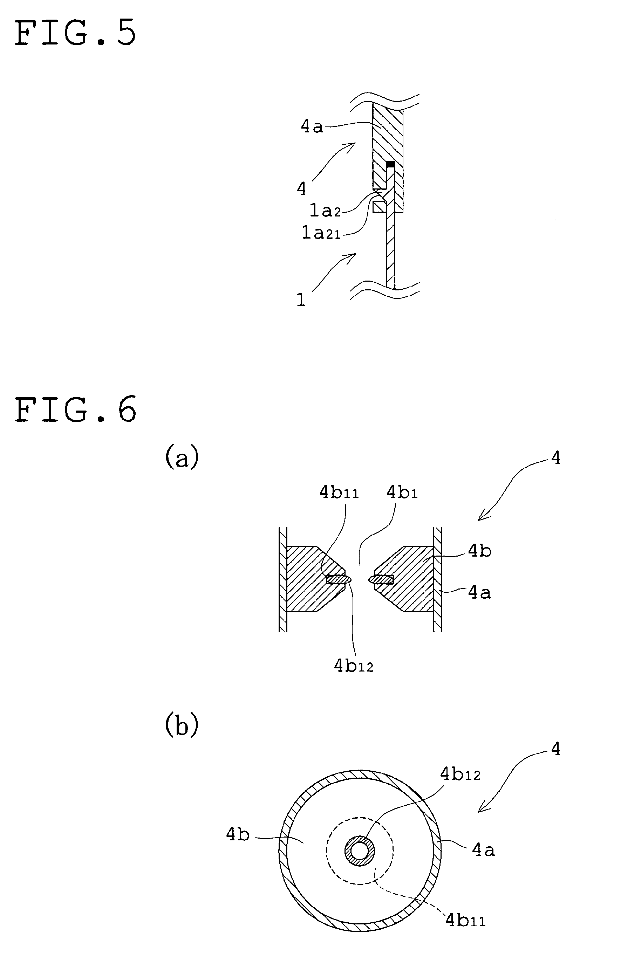

[Fig. 5] Fig. 5 is an explanatory view showing another example of variations of a main part of the container for specimen according to the second embodiment of the present invention and showing the cross section of the shape of the main part of the container body which is fitted to the intermediary unit.
[Fig. 6] Fig. 6 is a view showing the constitution of main parts of a container for specimen according to a third embodiment of the present invention, (a) is a sectional view showing the constitution of the penetration wall of the intermediary unit, and (b) is a top view of the intermediary unit.

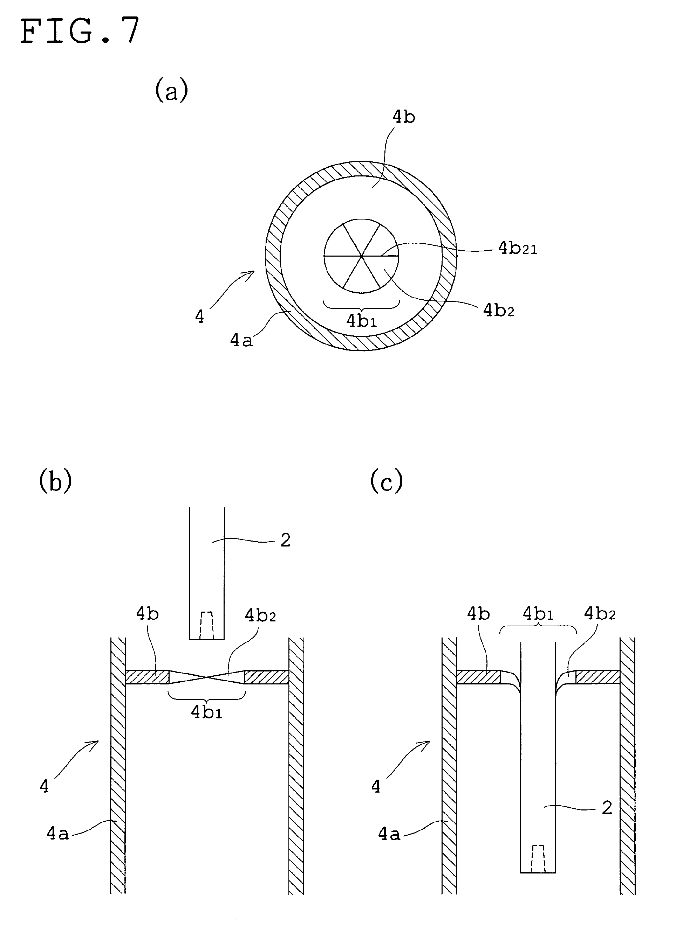

[Fig. 7] Fig. 7 is a view showing the constitution of main parts of a container for specimen according to a fourth embodiment of the present invention, (a) is a top view of the intermediary unit, (b) is a sectional view showing the constitution of the penetration wall of the intermediary unit, and (c) is a sectional view showing a state in which the specimen-picking rod penetrates through the penetration wall of the intermediary unit.

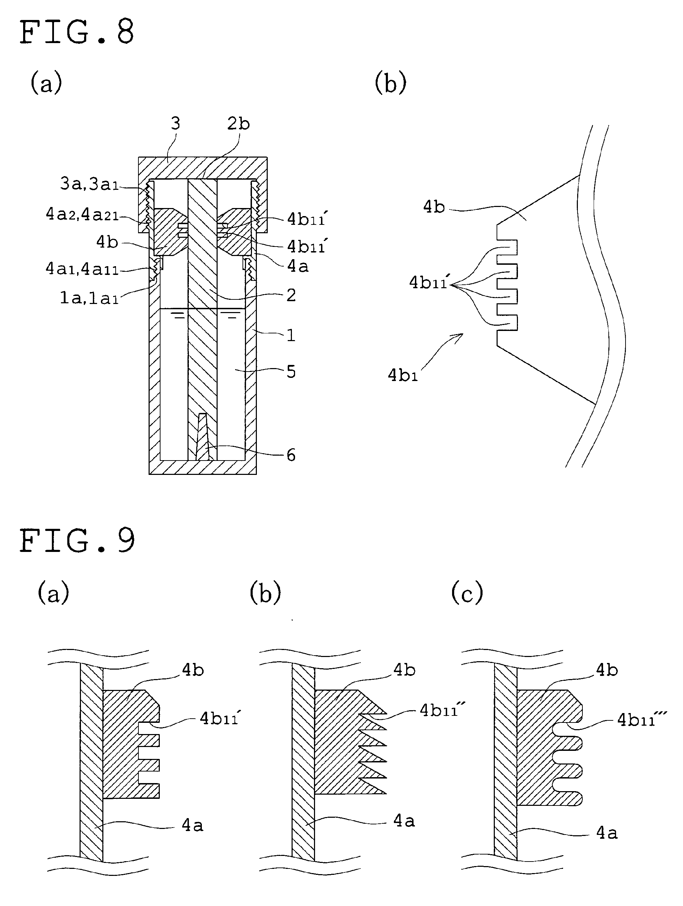

[Fig. 8] Fig. 8 is a view showing the constitution of a container for specimen according to a fifth embodiment of the present invention, (a) is a sectional view showing a state in which the cover member connecting with the specimen-picking rod is connected with the container body through the intermediary unit, and (b) is a partially enlarged sectional view of the penetration wall which is a main part show in (a).
[Fig. 9] Fig. 9 is an explanatory view showing examples of the formation of a cross sectional shape of a groove which is formed on the penetration wall in the container for specimen according to the fifth embodiment of the present invention, (a) is a partial sectional view showing a formation example of the shape of a groove which has the same cross sectional shape as the grooves shown in Fig. 8, (b) is a partial sectional view showing another formation example of the shape of a groove formed on the penetration wall, and (c) is a partial sectional view showing yet another formation example of the shape of a groove formed on the penetration wall.

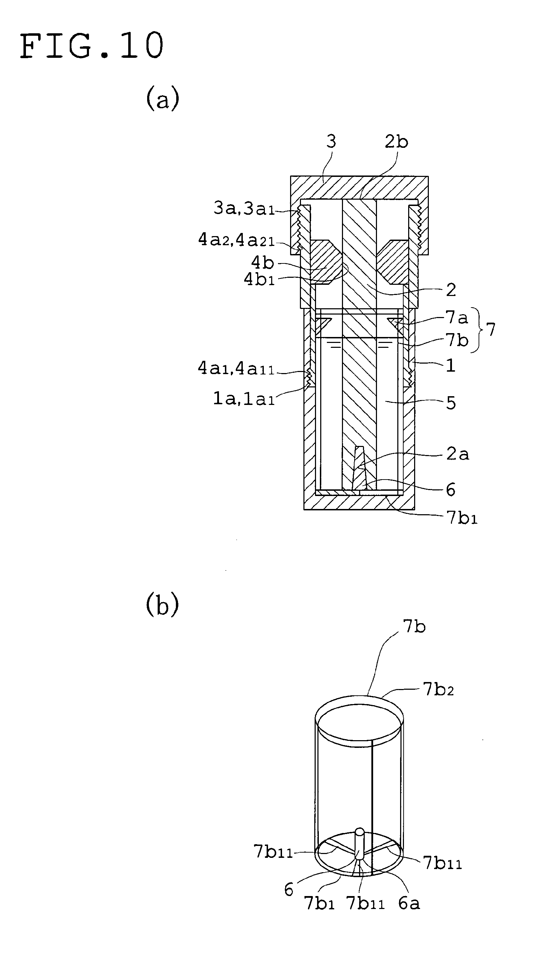

[Fig. 10] Fig. 10 is a view showing the constitution of a container for specimen according to a sixth embodiment of the present invention, (a) is a sectional view showing a state in which the cover member connecting with the specimen-picking rod is connected with the container body through the intermediary unit, and (b) is a perspective view showing the constitution of a main part of the container for specimen of the present embodiment.

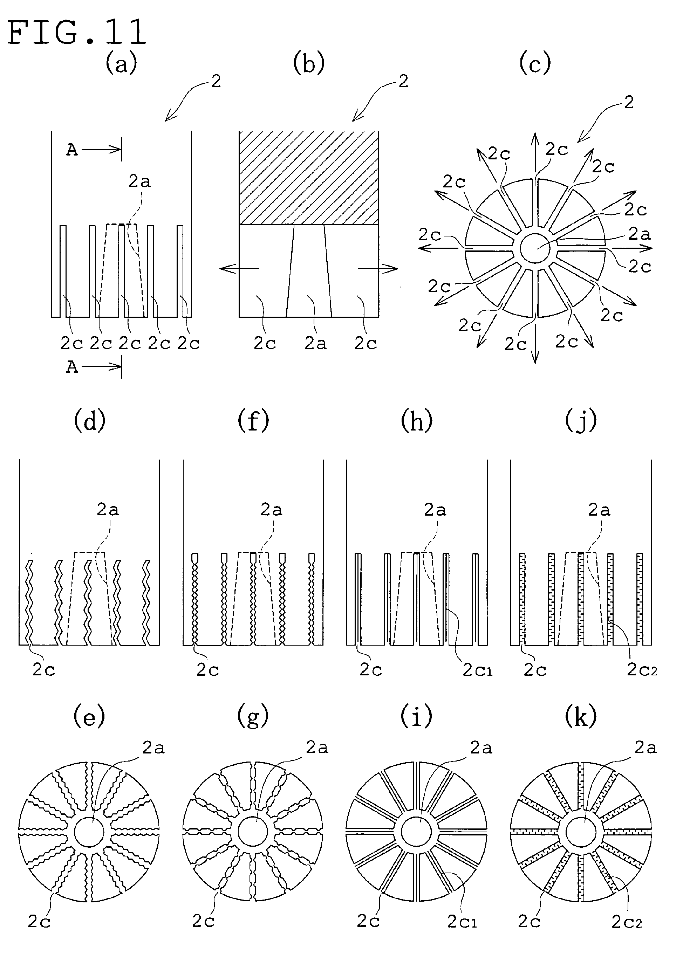

[Fig. 11] Fig. 11 is a fragmentary explanatory view of a main part in one example of the container for specimen according to a seventh embodiment of the present invention, (a) is a view showing one example of the front end portion of the specimen-picking rod, (b) is a cross sectional view along a line A-A of (a), (c) is a view in which the specimen-picking rod shown in (a) is viewed from the front-end side, (d) is a view showing another example of the front end portion of the specimen-picking rod, (e) is a view in which the specimen-picking rod show in (d) is viewed from the front-end side, (f) is a view showing yet another example of the front end portion of the specimen-picking rod, (g) is a view in which the specimen-picking rod shown in (f) is viewed from the front-end-portion side, (h) is a view showing yet another example of the front end portion of the specimen-picking rod, (i) is a view in which the specimen-picking rod shown in (h) is viewed from the front-end side, (j) is a view showing yet another example of the front end portion of the specimen-picking rod, and (k) is a view in which the specimen-picking rod shown in (j) is viewed from the front-end side.

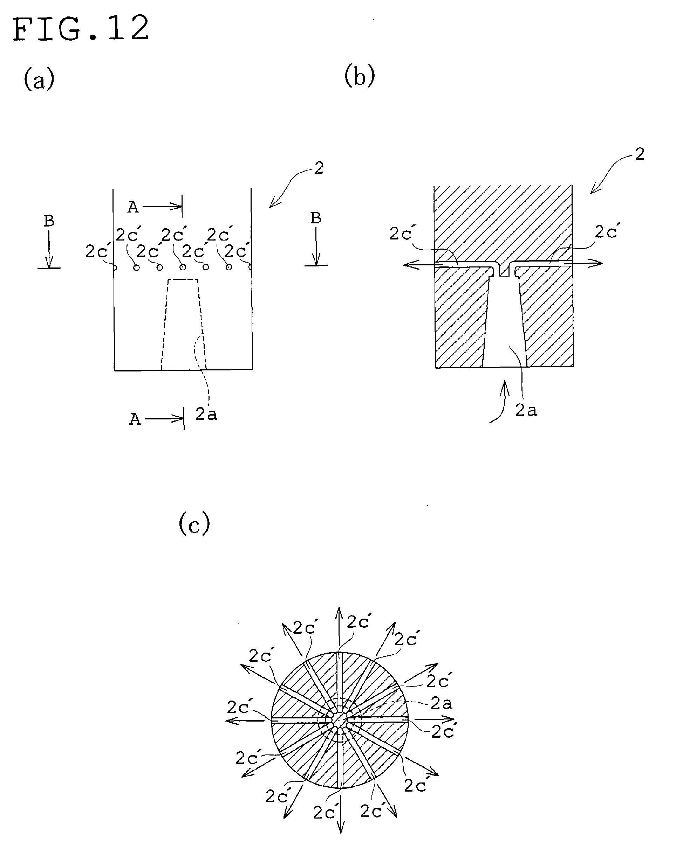

[Fig. 12] Fig. 12 is a fragmentary explanatory view showing the main part in another example of the container for specimen according to the seventh embodiment of the present invention, (a) is a view showing the front end portion of the specimen-picking rod, (b) is a cross sectional view along with the line A-A of (a), and (c) is a cross sectional along a line B-B view of (a).

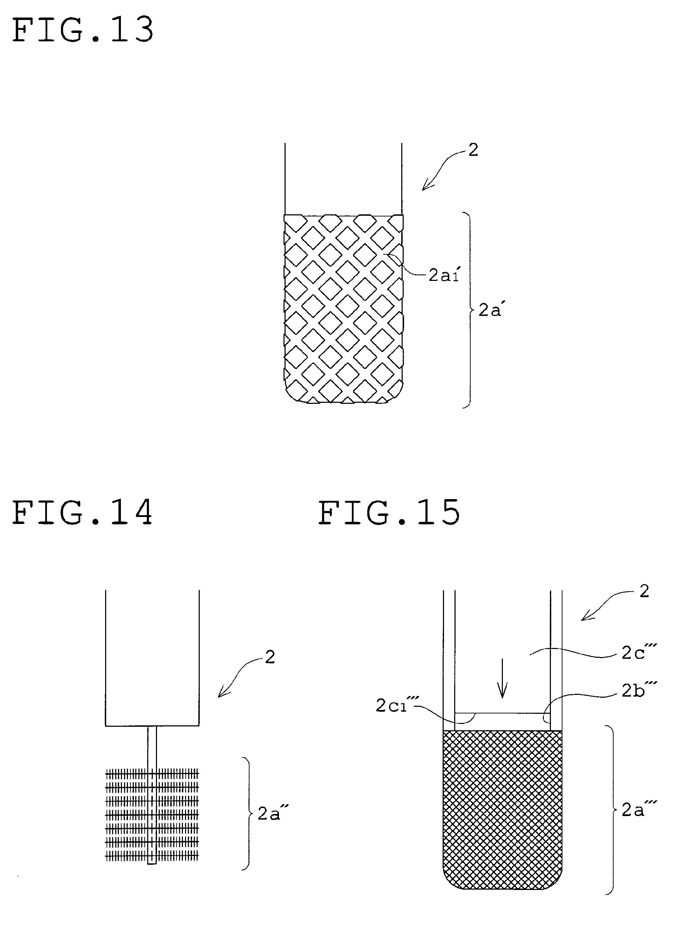

[Fig. 13] Fig. 13 is a fragmentary explanatory view showing a main part in one example of a container for specimen according to an eighth embodiment of the present invention and is a view showing the front end portion of the specimen-picking rod.
[Fig. 14] Fig. 14 is a fragmentary explanatory view showing a main part in another example of the container for specimen according to the eighth embodiment of the present invention and is a view showing the front end portion of the specimen-picking rod.
[Fig. 15] Fig. 15 is a fragmentary explanatory view showing a main part in a container for specimen according to a ninth embodiment of the present invention and is a view showing the front end portion of the specimen-picking rod.

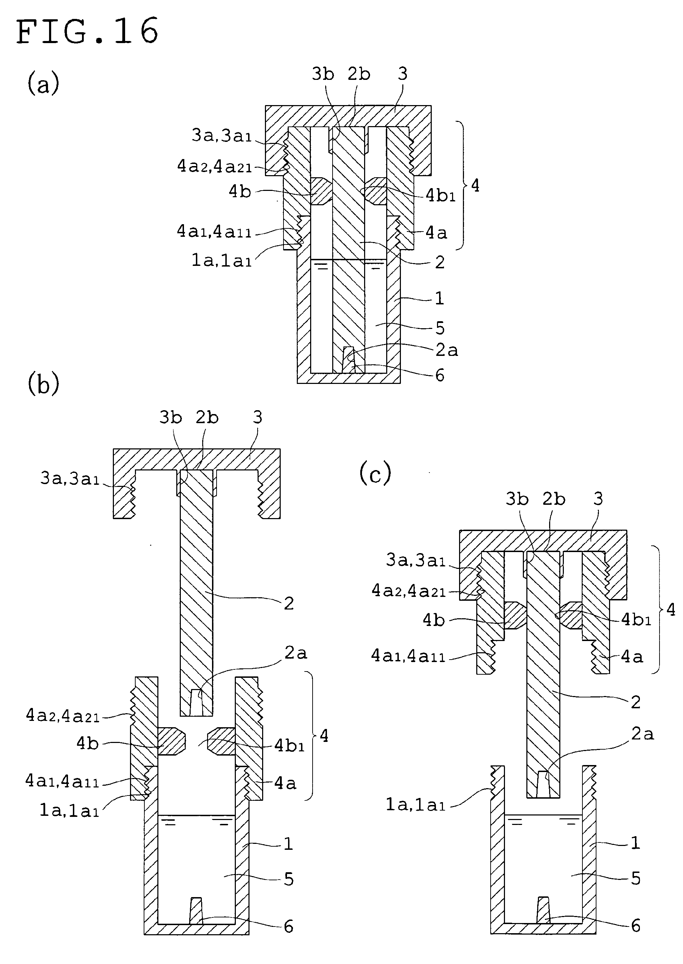

[Fig. 16] Fig. 16 is an explanatory view schematically showing the constitution of a container for specimen according to a tenth embodiment of the present invention, (a) is a sectional view showing a state in which the cover member connecting with the specimen-picking rod is connected with the container body through the intermediary unit, (b) is a sectional view showing a state in which the specimen-picking rod is separated from the intermediary unit connecting with the container body, and (c) is a sectional view showing a state in which the intermediary unit is separated from the container body with the intermediary unit connecting with the cover member that connects with the specimen-picking rod.

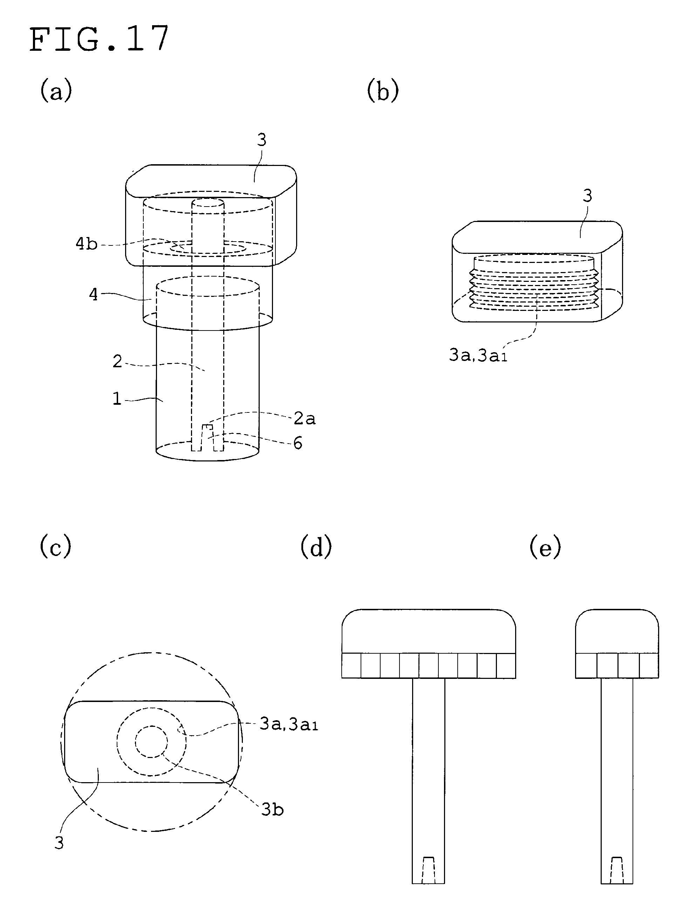

[Fig. 17] Fig. 17 is an explanatory view showing the constitution of the cover member of the container for specimen shown in Fig. 16, (a) is a perspective view showing the whole of the container for specimen, (b) is a perspective view showing the cover member shown in (a), (c) is a view in which the cover member shown in (b) is viewed from the back side, (d) is a side view of the cover member and specimen-picking rod shown in (a), and (e) is another side view of the cover member and specimen-picking rod shown in (a).

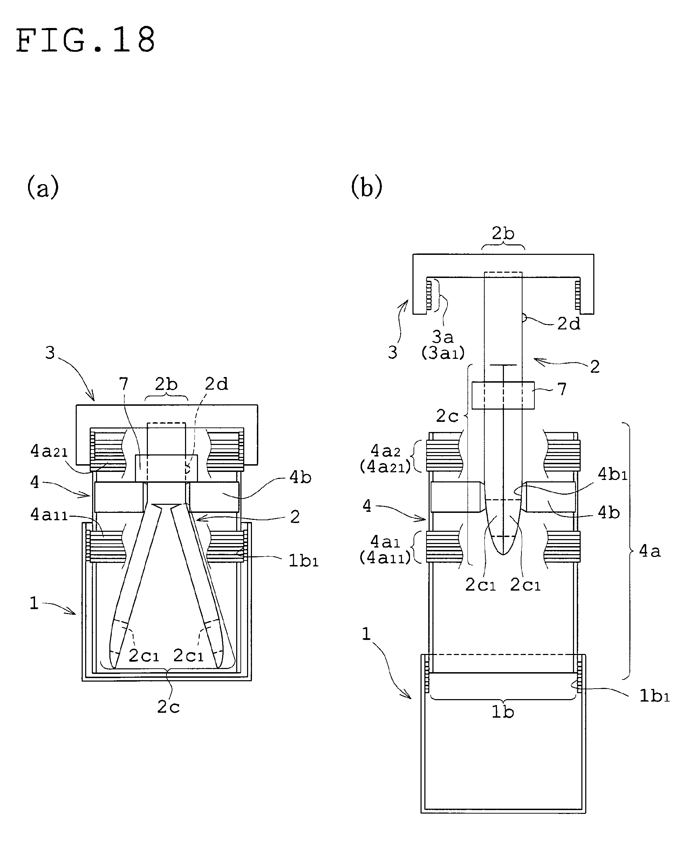

[Fig. 18] Fig. 18 is an explanatory view showing the constitution of a container for specimen according to an eleventh embodiment, (a) is a view showing an initial state of the container for specimen or a state of the container for specimen in which a collected specimen is preserved, and (b) is a view showing the container for specimen shown in (a) which is divided into its constitution members.

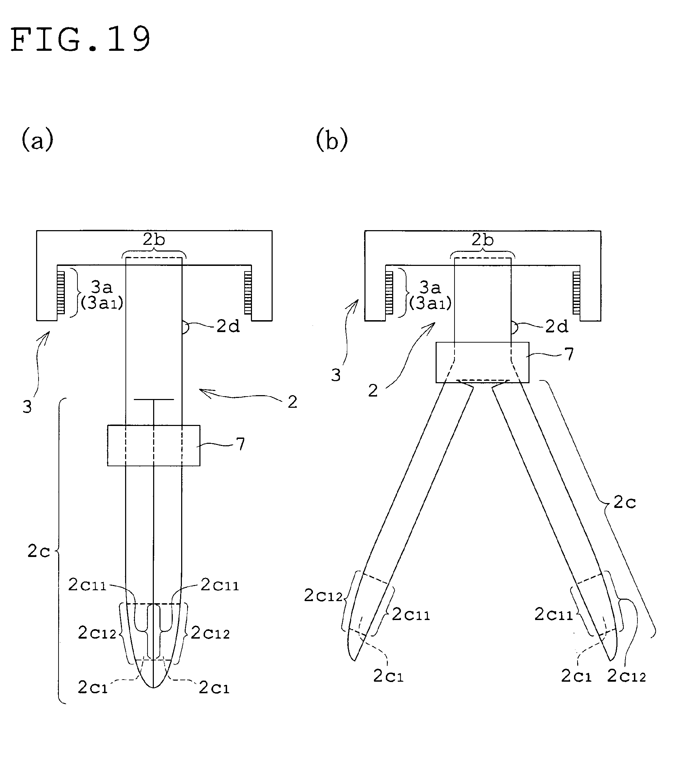

[Fig. 19] Fig. 19 is an explanatory view showing the constitutions of the cover member and specimen-picking rod in the container for specimen shown in Fig. 18, (a) is a view showing a state in which the forked portion of the specimen-picking rod is closed, and (b) is a view showing a state in which the forked portion of the specimen-picking rod is opened.

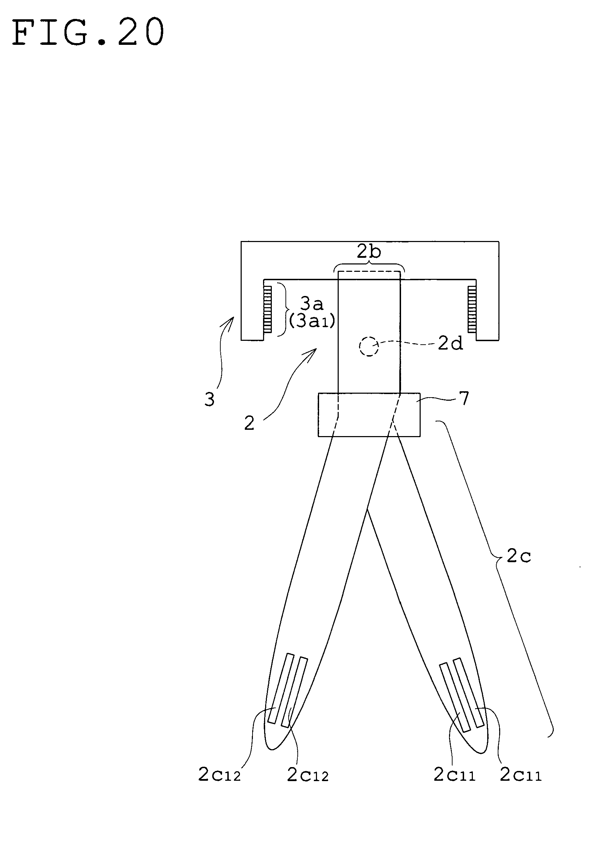

[Fig. 20] Fig. 20 is a side view of the cover member and specimen-picking portion shown in Fig. 19 (b).

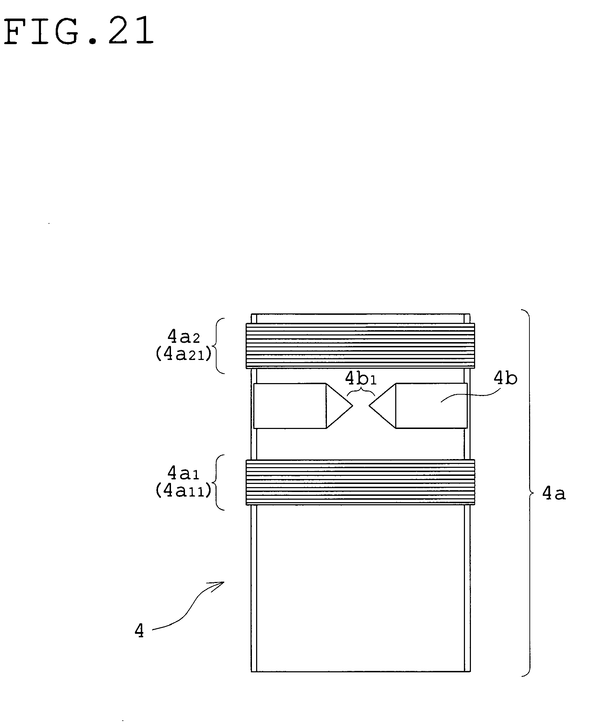

[Fig. 21] Fig. 21 is a view showing the constitution of the intermediary unit in the container for specimen shown in Fig. 18.

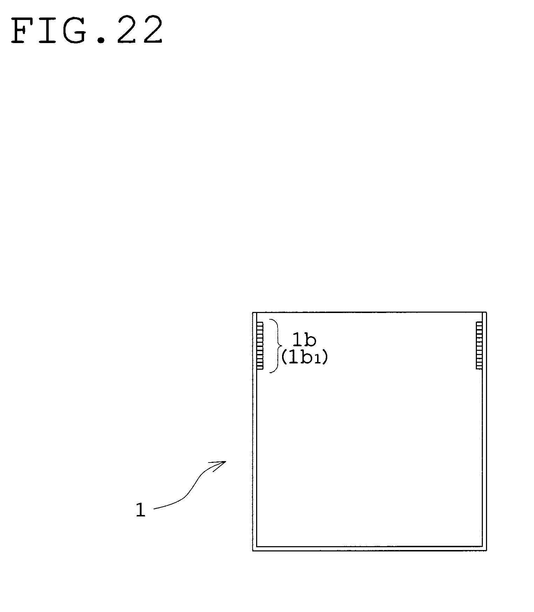

[Fig. 22] Fig. 22 is a view showing the constitution of the container body in the container for specimen shown in Fig. 21.

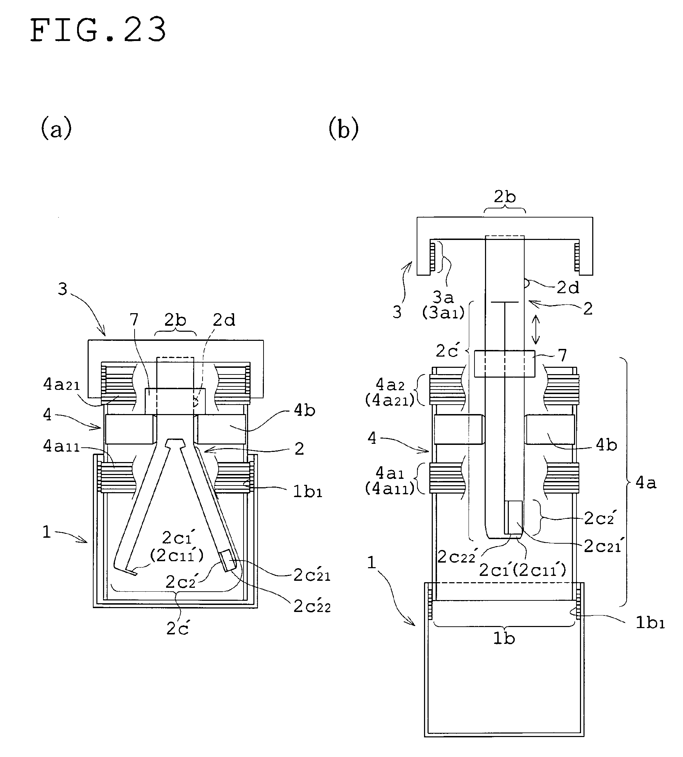

[Fig. 23}]Fig. 23 is an explanatory view showing the constitution of a container for specimen according to a twelfth embodiment, (a) is a view showing an initial state of the container for specimen or a state of the container for specimen in which a collected specimen is preserved, and (b) is a view showing the container for specimen shown in (a) which is divided into its constitution members.

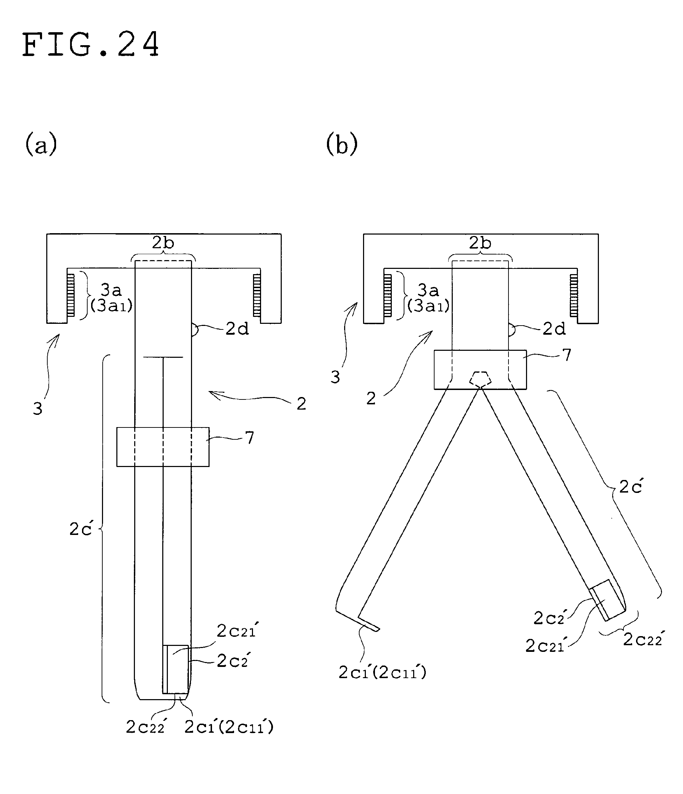

[Fig. 24] Fig. 24 is an explanatory view showing the constitutions of the cover member and specimen-picking rod in the container for specimen shown in Fig. 23, (a) is a view showing a state in which the forked portion of the specimen-picking rod is closed, and (b) is a view showing a state in which the forked portion of the specimen-picking rod is opened.

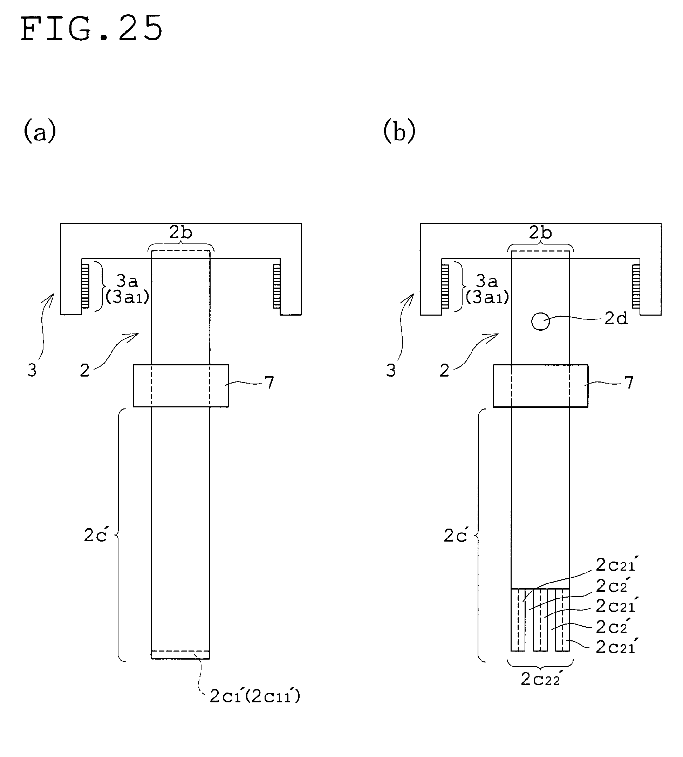

[Fig. 25] Fig. 25 is an explanatory view showing the constitutions of the cover member and specimen-picking rod in the container for specimen shown in Fig. 23, (a) is a view in which the cover member and specimen-picking rod shown in Fig. 24 (b) are viewed from the left side, and (b) is a view in which the cover member and specimen-picking rod shown in Fig. 24 (b) are viewed from the right side.

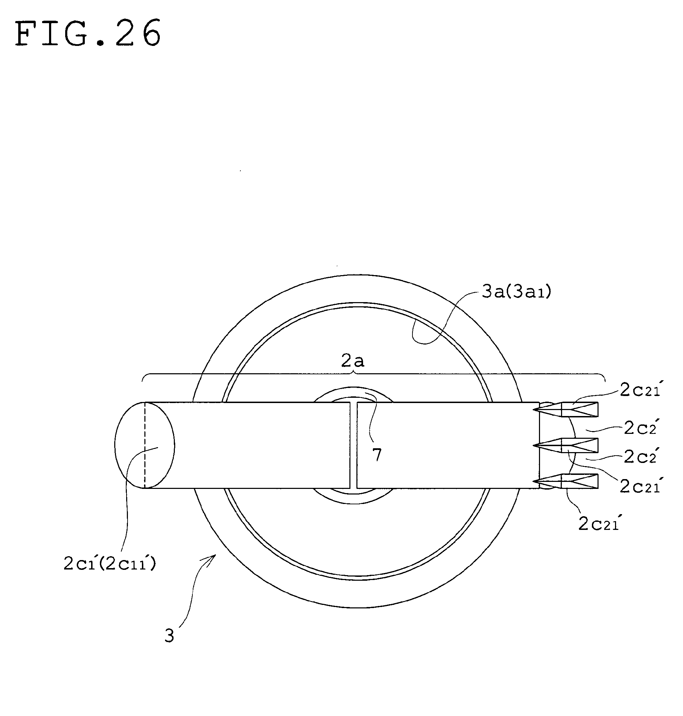

[Fig. 26] Fig. 26 is a view in which the cover member and specimen-picking rod shown in Fig. 24 (b) are viewed from the lower side.

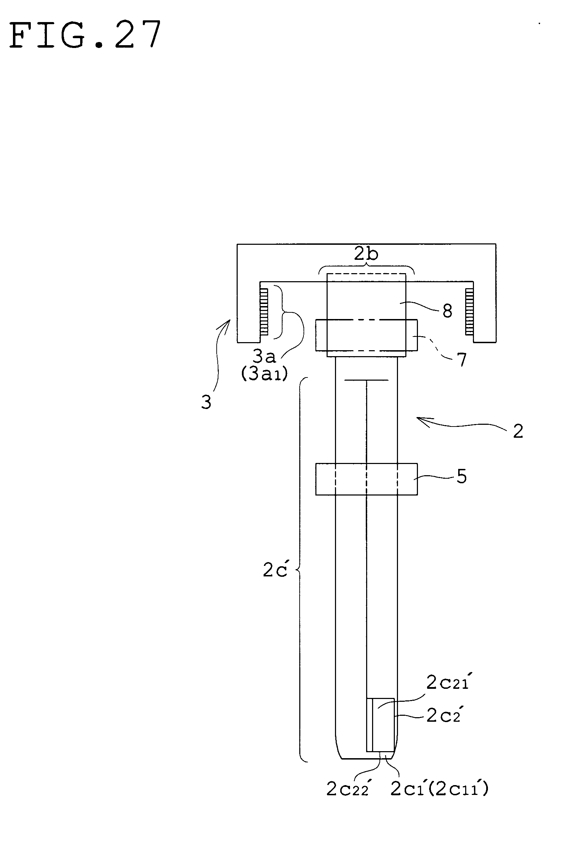

[Fig. 27] Fig. 27 is an explanatory view showing one example of variations of the annular portion-fixing means in the cover member and specimen-picking rod of the container for specimen according to the twelfth embodiment.

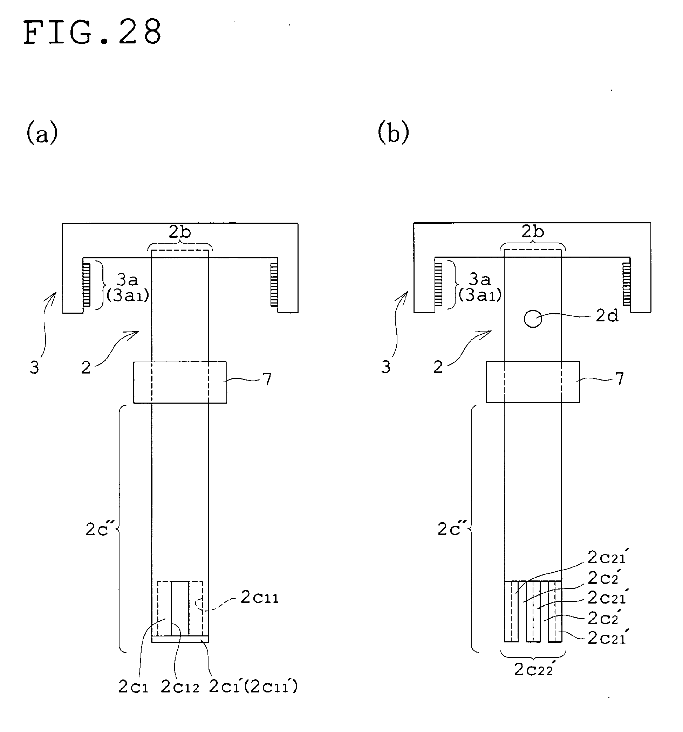

[Fig. 28] Fig 28 is an explanatory view showing the constitution of a main part of a container for specimen according to a thirteenth embodiment of the present invention, (a) is a view in which the cover member and specimen-picking rod are viewed from the left side, and (b) is a view in which the cover member and specimen-picking rod are viewed from the right side.

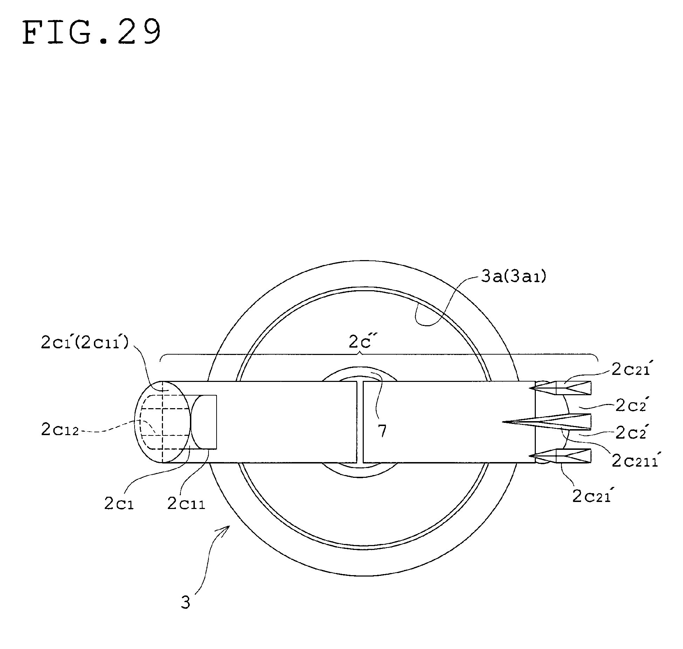

[Fig. 29] Fig. 29 is a view in which the cover member and specimen-picking rod shown in Fig. 28 are viewed from the lower side.

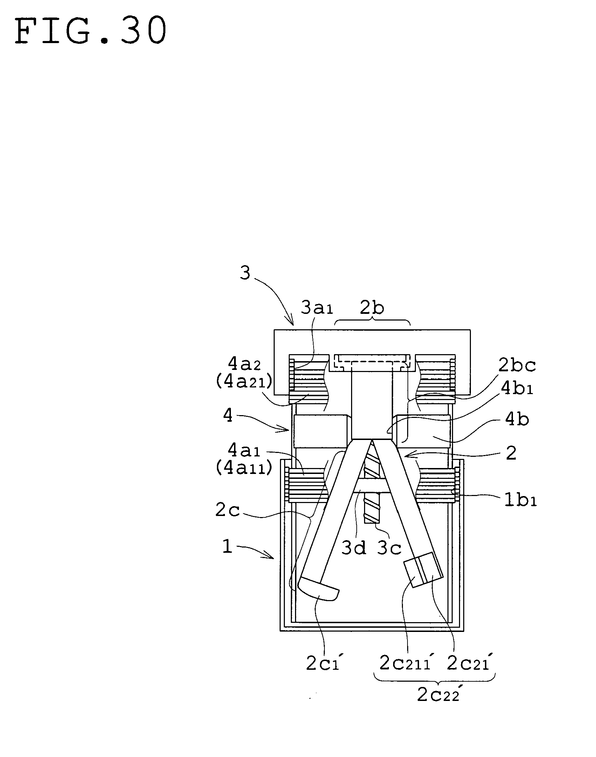

[Fig. 30] Fig. 30 is an explanatory view showing the constitution of a container for specimen according to a fourteenth embodiment of the present invention and is a view showing an initial view of the container for specimen or a state of the container for specimen in which a collected specimen is preserved.

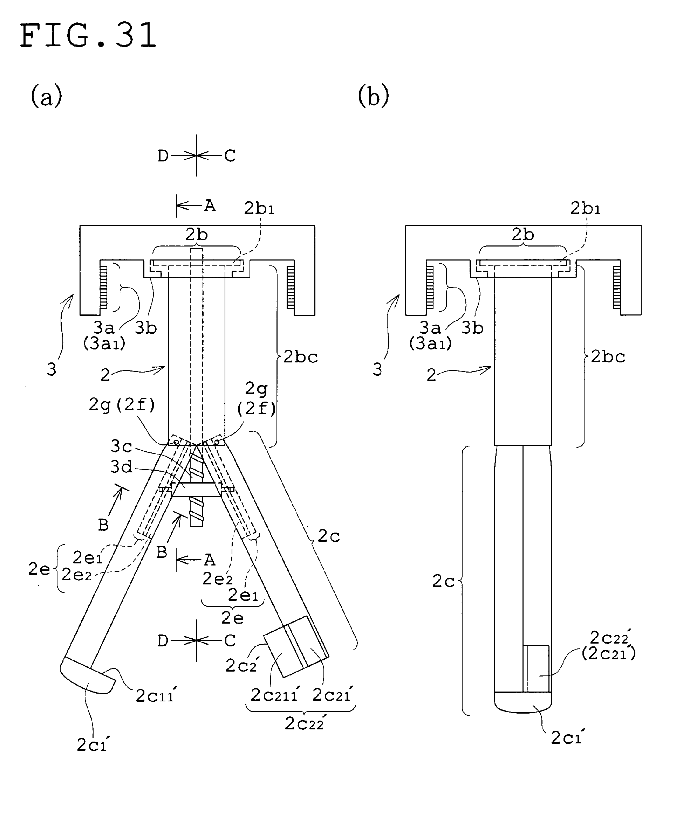

[Fig. 31] Fig. 31 is an explanatory view showing the constitutions of the cover member and specimen-picking rod in the container for specimen shown in Fig. 30, (a) is a view showing a state in which the forked portion of the specimen-picking rod is opened, and (b) is a view showing a state in which the forked portion of the specimen-picking rod is closed.

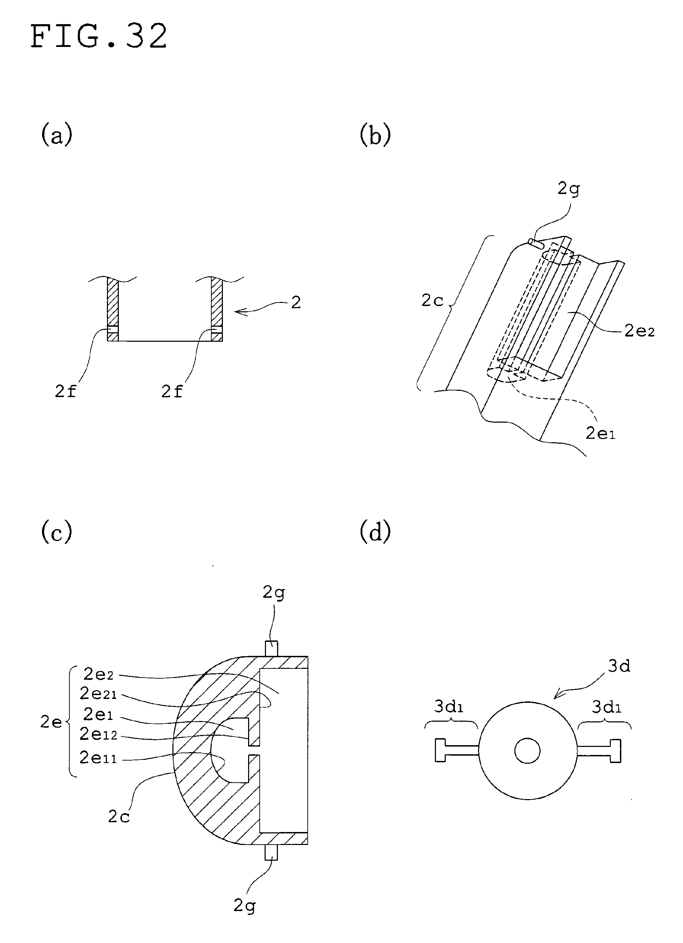

[Fig. 32] Fig. 32 is an explanatory view showing the constitutions of main parts of the cover member and specimen-picking rod shown in Fig. 31, (a) is a view showing a cross section along a line A-A of a part of the opening and closing support portion of the forked portion of the specimen-picking rod shown in Fig. 31 (a), (b) is a view in which a part of one branching portion of the forked portion of the specimen-picking rod shown in Fig. 31 (a) is viewed from the side of the one branching portion which touches the other branching portion of the forked portion in closing the forked portion, (c) is a view showing a cross section along a line B-B of a part of one branching portion of the forked portion of the specimen-picking rod shown in Fig. 31 (a), and (d) is a view in which the female screw in the specimen-picking rod shown in Fig. 31 (a) is viewed from the lower side.

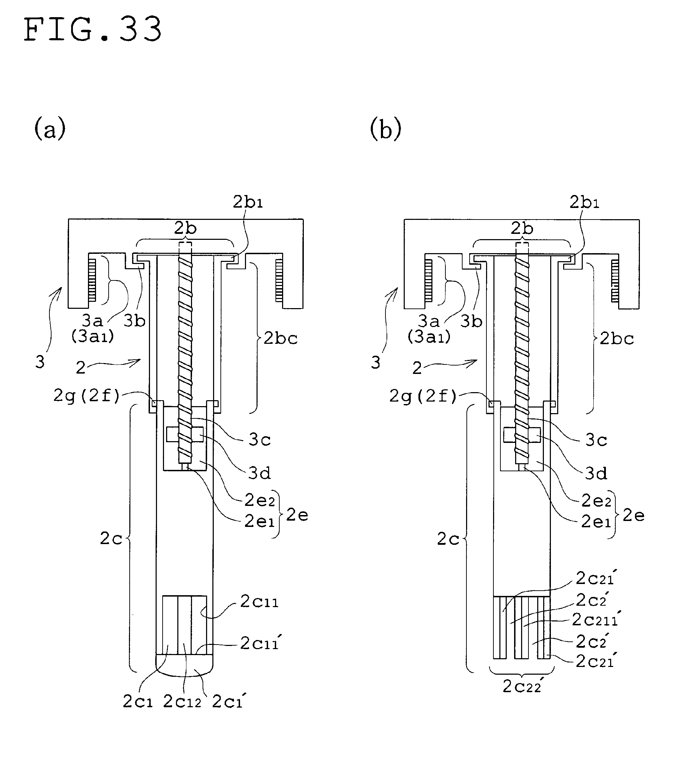

[Fig. 33] Fig. 33 is a sectional view of the cover member and specimen-picking rod shown in Fig. 31 (a), (a) is a cross sectional view along a line C-C in Fig. 31 (a), and (b) is a cross sectional view along a line D-D in Fig. 31 (a).

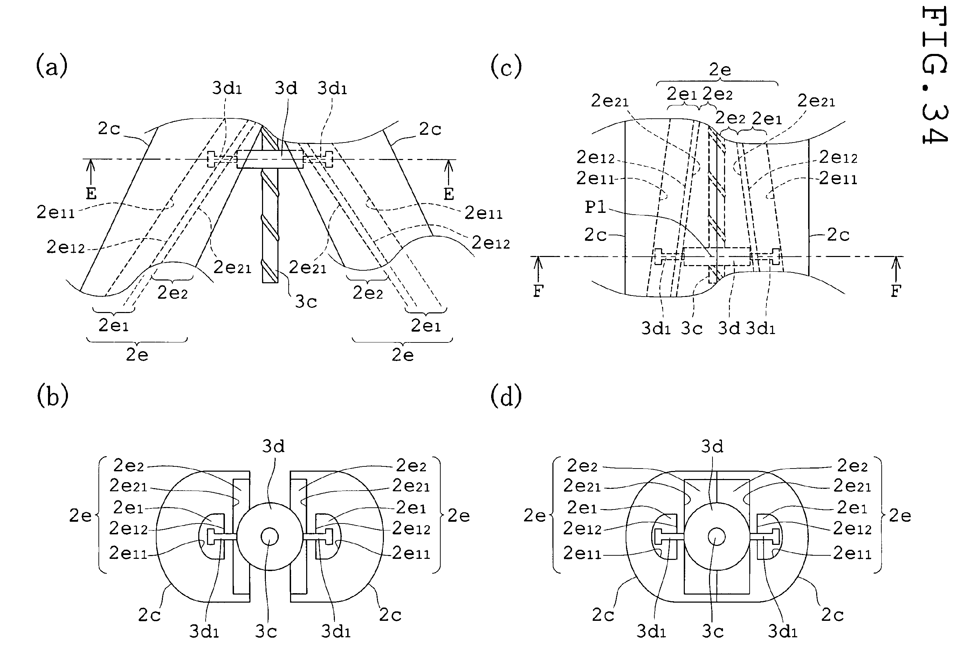

[Fig. 34] Fig. 34 is an explanatory view showing the constitution of a main part of the specimen-picking rod of the fourteenth embodiment, (a) is a partially enlarged of the forked portion which is in an opened state as shown in Fig. 31 (a), (b) is a cross sectional view along a line E-E of (a), (c) is a partially enlarged view of the forked portion which is in a closed state as shown in Fig. 31 (b), and (d) is a cross sectional view along a F-F line of (c).

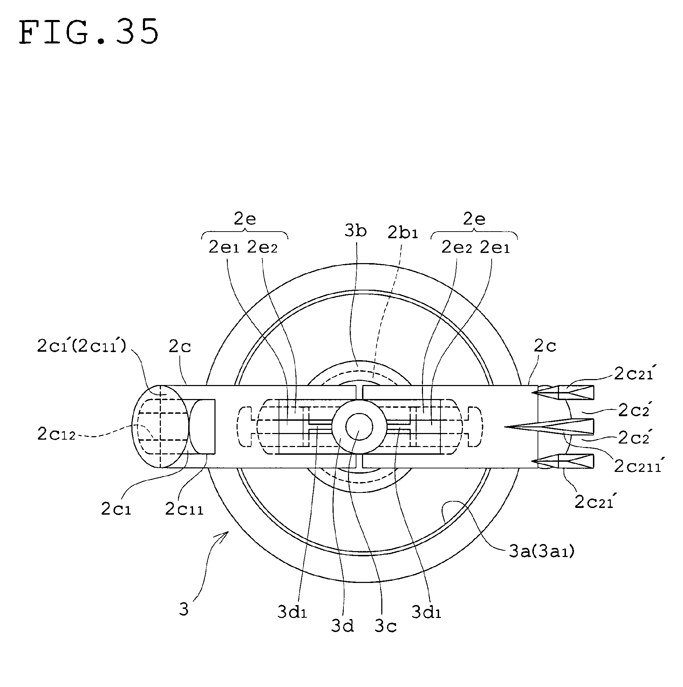

[Fig. 35] Fig. 35 is a view in which the cover member and specimen picking rod shown in Fig. 31 (a) are viewed from the lower side.

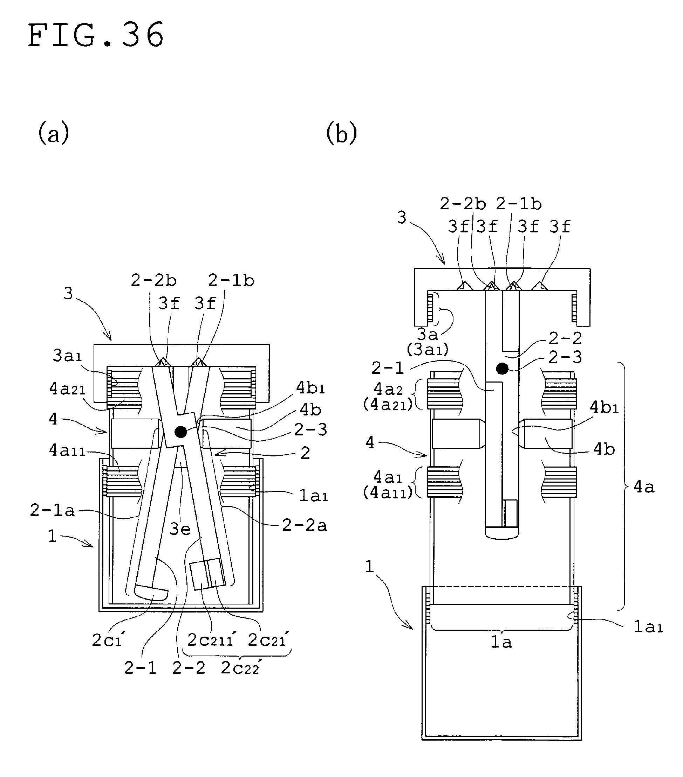

[Fig. 36] Fig. 36 is an explanatory view showing the constitution of a container for specimen according to the fifteenth embodiment of the present invention, (a) is a view showing an initial state of the container for specimen or a state of the container for specimen in which a collected specimen is preserved, and (b) is a view showing the container for specimen shown in (a) which is divided into its constitution members.

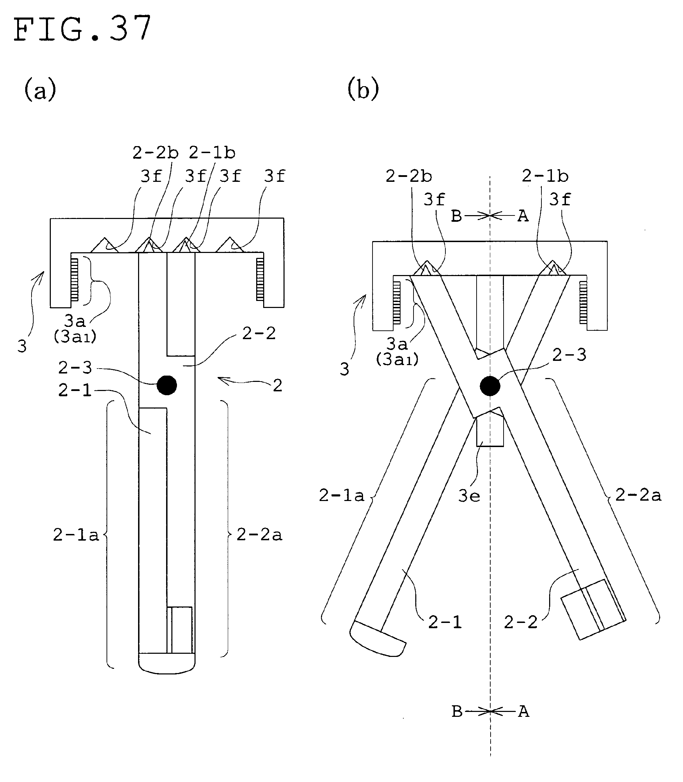

[Fig. 37] Fig. 37 is an explanatory view showing the constitutions of the cover member and specimen-picking rod in the container for specimen shown in Fig. 36, (a) is a view showing a state in which the forked portion of the specimen-picking rod is closed, and (b) is a view showing a state in which the forked portion of the specimen-picking rod is opened.

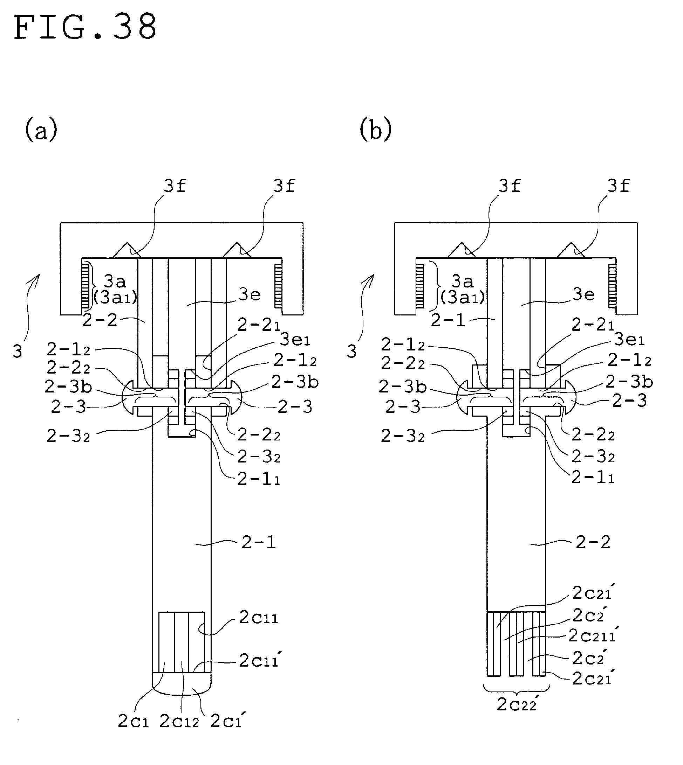

[Fig. 38] Fig. 38 is a sectional view of the cover member and specimen-picking rod shown in Fig. 37 (b), (a) is a cross sectional view along a line A-A in Fig. 37 (b), and (b) is a cross sectional view along a line B-B in Fig. 37 (b).

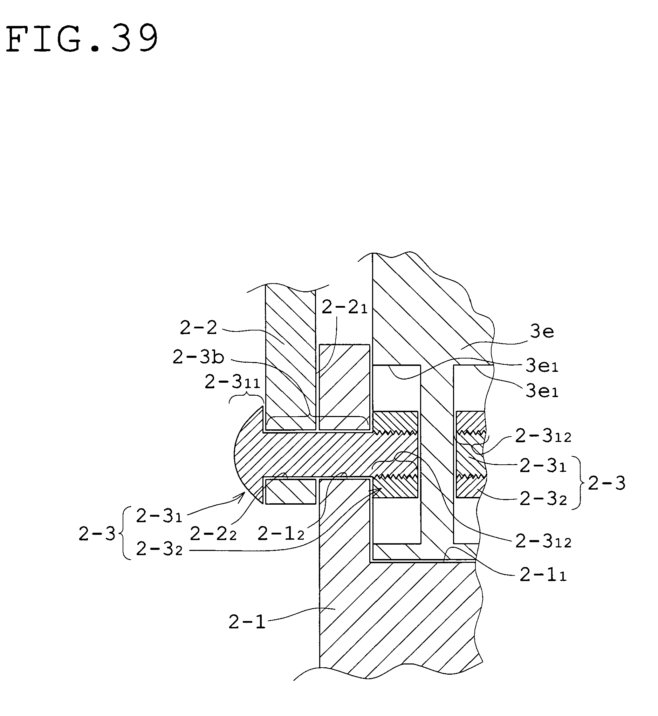

[Fig. 39] Fig. 39 is an enlarged view of the connection region of the forked portion in the sectional view of the cover member and specimen-picking rod shown in Fig. 38.

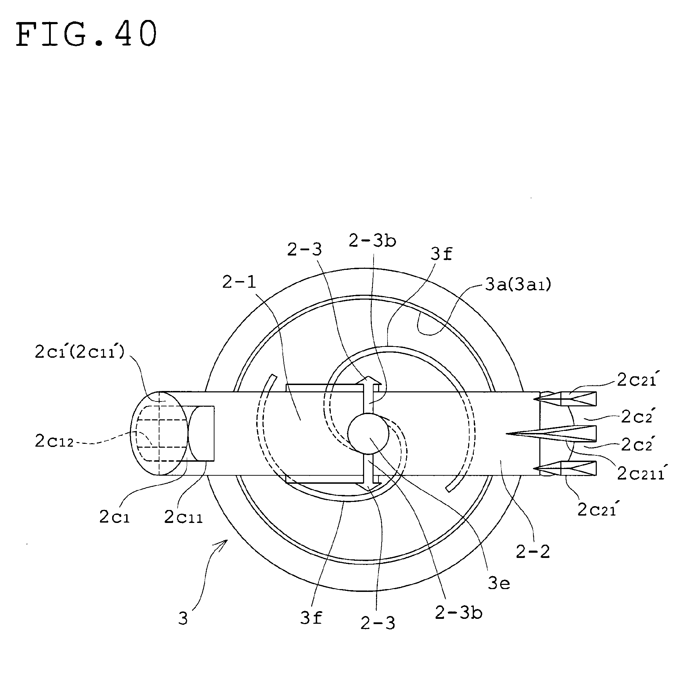

[Fig. 40}]Fig. 40 is a view in which the cover member and specimen-picking rod shown in Fig. 38 (b) are viewed from the lower side.

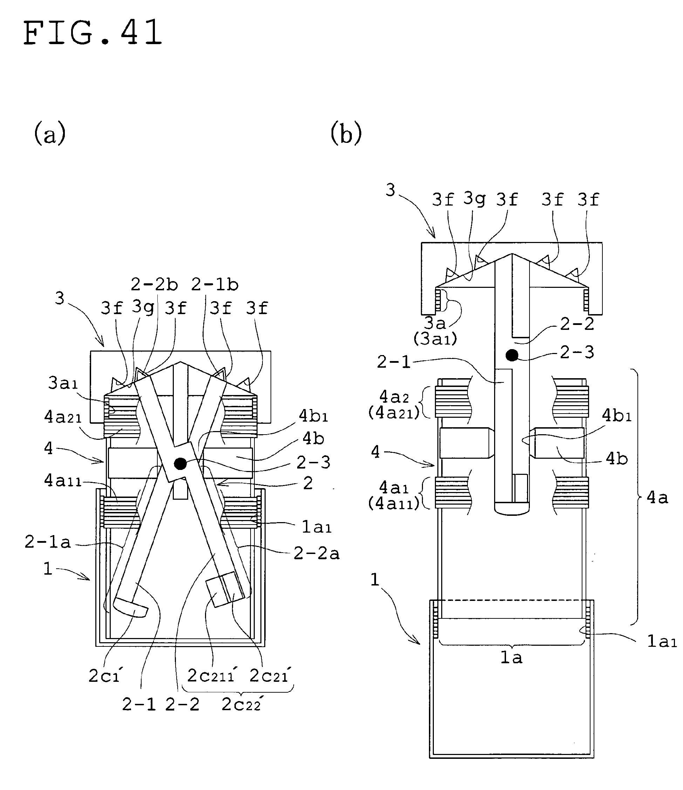

[Fig. 41] Fig. 41 is an explanatory view showing the constitution of a container for specimen according to the sixteenth embodiment of the present invention, (a) is a view showing an initial state of the container for specimen or a state of the container for specimen in which a collected specimen is preserved, and (b) is a view showing the container for specimen shown in (a) which is divided into its constitution members.

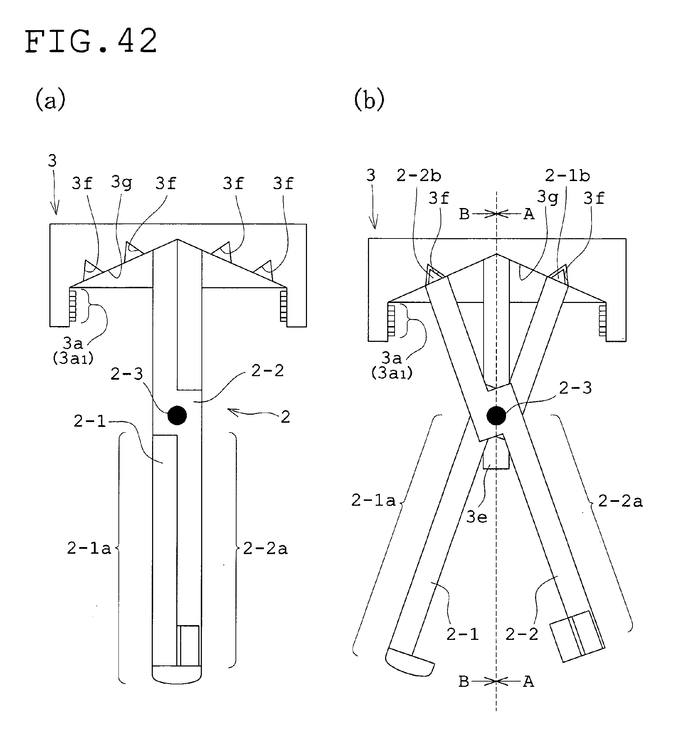

[Fig. 42] Fig. 42 is an explanatory view showing the constitutions of the cover member and specimen-picking rod in the container for specimen shown in Fig. 41, (a) is a view showing a state in which the forked portion of the specimen-picking rod is closed, and (b) is a view showing a state in which the forked portion of the specimen-picking rod is opened.

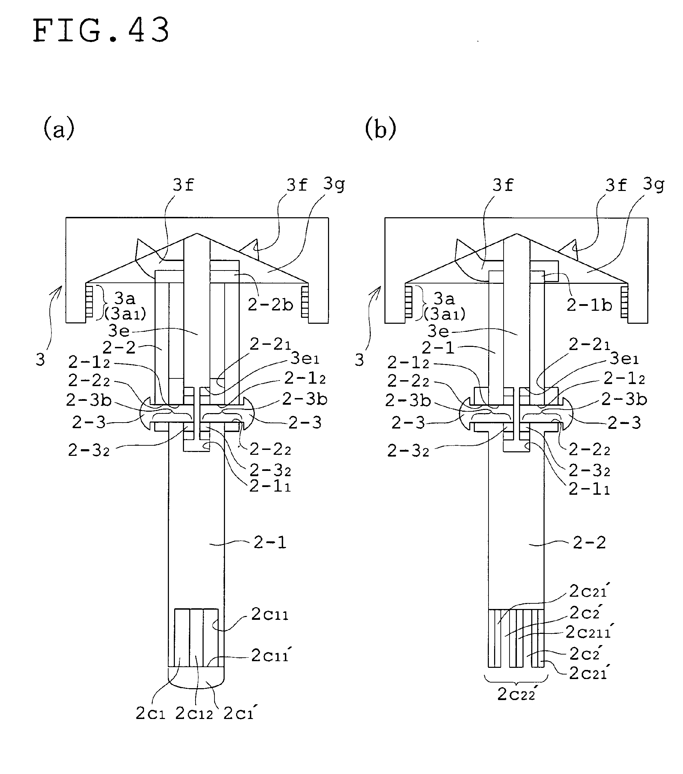

[Fig. 43] Fig. 43 is a sectional view of the cover member and specimen-picking rod shown in Fig. 42 (b), (a) is a cross sectional view along a line A-A in Fig. 42 (b), and (b) is a cross sectional view along a line B-B in Fig. 42 (b).

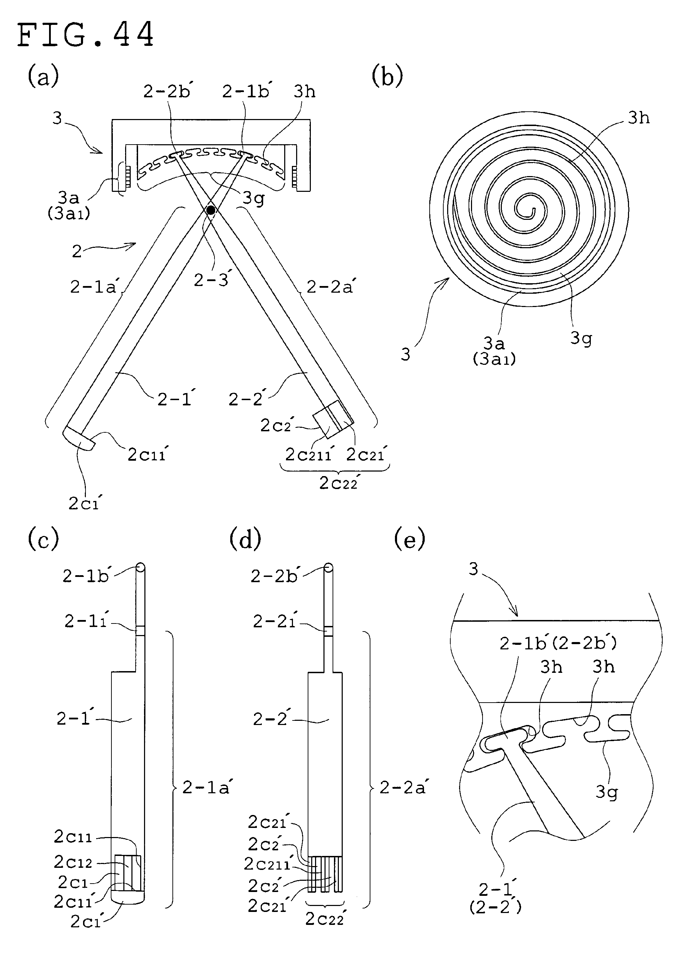

[Fig. 44] Fig. 44 is an explanatory view schematically showing the constitutions of the cover member and specimen-picking rod in a container for specimen according to the seventeenth embodiment of the present invention, (a) is a view showing a state in which the forked portion of the specimen-picking rod is opened, (b) is a view in which the cover member shown in (a) is viewed from the lower side, (c) is a view in which one branching portion of the forked portion of the specimen-picking rod shown in (a) is viewed from the side of the one branching portion which touches the other branching portion of the forked portion in closing the forked portion, (d) is a view in which the other branching portion of the forked portion of the specimen-picking rod shown in (a) is viewed from the side of the other branching portion which touches the one branching portion of the forked portion in the closing the forked portion, and (e) is an enlarged view of the region in which the cover member and the specimen-picking rod shown in (a) threadedly engages with each other.

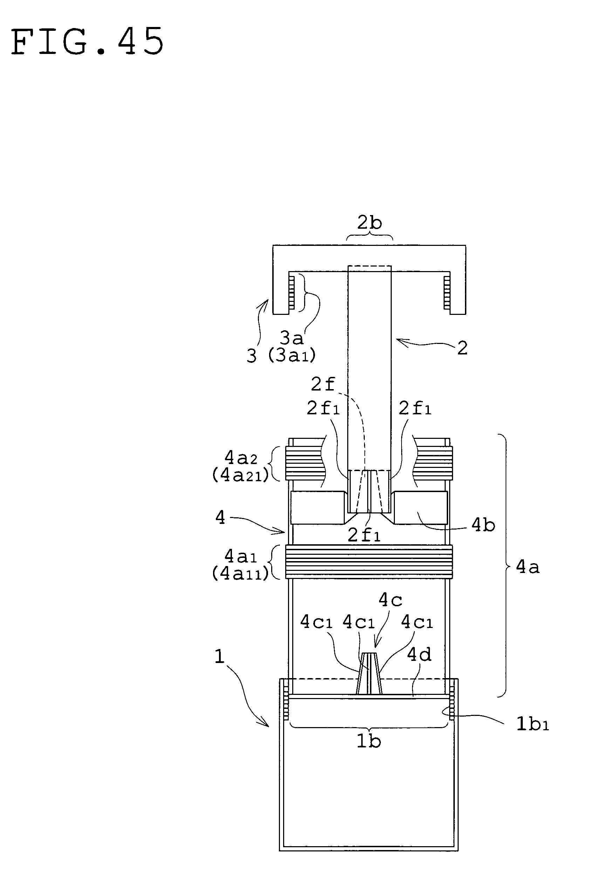

[Fig. 45] Fig. 45 is a view schematically showing the constitution of a container for specimen according to the eighteenth embodiment as the first reference example of the present invention with the container for specimen divided into its constitution members.

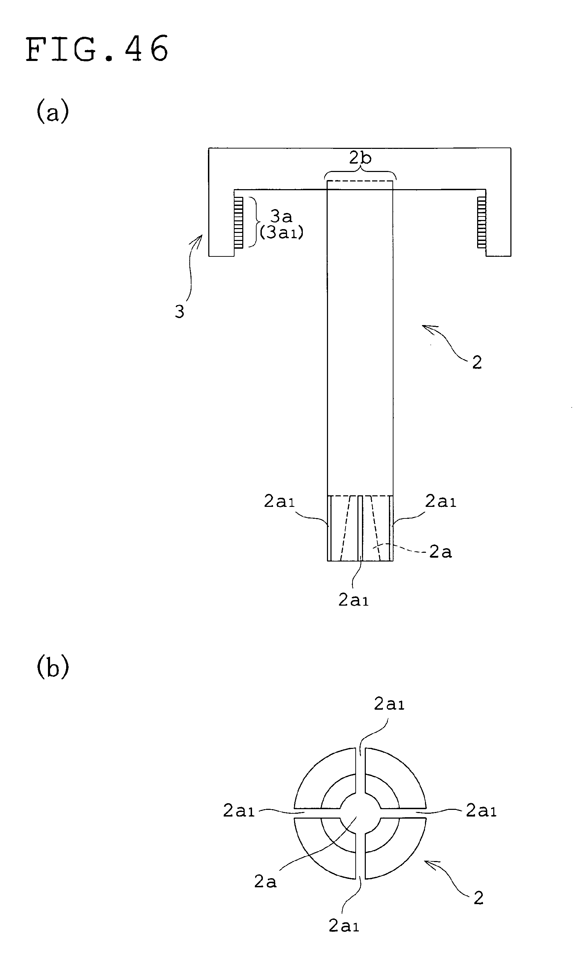

[Fig. 46] Fig. 46 is an explanatory view showing the constitutions of the cover member and specimen-picking rod in the container for specimen shown in Fig. 45, (a) is a view mainly showing a side cross section of the specimen-picking rod, and (b) is a view in which the front end of the specimen-picking rod is viewed from the lower side.

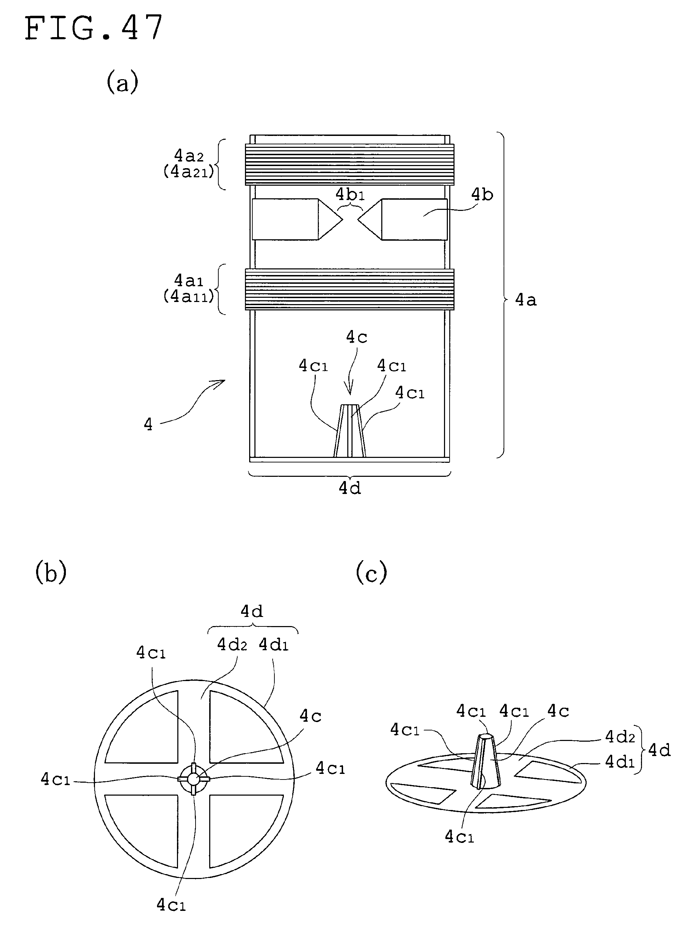

[Fig. 47] Fig. 47 is view showing the constitution of the intermediary unit in the container for specimen shown in Fig. 45, (a) is a side view of the intermediary unit, and (b) is a plan view of the convex removing portion and the connection portion, and (c) is a perspective view of the convex removing portion and the connection portion.

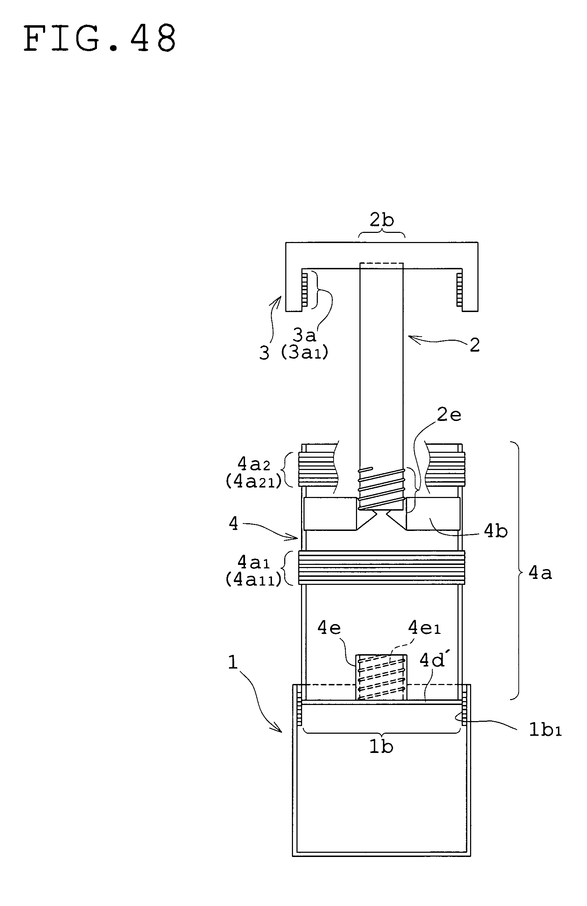

[Fig. 48] Fig. 48 is a view schematically showing the constitution of a container for specimen according to the nineteenth embodiment as the second reference example of the present invention with the container for specimen divided into its constitution members.

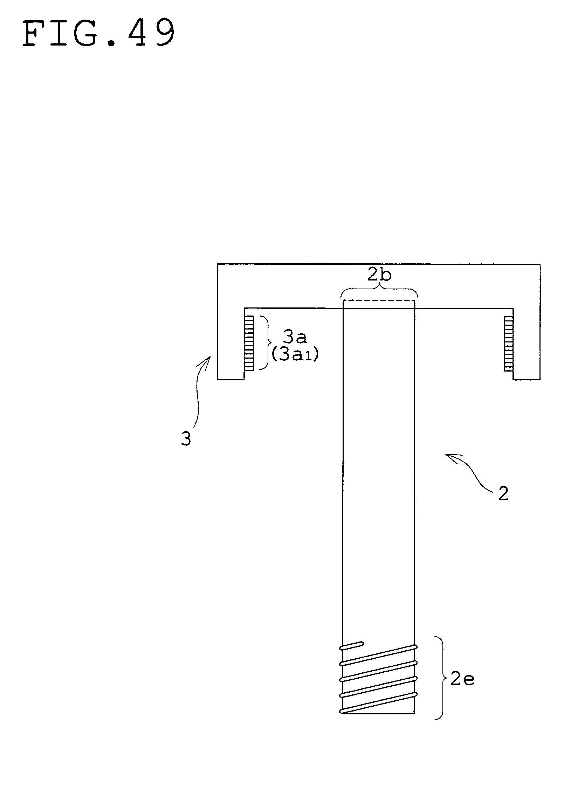

[Fig. 49] Fig. 49 is an explanatory view showing the constitutions of the cover member and the specimen-picking rod in the container body in the container for specimen shown in Fig. 48.

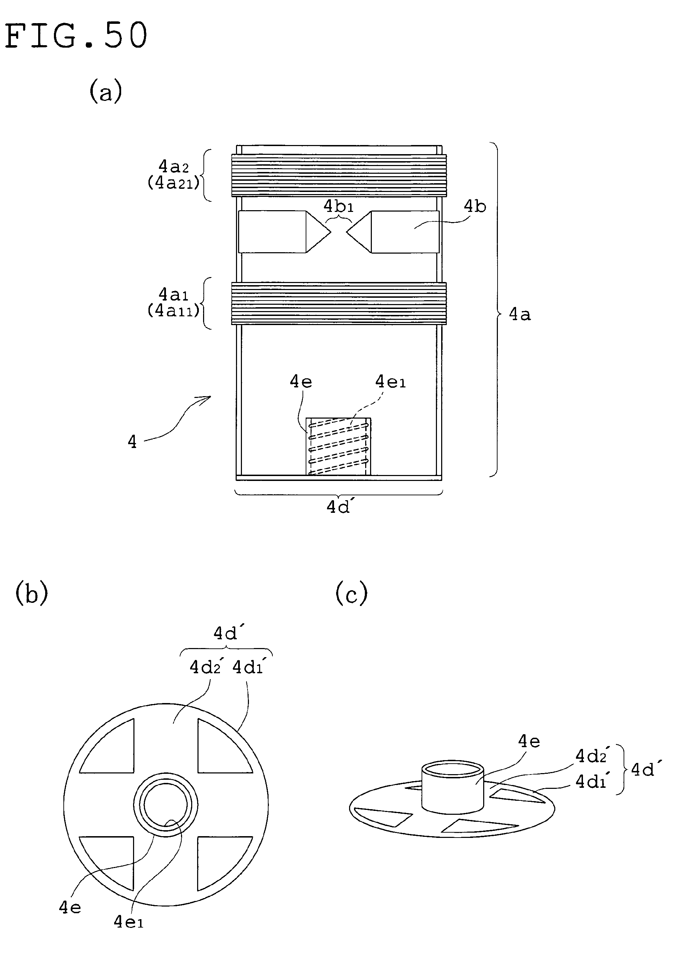

[Fig. 50] Fig. 50 is a view showing the constitution of the intermediary unit in the container for specimen shown in Fig. 48, (a) is a side view of the intermediary unit, (b) is a plan view of the convex removing portion and connection portion, (c) is a perspective view of the convex removing portion and the connection portion.

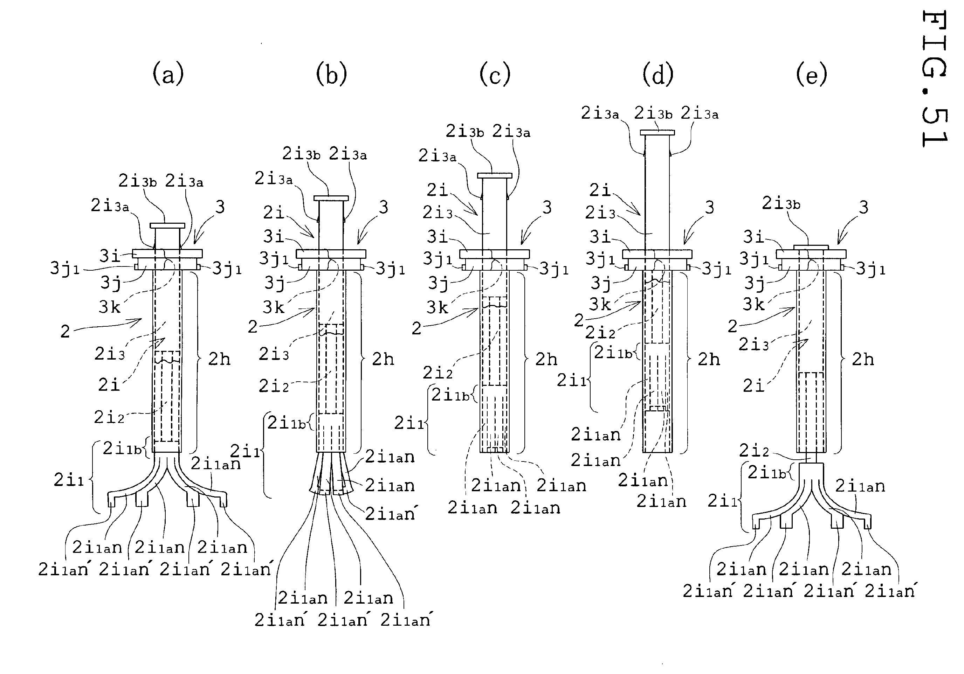

[Fig. 51] Fig. 51 is a state-explaining view showing the constitutions of the cover member and specimen-picking rod in a container for specimen according to the twentieth embodiment, (a) is a view showing a state in which the cylinder portion of the specimen-picking rod is pushed into the tube portion up to the position at which the cylinder portion is restrained by the stopper portion, (b) is a view showing a state in which the tube portion contains a plurality of the vertical divisions that are provided in the front end of the cylinder portion of the specimen-picking rod, by half, (c) is a view showing a state in which the tube portion completely contains a plurality of the vertical divisions that are provided in the front end of the cylinder portion of the specimen-picking rod, (d) is a view showing a state in which a space is formed between the front ends of the vertical divisions and the front end of the tube portion, and (e) is a view showing a state in which the cylinder portion of the specimen-picking rod having the state of (a) is pushed to the tube-portion side to the extent that the stopper portion is released.

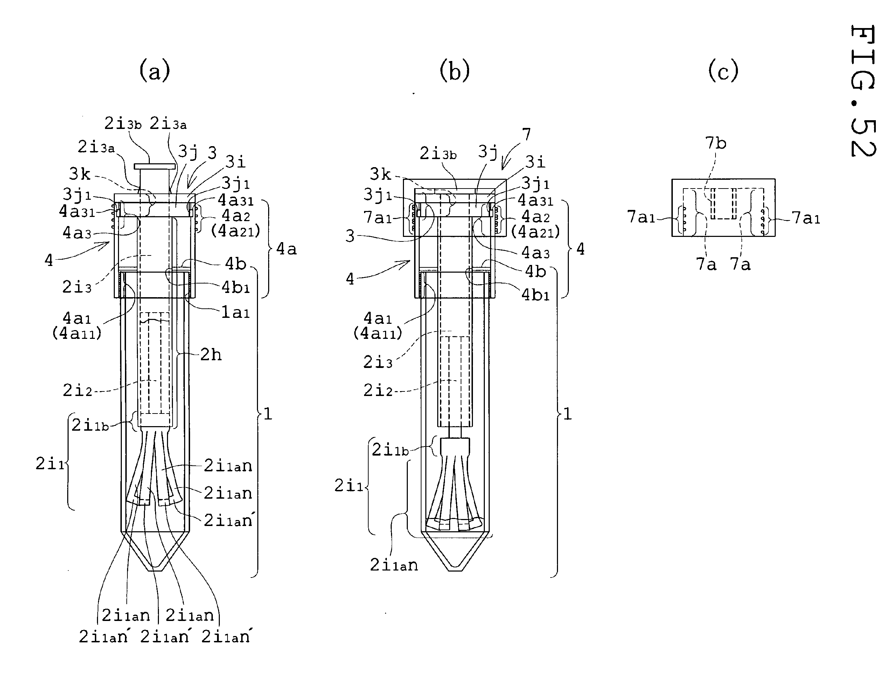

[Fig. 52] Fig. 52 is an explanatory view showing the constitution of a container for specimen according to the twenty-first embodiment, (a) is a view showing a state in which the cover member and specimen-picking rod shown in Fig. 34 is connected with the container body through the intermediary unit, and (b) is a view showing a state in which the outer cover is attached to the intermediary unit having the state of (a), and (c) is a view showing the constitution of the outer cover.

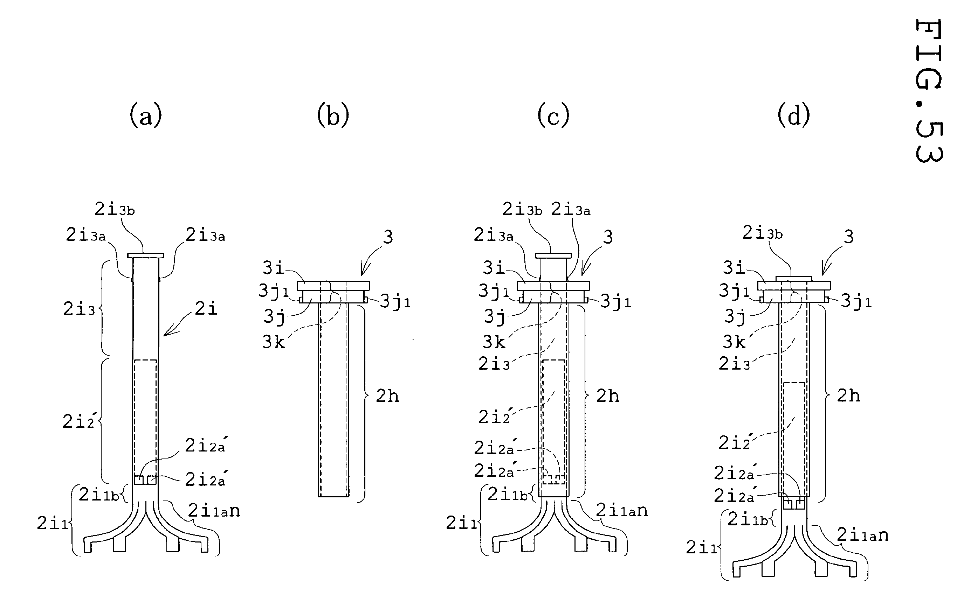

[Fig. 53] Fig. 53 is a state-explaining view showing the constitutions of the cover member and specimen-picking rod in a container for specimen according to the twenty-second embodiment of the present invention, (a) is a view showing the cylinder portion of the specimen-picking rod, (b) is a view showing the tube portion of the specimen-picking rod, (c) is a view showing a state in which the cylinder portion of the specimen-picking rod is pushed into the tube portion up to the position at which the cylinder portion is restrained by the stopper portion, and (d) is a view showing a state in which the cylinder portion of the specimen-picking rod having the state of (c) is pushed on the tube-portion side to the extent that the stopper portion is released.

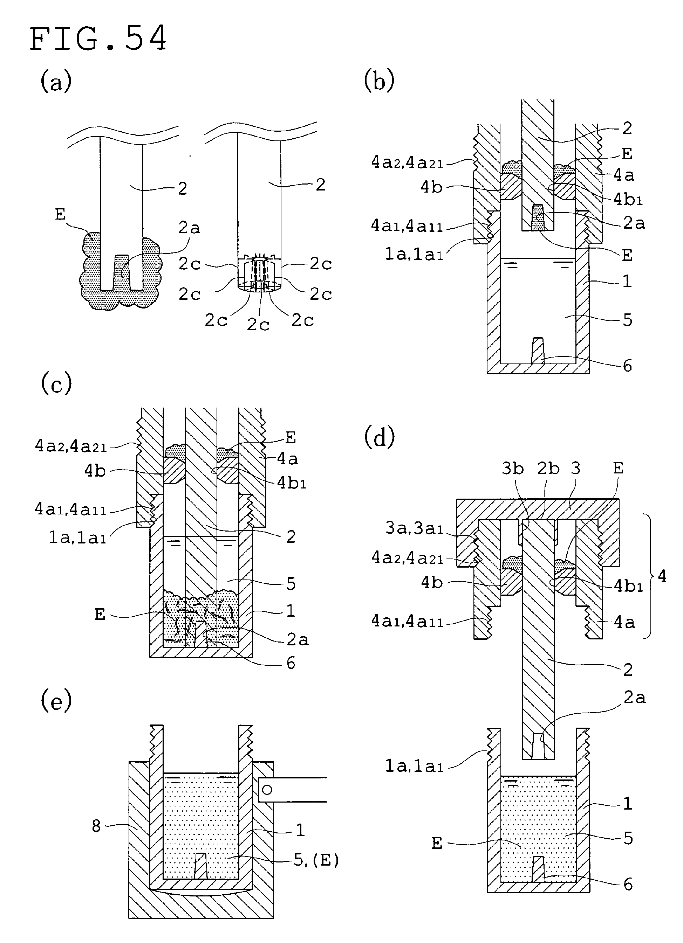

[Fig. 54] Fig. 54 is an explanatory view showing a mode of process including collection and preservation of an artificial stool using the container for specimen according to the first embodiment of the present invention, the attachment of the container to a centrifuge, and extraction of nucleic acids through the centrifuge, (a) shows a state in which a stool is picked by the specimen-picking rod and the right side of (a) is a perspective view showing the front end portion of the specimen-picking rod, (b) is a view showing a state in which the specimen-picking rod holding the picked stool penetrates through the through hole of the intermediary unit, (c) is a view showing a state in which the picked stool that is held by the front end portion of the specimen-picking rod is dispersed in a solution of the container body with the specimen-picking rod inserted into the intermediary unit, (d) is a view showing a state in which the intermediary unit is removed from the container body with the specimen-picking rod fixed to the cover member and the cover member fitted to the intermediary unit, and (e) is a view showing a state in which the container body from which the intermediary unit is removed is attached to the centrifuge.

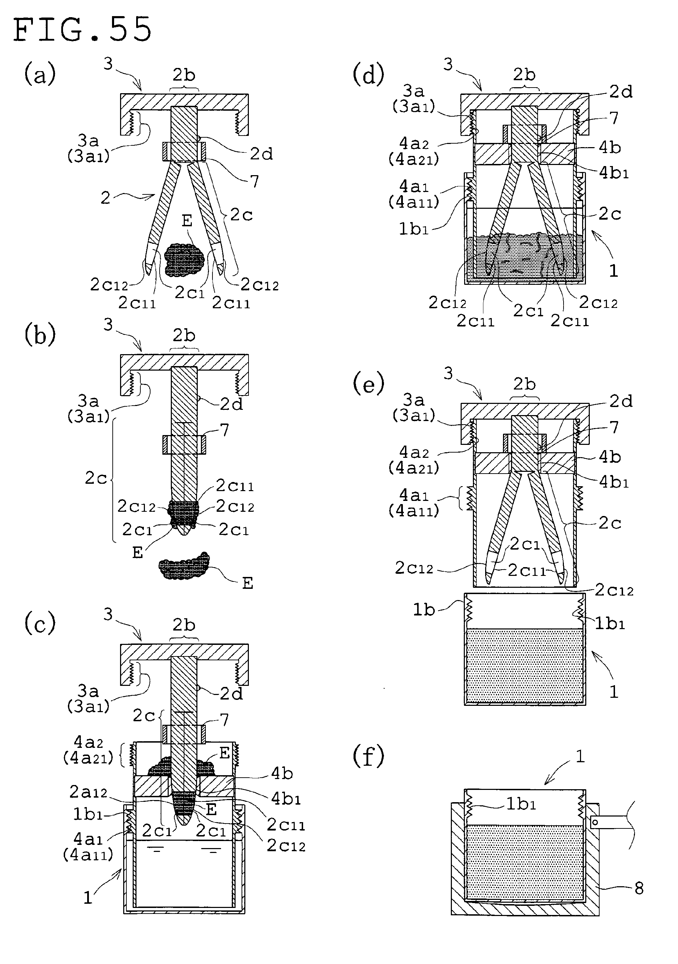

[Fig. 55] Fig. 55 is an explanatory view showing a mode of process including collection and preservation of an artificial stool using the container for specimen according to the eleventh embodiment of the present invention, the attachment of the container to a centrifuge, and extraction of nucleic acids through the centrifuge, (a) is a sectional view showing a state in which the forked portion is opened in order to pick the stool through the specimen-picking rod, (b) is a sectional view showing a state in which the forked portion is closed so that the stool is picked, (c) is a view showing a state in which the specimen-picking rod holding the picked stool penetrates through the through hole of the intermediary unit, (d) is a view showing a state in which the picked stool that is held by the front end portion of the specimen-picking rod is dispersed in a solution of the container body with the specimen-picking rod inserted into the intermediary unit, (e) is a view showing a state in which the intermediary unit is removed from the container body with the specimen-picking rod fixed to the cover member and the cover member fitted to the intermediary unit, and (f) is a view showing a state in which the container body from which the intermediary unit is removed is attached to the centrifuge.

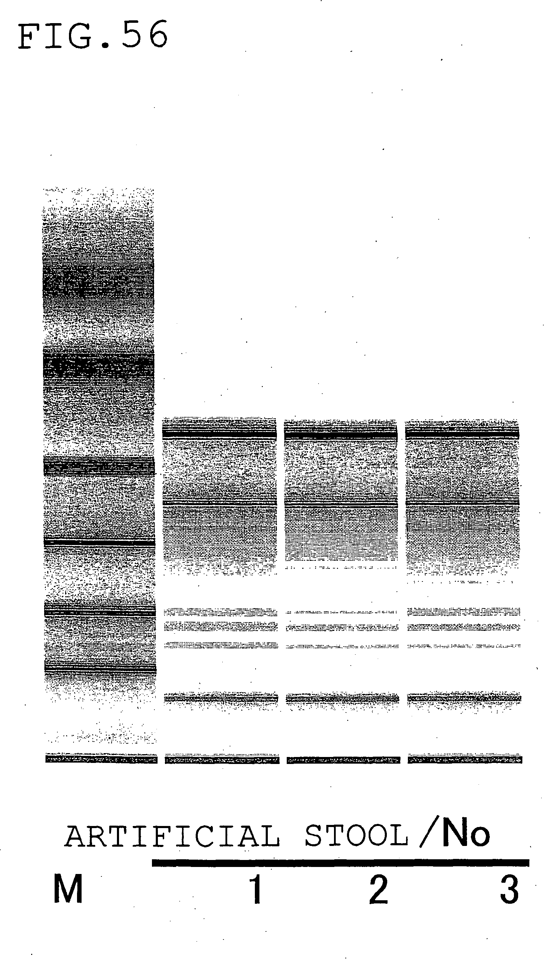

[Fig. 56] Fig. 56 is a view showing the results of the analysis of nucleic acids by electrophoresis after the nucleic acids are extracted by centrifuging the specimen that is collected by the container for specimen of the first embodiment with a general-purpose centrifuge.

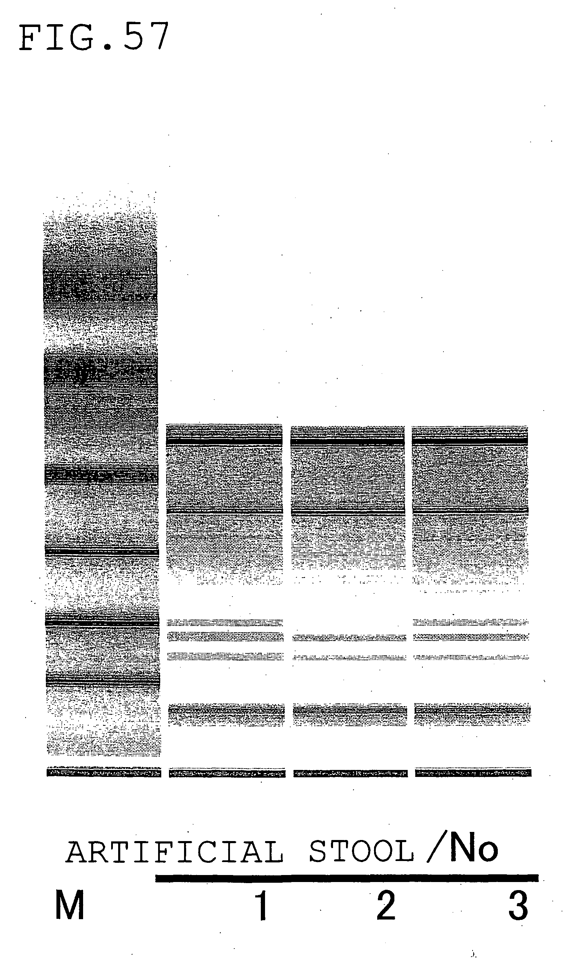

[Fig. 57] Fig. 57 is a view showing the results of the analysis of nucleic acids by electrophoresis after the nucleic acids are extracted by centrifuging the specimen that is collected by the container for preserving specimen of the second embodiment with a general-purpose apparatus for physical and chemical analysis.

### Best Form for Embodying Invention

Before undertaking the descriptions of the embodiments of the present invention, the operation effects of the present invention will be explained in detail.
A container for specimen of the present invention comprises: a container body one end of which is opened and which can hold a solution for dispersing or preserving a specimen and is formed in the shape of a tube so as to be capable of being attached to a general-purpose apparatus for physical an chemical analysis; a specimen-picking rod for picking a specimen; a cover member which connects with the rear end of the specimen-picking rod; and an intermediary unit which connects the container body and the cover member, wherein the intermediary unit includes a connection portion the both ends of which are open, the one end of which can be removably fitted to the open end of the container body, the other end of which can be removably fitted to the cover member, and which is formed in the shape of a tube.

The intermediary unit is provided between the cover member and the container body in such a way that the intermediary unit can be removably fitted to the cover member and the container body. As a result, when the specimen-picking rod holding a picked specimen is inserted through the intermediary unit into the container body with the intermediary unit fitted to the container body and the cover member connecting with the specimen-picking rod is fitted to the intermediary unit, the specimen can be preserved in the container body. The specimen is preserved with the specimen dispersed by a liquid in the container body. In addition, when the intermediary unit is removed from the container body with the cover member fitted to the intermediary unit, the container body containing the specimen can be attached to a nucleic acid extraction apparatus or the like, as a sample tube.

Also, in a container for specimen of the present invention, the intermediary unit includes: a through hole into which the specimen-picking rod can penetrate inside the connection portion; and a penetration wall which separates a space into two divisions on the upper and lower sides with the specimen-picking rod penetrating into the through hole. As a result, the penetration of the specimen-picking rod through the penetration wall makes it possible to remove a surplus part of the specimen which adheres to the side surface of the specimen-picking rod, so that the specimen can be quantified to be used for test. In addition, the close contact between the through hole and the specimen-picking rod makes it possible to preserve the specimen in the container body with the container sealed off.
And, according to a container for specimen of the present invention, in the case where a stool is collected as a specimen of human origin for example, when the container for specimen is formed in such a way that collection amounts of specimen range from 0.1 to 2g, a stool can be collected in such a way that the stool is quantified within an error of not over ± 10 percent. Besides, it is preferred more that the container for specimen is formed in such a way that the collection amounts of specimen range from 0.1 to 0.5g.
Also, it is preferred that a container for specimen of the present invention is formed in such a way that collection amounts of specimen range from 0.1 to 2g even in the case of another specimen of human origin which has a high viscosity.

Besides, it is preferred that the penetration wall is made of elastic material. As a result, the elastic deformation of the penetration wall makes it easy for the through hole to come into close contact with the specimen-picking rod even though the diameter of the through hole is smaller than that of the specimen-picking rod.
Or, an elastic member may be provided in the vicinity of the through hole of the penetration wall. In this case, for example, a container for specimen of the present invention should be formed in such a way that an annular groove is formed on the surface on which the through hole of the penetration wall is formed and an annular elastic member the internal diameter of which is smaller than the diameter of the through hole is fitted into the annular groove. Also, for example, the penetration wall may be provided with an elastic member which includes radial slits passing through the center of the through hole, covers the through hole, and deforms to come into close contact with the specimen-picking rod when the specimen-picking rod is inserted into the through hole.

Or, the penetration wall may be formed in such a way that the penetration wall is provided with a plurality of annular grooves which are formed on the surface forming the through hole and along the penetration direction. As a result, the portions of the penetration wall which lie between the annular grooves become thin, so that the portions of the penetration wall can easily deform in the penetration direction. Accordingly, the deformation of the penetration wall makes it easy for the through hole to come into close contact with the specimen-picking rod even though the diameter of the through hole is smaller than that of the specimen-picking rod.
Besides, if the portions of the penetration wall which lie between the annular grooves can be made thin so that the portions of the penetration wall can easily deform in the penetration direction, then the cross sectional shapes of a plurality of the annular grooves which are formed on the surface forming the through hole of the penetration wall may be formed in the shape of any one of the letter "U", the letter "V", and an arc.

Also, in a container for specimen of the present invention, it is preferred that the specimen-picking rod is provided with a concave picking portion on the front end portion of the specimen-picking rod and the container body is provided with a convex removing portion which is placed on the bottom portion of the container body and can be inserted into the concave picking portion. As a result, a specimen can be quantitatively collected by the concave picking portion and, in addition, the insertion of the convex removing portion into the concave picking portion makes it easy to remove a specimen picked by the concave picking portion from the concave picking portion. Accordingly, it is possible to use a quantified collection amount of a specimen for test.

Besides, it is preferred that the concave picking portion and convex removing portion are formed in such a way that the concave picking and convex removing portions can be fitted to each other and can be separated from each other from a state in which the concave picking portion is fitted to the convex removing portion. As a result, when the concave picking portion is provided with a passage which is described below and connects the concave picking portion to the external peripheral surface of the specimen-picking rod, it is possible to quantitatively remove a specimen which is picked by the concave picking portion in fitting the concave picking portion to the convex removing portion.
Also, when the concave picking portion can be separated from the convex removing portion from a state in which the concave picking portion is fitted to the convex removing portion, it is possible to remove the specimen-picking portion from the container body in picking a specimen using the specimen-picking rod or in attaching the container body to a general-purpose apparatus for physical and chemical analysis after the specimen is removed to the container body.

Besides, the convex removing portion may be freely fitted to the concave picking portion. As a result, it is possible to remove a specimen to the container body from the space between the concave picking and convex removing portions.

Also, in a container for specimen of the present invention, it is preferred that the specimen-picking rod is provided with a passage which connects the concave picking portion to the external peripheral surface of the specimen-picking rod and a specimen passes through the passage to be pressed out to the outside when the convex removing portion is inserted into the concave picking portion. As a result, a specimen which is quantitatively collected by the concave picking portion becomes easy to remove to the container body through the convex removing portion. Besides, it is supposed that a part of a specimen remains in the passage when the specimen is pressed out through the convex removing portion. With respect to this matter, the concave picking portion is designed with an amount of a part of specimen remaining in the passage taken into account in advance, so that it is possible to remove to the container body a collection amount of a specimen which is quantified within a desired permissible range and is necessary for test.
It is preferred that the passage which connects the concave picking portion of the specimen-picking rod to the external peripheral surface of the specimen-picking rod is formed, for example, as a plurality of the slits which connect the side surface of the concave picking portion to the external peripheral surface of the specimen-picking rod. Also, for example, the passage may be formed as a plurality of press-out holes which connect the bottom surface of the concave picking portion to the external peripheral surface of the specimen-picking rod.

Also, in a container for specimen of the present invention, it is preferred that the specimen-picking rod is provided with a picking portion which is composed of a lattice-shaped groove and is provided on the external peripheral surface of the front end of the specimen-picking rod.
In the case where a specimen is a gel-like and soft object, the specimen is difficult to fill up to the recesses of the concave picking portion even though the concave picking portion as described above is used for picking the specimen and, in addition, the picked specimen easily escapes from the opening of the concave picking portion. On the other hand, when the picking portion of the specimen-picking rod is formed as a lattice-like groove which is provided on the external peripheral surface of the front end portion of the specimen-picking rod, a gel-like specimen easily adheres to the picking portion of the specimen-picking rod and is easy to pick quantitatively.

Also, a gel-like specimen is easily dispersed in a liquid of the container body to easily make the liquid turbid. Accordingly, even though the lattice-like groove is used for picking the gel-like specimen, it is difficult for the gel-like specimen to remain in the groove. As a result, it is possible to quantitatively remove the gel-like specimen to the container body. Besides, a surplus part of the specimen which adheres to the side surface of the specimen-picking rod except the lattice-like groove is removed from the specimen-picking rod when the specimen-picking rod passes through the through hole of the penetration wall, so that there is no hindrance to the quantification of the amount of the specimen removed to the container body. However, a gel-like and soft specimen has weak adhesive force to the lattice-like groove. Accordingly, if the external peripheral surface on which the lattice-like groove is provided slides on the penetration wall with the external peripheral surface coming into contact with the penetration wall to the extent that the penetration wall is deformed when the external peripheral surface having the lattice-like groove passes through the through hole of the penetration wall, then a part of the specimen adhering to the lattice-like groove also adheres to the through hole, so that there is a fear that the adhesion of the specimen picked by the lattice-like groove to the through hole hinders the quantification of the amount of the specimen. Accordingly, in the case where the picking portion is formed as lattice-like groove which is provided on the external peripheral surface of the front end portion of the specimen-picking rod for the purpose of collecting a gel-like specimen, it is preferred that the diameters of the through hole of the penetration wall and the specimen-picking rod are designed so that specimen-picking rod slides on the through hole without deforming the penetration wall.

Or, in a container for specimen of the present invention, it is preferred that the specimen-picking rod is provided with a brush-shaped picking portion on the front end portion of the specimen-picking rod. As a result, for example, it is possible to pick specimens having some degree of viscosity such as uterine mucosa and oral mucosa.

Or, in a container for specimen of the present invention, it is preferred that the specimen-picking rod is formed in such a way that: at least the front end portion of the specimen-picking rod can be opened and closed; the specimen-picking rod is provided with a separation portion which is formed so as to hold a predetermined amount of a specimen in a state of the specimen-picking rod closed; and the through hole is penetrated by the specimen-picking rod which slides on the through hole with the separation portion closed. As a result, when the separation portion is closed so that a specimen is pinched by the specimen-picking rod to be picked, the specimen-picking rod is easy to insert into a specimen like stool without applying large force to the specimen-picking rod, for example, in the case of collecting a stool which is excreted on a sheet floating on water in a bowl. Accordingly, such specimen-picking rod makes it easy to pick a sufficient amount of a stool to detect a cancer cell or the like even though the stool is hard.

In addition, when a container for specimen of the present invention is formed in such a way that: the separation portion holds a predetermined amount of a specimen in a state of the separation portion closed; and the through hole is penetrated by the specimen-picking rod which slides on the through hole with the separation portion closed, the specimen can be quantitatively collected and be preserved in the container body.

And, in a container for specimen of the present invention in which the constitution of the above-described separation portion of the present invention is more embodied, the specimen-picking rod is provided with a forked portion at least the front end of which can open and close and which holds a predetermined amount of a fragmented specimen to be examined in a state of the forked portion closed, and the through hole is penetrated by the specimen-picking rod which slides on the through hole with the forked portion closed.

As described above, when the forked portion is closed so that a specimen is pinched by the specimen-picking rod to be picked, the specimen-picking rod is easy to insert into a specimen like stool without applying large force to the specimen-picking rod, for example, in the case of collecting a stool which is excreted on a sheet floating on water in a bowl. Accordingly, such specimen-picking rod makes it easy to pick a sufficient amount of a stool to detect a cancer cell or the like even though the stool is hard.

Also, when the forked portion holds a specimen with the specimen fragmented in a state of the forked portion closed, the specimen picked by the specimen-picking rod becomes easy to disperse in a liquid of the container body.
In addition, when a container for specimen of the present invention is formed in such a way that the forked portion holds a predetermined amount of a specimen in a state of the forked portion closed and the through hole is penetrated by the specimen-picking rod which slides on the through hole with the forked portion closed, the specimen can be quantitatively collected and be preserved in the container body.

In a more detailed explanation, for example, the forked portion is provided with at least one specimen-holding portion which is placed on at least one of the branching portions of the forked portion and includes a specimen-introducing inlet and specimen-exhausting outlet, where the specimen-introducing inlet is provided on the side of the one branching portion through which the one branching portion touches the other branching portion of the forked portion in closing the forked portion and the specimen-exhausting outlet is provided on the opposite side to the side on which the specimen-introducing inlet is provided.
As a result, a specimen can be fragmented up to enough size to easily disperse the specimen in a liquid of the container body through the specimen-holding portion. Also, a surplus part of a specimen can be exhausted through the specimen-exhausting outlet. Accordingly, when the specimen-picking rod is inserted into the through hole with the forked portion closed, a specimen can be quantitatively collected and be preserved in the container body.

Also, for example, a container for specimen of the present invention is formed in such a way that the front end of one of the branching portions of the forked portion is provided with at least one groove which is formed by placing at least two thin plate-shaped plane portions along the direction of the forked portion opening or closing and the front end of the other branching portion of the forked portion is provided with a shield portion which is composed of a thin plate-shaped plane portion that protrudes towards the one-branching-portion side so that the thin plate-shaped plane portion covers the opening side of the groove along the direction of the forked portion opening or closing when the forked portion is closed.

As a result, when the forked portion is closed so that the shield portion covers the opening side of the groove along the direction of the forked portion opening or closing, the opening portion of the groove on the closed side of the forked portion functions as the specimen-introducing inlet, the opening portion of the groove on the opened side of the forked portion functions as the specimen-exhausting outlet, and a space which is surrounded by the groove and shield portion functions as the specimen-holding portion. Accordingly, a specimen can be fragmented up to enough size to easily disperse the specimen in liquid of the container body. Also, a surplus part of a specimen can be exhausted through the specimen-exhausting outlet on the opened side of the forked portion in the groove. Accordingly, when the specimen-picking rod is inserted into the through hole with the forked portion closed, a specimen can be quantitatively collected and be preserved in the container body.

Also, the shield portion is provided with a thin plate-shaped plan portion that protrudes towards the groove-having-one-branching-portion side so that the shield portion covers the opening side of the groove along the direction of the forked portion opening or closing. As a result, when the forked potion is closed so that a specimen is pinched by the specimen-picking rod to be picked, the specimen can be scooped with the lower portion of the specimen supported by the shield portion. Accordingly, a specimen like stool becomes easy to pick surely in the case of collecting a stool which is excreted on a sheet floating on water in a bowl.
In addition, when the forked portion is opened inside the container body, the shield portion does not cover the opening side of the groove along the direction of the forked portion opening or closing, so that a specimen like stool which is picked by the groove can have a large area of the surface of the specimen that comes in contact with liquid in the container body. As a result, a specimen which is picked by the specimen-picking rod becomes yet easier to disperse in liquid of the container body.

Also, for example, a container for specimen of the present invention is formed in such a way that: the front end of one of the branching portions of the forked portion is provided with at least one groove which is formed by placing at least two thin plate-shaped plane portions along the direction of the forked portion opening or closing; the other branching portion of the forked portion is provided with a specimen-holding portion which includes a specimen-introducing inlet that is provided on the side of the other branching portion through which the other branching portion touches the one branching portion in closing the forked portion and a specimen-exhausting outlet that is provided on the opposite side to the side on which the specimen-introducing inlet is provided; and the front end of the other branching portion of the forked portion is provided with a shield portion which is composed of a thin plate-shaped plane portion that protrudes towards the one-branching-portion side so that the shield portion covers the opening side of the groove along the direction of the forked portion opening or closing when the forked portion closed.
As a result, the container for specimen has both the constitution including the above specimen-holding portion and the constitution including the above groove and the above shield portion, so that the same effect is obtained by each of the constitutions including a specimen-holding portion and including the groove and the shield portion.

Also, in a container for specimen of the present invention, it is preferred that the at least one groove is formed in such a way that at least one of thin plate-shaped plane portions forming the groove protrudes towards the the-other-branching-potion side so that the thin plate-shaped plane portion is inserted into the specimen-holding portion when the forked portion is closed.
As a result, the insertion of the thin plate-shaped plane portion into the specimen-holding portion makes it possible to yet more fragment a specimen that is fragmented by the specimen-holding portion holding the specimen, so that the specimen which is picked by the specimen-picking rod becomes yet easier to disperse in liquid of the container body.

Also, in a container for specimen of the present invention, it is preferred that the thin plate-shaped plane portions forming the groove are formed in such a way that the thin plate-shaped plane potions become thinner and thinner towards the side on which the one branching portion of the forked portion touches the other branching portion of the forked portion when the forked portion is closed.
As a result, a specimen becomes easy to introduce into the groove in the direction from the opening portion of the groove on the forked portion-closing side to the opening portion of the groove on the forked portion-opening side when the forked portion is closed. Also, in the constitution in which the thin plate-shaped plane portion is inserted into the specimen-holding portion, the insertion of the thin plate-shaped plane portion into the specimen-holding portion makes it possible to yet more fragment a specimen that is fragmented by the process of the specimen-holding portion holding the specimen, so that the specimen which is picked by the specimen-picking rod becomes yet easier to disperse in liquid of the container body.

Also, in a container for specimen of the present invention, it is preferred that: the forked portion of the specimen-picking rod is formed so as to have such a behavior that the opening of the forked portion is accompanied by elastic force; and, in addition, the external peripheral surface of the specimen-picking rod is provided with an annular member which can be moved along the longitudinal direction, is moved towards the front end by a predetermined distance to make the forked portion close, and is moved towards the rear end by a predetermined distance to release the forked portion which is in a closed state.
This way makes it easy to open and close the forked portion.
Also, in a container for specimen of the present invention, it is preferred that the specimen-picking rod is provided with an annular member-fixing means by which the annular member is fixed at a predetermined position at which the forked portion is in an open state.

In the above-described container for specimen of the present invention, the cover member connecting with the rear end of the specimen-picking rod is connected to the container body through the intermediary unit in an initial state of the container for specimen before collecting a specimen. And, when a specimen is collected, the cover member is removed from the intermediary unit. In this case, a user must grasp the container body with one hand of the user while grasping the cover member with the other hand of the user. As a result, in the case where the annular member is movably provided on the external peripheral surface of the specimen-picking rod, the user cannot hold the annular member with a user's hand when the user removes the cover member from the container body, so that there is a fear that the annular member comes out of the specimen-picking rod to fall downward.

however, as described above, if the specimen-picking rod is provided with the annular member fixing means, then the annular member can be fixed to the specimen-picking rod in the initial state, so that it is possible to prevent the annular member from coming out of the specimen-picking rod to fall downward even though the annular member is not held with a user's hand in removing the cover member from the container body and the specimen-picking rod can be kept open. Accordingly, the user can easily undertake the collection of a specimen.

Besides, when the user collects a specimen, the user moves the annular member towards the direction of the forked portion closed (or, towards the front end) while holding the annular portion with a user's hand. As a result, it is possible to prevent the annular member from coming out of the specimen-picking rod to fall downward. In addition, after collecting the specimen, the user connects the cover member to the container body through the intermediary unit while grasping the cover member with one hand of the user and grasping the container body with the other hand of the user. In this case, the annular member is supported by the penetration wall around the through hole in the intermediary unit even though the annular member moves on the specimen-picking rod, so that it is possible to prevent the annular member from coming out of the specimen-picking rod to fall downward.

Also, in a container for specimen of the present invention, it is preferred that: the specimen-picking rod includes a flange at the rear end of the specimen-picking rod; the trunk portion of the specimen-picking rod which runs from the base of the forked portion to the rear end is formed as a hollow part and, in addition, the specimen-picking rod includes a pair of guide grooves consisting of a groove with a T-shaped cross section which is provided for each of the branching portions along the longitudinal direction of the specimen-picking rod and on the side on which the one branching portion of the forked portion touches the other branching portion of the forked portion when the forked portion is closed and a second groove which continues to the one edge of the T-shaped groove; the cover member includes a holding portion by which the flange at the rear end of the specimen-picking rod is rotatably held, a shaft-shaped male screw which is fixed in the middle of the cover member and runs towards the front end of the specimen-picking rod with the male screw having a predetermined length, a female screw into which the male screw is screwed and the diameter of which has such a size that the second grooves of the both guide grooves can hold the female screw when the male screw is screwed into the female screw up to a predetermined position and the forked portion is closed, and T-shaped portions which are placed on the external peripheral surface of the female screw and at positions at which the T-shaped portions are opposite to each other and which are freely fitted to the T-shaped grooves of the guide grooves in the forked portion respectively; and the rotation of the cover member relative to the specimen-picking rod makes the forked portion open and close.

As a result, when the cover member is rotated with the specimen-picking rod fixed to the cover member, the forked portion can be opened or closed. For example, as the cover member is rotated more and more in the direction in which the cover member is screwed into the intermediary unit connecting with the container body, the female screw is pushed up through the male screw more and more, so that the forked portion is opened. In addition, as the cover member is rotated more and more in the direction in which the cover member is unfastened from the intermediary unit connecting with the container body, the female screw is pushed down through the male screw more and more, so that the forked portion is closed. Accordingly, even though the forked portion has no elasticity, it is possible to obtain the same effect as that of constitution including the forked portion having elasticity, as described above.

Also, in a container for specimen of the present invention, it is preferred that: the specimen-picking rod is connected with at least one headed pin-shaped connection member at the middle portion of the specimen-picking rod and can open and close with the shaft of the headed pin-shaped connection member as a fulcrum; the portion running from the front end of the specimen-picking rod to the fulcrum is composed of two members which form the forked portion, a first member of the two members includes a first nick portion which runs from the rear end to the vicinity of the fulcrum, and a second member of the two members includes a second nick portion which runs from the rear end to the vicinity of the fulcrum and into which the portion of the first member running from the rear end of the first member to the vicinity of the fulcrum can be slidably inserted; and the cover member includes a shaft-shaped supporting member which is fixed in the middle of the cover member, has a proper diameter and a length so that the supporting portion can be slidably inserted into the first nick portion of the first member, and includes an annular groove that can hold the head of the headed pin-shaped connection member connecting the two members in the circumferential direction, and a groove which is formed in the internal surface of the cover member so as to be slidably fitted to the rear ends of the two members and is formed in the shape of a swirl converging to the center of the cover member.

This way also makes it possible to open and close the forked portion when the cover member is rotated with the specimen-picking rod fixed to the cover member. For example, as the cover member is rotated more and more in the direction in which the cover member is screwed into the intermediary unit connecting with the container body, the rear ends of the two members of the forked portion move more and more on the swirl-shaped groove in the direction in which the swirl-shaped groove curves with the swirl-shaped groove having a large diameter, so that the forked portion is opened with the shaft of the headed pin-shaped connection member as a fulcrum. In addition, as the cover member is rotated more and more in the direction in which the cover member is unfastened from the intermediary unit connecting with the container body, the rear ends of the two members of the forked portion move more and more on the swirl-shaped groove in the direction in which the swirl-shaped groove converges, so that the forked portion is closed with the shaft of the headed pin-shaped connection member as a fulcrum. Accordingly, even though the forked portion has no elasticity, it is possible to obtain the same effect as that of constitution including the forked portion having elasticity, as described above.

Also, in a container for specimen of the present invention, it is preferred that the container for specimen includes a connection means which connects the intermediary unit with the convex picking portion. As a result, when the intermediary unit is removed from the container body, it is possible to remove the convex picking portion together with the intermediary unit. For example, it is preferred that the connection means includes: an engaging member which is fixed on the inside of the connection portion; and a frame member which is formed in the shape of a tube so as to be capable of being contained inside the container body, the bottom portion of which the convex picking portion is fixed on, and the upper portion of which can engage with the engaging member.

Also, in a container for specimen of the present invention, it is preferred that the specimen-picking rod includes: an absorbing portion which is placed at the end of the specimen-picking rod and is spongy; a tube member which connects with the absorbing portion; and a rod member which moves inside the tube member, the front end portion of which can push the absorbing portion, and the other end of which connects with the cover member. As a result, it is possible to collect specimens which are high in water and have viscosity, such as sputum, urine, and blood.

Besides, in a container for specimen of the present invention, it is preferred that threads are formed in the internal peripheral portion of one end of the connection portion in the intermediary unit and in the external peripheral portion of the open end of the container body so that the open end of the container body is screwed into the one end of the connection portion of the intermediary unit through the threads. As a result, the intermediary unit can be removably fitted to the container body without deforming one end of the connection portion of the intermediary unit relative to the open end of the container body.

Also, for example, a container for specimen of the present invention may be formed in such a way that the external peripheral portion of the one end of the connection portion in the intermediary unit is provided with: at least one annular convex portion which is formed along the penetration direction; an annular stopper portion the diameter of which is larger than the internal peripheral portion of the open end of the container body; and an O-ring which is placed on the container-body side of the stopper member and that the internal peripheral portion of the open end of the container body is provided with an annular concave portion which can be fitted to the annular convex portion. As a result, the intermediary unit can be removably fitted to the container body without rotating the one end of the connection portion of the intermediary unit relative to the open end of the container body.

Also, for example, a container for specimen of the present invention may be formed in such a way that: the edge portion of the one end of the connection portion in the intermediary unit is provided with an annular groove which is formed so as to be capable of being fitted to the open end of the container body, an O-ring which is placed on the bottom of the annular groove, and a plurality of opening portions which are provided on the circumference of the connection portion of the intermediary unit so as to link the annular groove and the external peripheral portion of the one end of the connection portion of the intermediary unit; and the external peripheral portion of the open end of the container body is provided with a plurality of catch portions which can be inserted into the opening portions of the connection portion of the intermediary unit and are formed in such a way that the catch portions break off at the bases when the catch portions are inserted into the opening portions and force is applied in a set direction crossing the insertion direction. As a result, also in this case, the intermediary unit can be removably fitted to the container body without rotating the one end of the connection portion of the intermediary unit relative to the open end of the container body.

Also, for example, a container for specimen of the present invention may be formed in such a way that: the edge portion of the one end of the connection portion in the intermediary unit is provided with an annular groove which is formed so as to be capable of being fitted to the open end of the container body, an O-ring which is placed on the bottom of the annular groove, and a plurality of opening portions which are provided on the circumference of the intermediary unit so as to link the annular groove and the external peripheral portion of the one end of the connection portion of the intermediary unit; and the external peripheral portion of the open end of the container body is provided with a plurality of catch portions which can be inserted into the opening portions of the connection portion of the intermediary unit and are formed in such a way that the catch portions have slanted surfaces so that the catch portions come out of the opening portions when force is applied so as to move the intermediary unit in the direction opposite to the direction toward the container-body side with the catch portions inserted into the opening portions. As a result, also in this case, the intermediary unit can be removably fitted to the container body without rotating one end of the connection portion of the intermediary unit relative to the open end of the container body. In addition, the intermediary unit becomes easy to remove from the container body.

Also, in a container for specimen of the present invention, it is preferred that the connection portion of the intermediary unit, the cover member, and container body are made of elastic material, the elastic modulus of the connection portion of the intermediary unit is smaller than those of the penetration wall and the cover member and is larger than that of the container body, and the elastic modulus of the cover member is smaller than that of the penetration wall.
As a result, when a specimen is collected, the cover member connecting with the specimen-picking rod becomes easy to remove from the intermediary unit with the intermediary unit kept fitted to the container body. Also, when the specimen-picking rod is made to penetrate the penetration wall of the intermediary unit to be inserted into the container body after collecting the specimen, the deformation of the intermediary unit can be suppressed to the utmost. Also, also when the cover member is fitted to the intermediary unit, the deformation of the intermediary unit can be suppressed. In addition, the intermediary unit becomes easy to remove from the container body.

Also, in a container for specimen of the present invention, it is preferred that the connection portion of the intermediary unit, the cover member, and container body are made of elastic material, the elastic modulus of the connection portion of the intermediary unit is smaller than that of the penetration wall and is larger than those of the cover member and the container body.
As a result, also in this case, when a specimen is collected, the cover member connecting with the specimen-picking rod becomes easy to remove from the intermediary unit with the intermediary unit kept fitted to the container body. Also, when the specimen-picking rod is made to penetrate the penetration wall of the intermediary unit to be inserted into the container body after collecting the specimen, the deformation of the intermediary unit can be suppressed to the utmost. Also, when the cover member is fitted to the intermediary unit, the deformation of the cover member can be suppressed. In addition, the intermediary unit becomes easy to remove from the container body.

Also, in a container for specimen of the present invention, it is preferred that threads are provided on the external peripheral portion of the other end of the connection portion of the intermediary unit and on the internal peripheral portion of the cover member, and the other end of the connection portion of the intermediary unit is screwed into the cover member through the threads. As a result, the cover member can be removably fitted to the intermediary unit without deforming both the cover member and the other end of the connection portion of the intermediary unit.

Also, in a container for specimen of the present invention, it is preferred that torque by which the other end of the connection portion of the intermediary unit is screwed into the cover member is smaller than torque by which the open end of the container body is screwed into the one end of the connection portion of the intermediary unit. As a result, when the cover member connecting with the specimen-picking rod is removed from the intermediary unit in order to collect a specimen, it is possible to prevent the intermediary unit from coming out of the container body.

Also, in a container for specimen of the present invention, it is preferred that the specimen-picking rod is formed so as to be removably fitted to the cover member. As a result, an optimum collection of a specimen can be performed by fitting the specimen-picking rod which is formed in a suitable formation for collecting of a specimen in accordance with the shape, hardness, viscosity and so on of the specimen.

Also, in a container for specimen of the present invention, it is preferred that the threads are formed in the internal peripheral portion of the one end of the connection portion of the intermediary unit, in the external peripheral portion of the open end of the container body, in the external peripheral portion of the other end of the connection portion of the intermediary unit, and in the internal peripheral portion of the cover member respectively in such a way that the opening or closing direction in rotating the intermediary unit in a set direction through the threads formed in the internal peripheral portion of the one end of the connection portion of the intermediary unit and in the external peripheral portion of the open end of the container body is opposite to the opening or closing direction in rotating the intermediary unit in a set direction through the threads formed in the external peripheral portion of the other end of the connection portion of the intermediary unit and in the internal peripheral portion of the cover member. As a result, for example, the rotational direction in rotating the cover member so as to unfasten the cover member from the intermediary unit in order to collect a specimen coincides with the rotational direction in rotating the intermediary unit so as to fasten the intermediary unit to the container body. In addition, the rotational direction in rotating the cover member so as to fasten the cover member to the intermediary unit after collecting the specimen coincides with the rotational direction in rotating the intermediary unit so as to unfasten the intermediary unit from the container body. Accordingly, by only a change in the rotational direction of the cover member, the cover member can be removed from the intermediary unit with the intermediary unit kept fitted to the container body in collecting a specimen while the intermediary unit can be removed from the container body with the cover member kept fitted to the intermediary unit in testing the specimen with a general-purpose testing apparatus after removing the collected specimen to the container body.

Also, in a container for specimen of the present invention, it is preferred that the cover member is provided with a grasping portion.
In the present invention, the grasping portion means a portion which is formed so as to be capable of being grasped in order to make the cover member easy to rotate. For example, it is preferred that the grasping portion is formed in the shape of a rectangle or polygon, in the D-Cut shape, or the like so that at least a part of the cover member has an approximately plane surface, or is formed in the shape by which at least a part of the cover member has a convex or concave portion. As a result, in the case where the cover member can be removably fitted to the intermediary unit through threads, the cover member becomes easy to rotate through the grasping portion, so that the cover member becomes easy to removably fitted to the intermediary unit.

In addition, in the case where the opening or closing direction in rotating the intermediary unit in a set direction through the threads which are formed in the internal peripheral portion of the one end of the connection portion of the intermediary unit and in the external peripheral portion of the open end of the container body is opposite to the opening or closing direction in rotating the intermediary unit in a set direction through the threads which are formed in the external peripheral portion of the other end of the connection portion of the intermediary unit and in the internal peripheral portion of the cover member, the grasping portion makes it easy to remove the cover member from the intermediary unit with the intermediary unit kept fitted to the container body in collecting a specimen and to remove the intermediary unit from the container body with the cover member kept fitted to the intermediary unit in testing the collected specimen with a general-purpose testing apparatus after removing the specimen to the container body.

Also, in a container for specimen of the present invention, it is preferred that the outer diameter of the cover member is larger than that of the connection portion of the intermediary unit and the outer diameter of the connection portion of the intermediary unit is larger than that of the container body. As a result, in the case where the cover member and the intermediary unit can be removably fitted to each other through threads and the intermediary unit and the container body can be removably fitted to each other through threads, it is possible to make force for removal from the intermediary unit weaker than the minimum force necessary for unfastening the intermediary unit from the container body. Accordingly, the intermediary unit can be given an action of the intermediary unit not coming out of the container body together with the cover member in removing the cover member from the intermediary unit. In addition, when the connection portion of the intermediary unit is formed in such a way that the outer diameter of the connection portion of the intermediary unit is larger than that of the container body, the intermediary unit becomes easy to remove from the container body.

Also, in a container for specimen of the present invention, it is preferred that the cover member is different from the connection portion of the intermediary unit in material. As a result, the air-tightness of the container becomes easy to keep and the cover member becomes easy to removably fit to the intermediary unit.

Also, in a container for specimen of the present invention, it is preferred that the container body is different from the connection portion of the intermediary unit in material. As a result, the air-tightness of the container becomes easy to keep and the intermediary unit becomes easy to removably fit to the container body.

Also, in a container for specimen of the present invention, it is preferred that the internal surface of the cover member which is provided with the swirl groove is formed in the shape of a cone. As a result, the opening and closing operation of the forked portion which is related to the rotation of the cover member can be made to become all the slower because the radius of the back surface of the cover member, or the length of the groove which is formed in the shape of a swirl running from the center to the edge becomes large. Accordingly, a specimen becomes easy to pick.

Also, in a container for specimen of the present invention, it is preferred that: the internal surface of the cover member is formed in the shape of a cone and is provided with a groove which is formed in the shape of a spiral converging to the center of the cover member and has a T-shaped cross section; the specimen-picking rod is connected with at least one headed pin-shaped connection member at the middle portion of the specimen-picking rod and can open and close with the shaft of the headed pin-shaped connection member as a fulcrum; and the portion running from the front end of the specimen-picking rod to the fulcrum is composed of two members which form the forked portion, the two members are formed in the shapes that enable the two members to overlap with each other over the range from the rear end to the vicinity of the fulcrum, the inside surfaces of the forked portion are formed in the shapes that enable the inside surfaces of the forked portion to abut each other, and the rear ends of the two members are provided with T-shaped portions which are freely fitted to the T-shaped groove of the cover member; and the rotation of the cover member relative to the specimen-picking rod makes the forked portion open and close.

As a result, also in this case, when the cover member is rotated with the specimen-picking rod fixed to the cover member, the forked portion can be opened and closed. For example, as the cover member is rotated more in the direction in which the cover member is screwed into the intermediary unit connecting with the container body, the T-shaped portions on the rear ends of the forked portion move more in the spirally-formed and T-shaped groove in the direction in which the T-shaped portions gradually curve with a large diameter, so that the forked portion can be opened. In addition, as the cover member is rotated more in the direction in which the cover member is unscrewed from the intermediary unit connecting with the container body, the T-shaped portions on the rear ends of the forked portion move more in the spirally-formed and T-shaped groove in the direction in which the T-shaped portions gradually curve with a small diameter, so that the forked portion can be closed. Accordingly, even though the forked portion has no elasticity, it is possible to obtain the same effect as that of the constitution including the forked portion having elasticity, as described above.

Also, in a container for specimen of the present invention in which the constitution of the separation portion in the container body of the present invention is embodied more, the specimen-picking rod includes a plurality of vertical divisions which can open and close at least on the front end of the specimen-picking rod and hold a set amount of specimen when the vertical divisions are closed, a plurality of the vertical divisions are formed in such a way that at least a part of each of the vertical divisions expands towards the outside more and more towards the front end with the parts of the vertical divisions curving, and the specimen-picking rod slides on the through hole to penetrate through the through hole with a plurality of the vertical divisions closed. As a result, when the separation portion is closed so that a specimen is pinched by the separation portion to be collected, the specimen-picking rod is easy to insert into a specimen like stool without applying large force to the specimen-picking rod, for example, in the case of collecting a stool which is excreted on a sheet floating on water in a bowl. Accordingly, such specimen-picking rod makes it easy to collect a sufficient amount of a stool to detect a cancer cell or the like even though the stool is hard.

Also, when a plurality of the vertical divisions are formed in such a way that at least a part of each of the vertical divisions expands towards the outside more and more towards the front end with the parts of the vertical divisions curving, a specimen like a stool that is excreted on a sheet floating on water in a bowl becomes yet easier to collect. Also, it is possible to collect a specimen on the surface of a stool or the like that widely extends, so that a probability that cells on the surface of a specimen can be collected becomes high. Also, it is also possible to collect both the surface and inside of a specimen at the same time. Also, a plurality of the vertical divisions can be used as a stirrer, so that a specimen becomes easy to mix with a solution in the container body. In addition, a plurality of the vertical divisions are made to hold a set amount of specimen in a state of the vertical divisions closed, and the specimen-picking rod is made to slide on the through hole to penetrate through the through hole with a plurality of the vertical divisions closed. As a result, a specimen can be quantitatively collected and be preserved in the container body.

Also, in a container for specimen of the present invention, it is preferred that the front end of each of a plurality of the vertical divisions bends inward so as to be capable of holding a set amount of specimen when the vertical divisions are closed. As a result, a specimen becomes easy to collect and the collected specimen becomes easy to hold in the specimen-picking rod. Also, when a plurality of the vertical divisions are closed, the inside of the vertical divisions can have a predetermined space, so that a collection amount of the specimen becomes easy to quantify into a predetermined amount. Also, even though a specimen is hard, the inward-bent front ends of the vertical divisions are made to bite into the specimen by closing a plurality of the vertical divisions, so that the specimen can be pinched to be collected. Also, in the case where a specimen is hard and shaped like a small grain, the specimen can be pinched by the inward-bent front ends of the vertical divisions to be collected.

Also, with respect to a more embodied constitution in a container for specimen of the present invention, it is preferred that: the specimen-picking rod includes a tube portion which connects with the cover member, and a cylinder portion which is inserted into the tube portion; and a plurality of the vertical divisions are placed on the front end of the cylinder portion respectively and are formed so as to expand towards the outside more and more towards the front end with the vertical divisions curving when the vertical divisions protrude from the tube portion to the outside.

Also, it is preferred that the specimen-picking rod is formed in such a way that the cylinder portion can slide with the specimen-picking rod kept air-tightness when the vertical divisions are contained in the tube portion. As a result, when a plurality of the vertical divisions on the front end of the cylinder portion are moved into the tube portion with the vertical divisions closed, the specimen-picking rod can function as a syringe, so that a specimen having a low viscosity can be sucked into the tube portion. As a result, it is possible to collect specimens which have various hardness and viscosity, such as liquid specimen together with hard and soft specimens

Also, in the container for specimen having the above-described constitution, it is preferred that the specimen-picking rod includes a reagent-holding portion which is placed on the cylinder portion.

Even though the above-described fecal occult blood test has been used widely, it has been known that a patient suffering from hemorrhoid also may test positive for cancer by the above-described fecal occult blood test and results of the above-described fecal occult blood test are liable to become false-positive for colorectal cancer. Accordingly, the above-described fecal occult blood test requires the detection of tumor markers in the stool, cancer cells which are dissociated in the stool, or the like by extracting nucleic acids from a stool with a general-purpose apparatus for physical or chemical analysis or a nucleic acid extracting apparatus like the above-described centrifuge. However, stools contain only trace amounts of tumor markers and dissociated cancer cells and, in addition, tumor markers and dissociated cancer cells are easily broken down by an enzyme or the like in the stool, so that a specimen must be frozen or treated with a reagent immediately after collecting the specimen.

Accordingly, when the cylinder portion of the specimen-picking rod is provided with a reagent-holding portion as in the refined container for specimen of the present invention, a specimen which is collected by the specimen-picking rod can be removed to the container body while being treated with a reagent after collecting the specimen, without filling the container body with a reagent. As a result, tumor markers in a specimen, cancer cells dissociated in a specimen, and so on can be preserved with the tumor markers, the cancer cells, and so on in a stable state. Also, when the first reagent is contained in the container body and the second reagent is contained in the reagent-holding rod, a specimen which is collected by the specimen-picking rod can be treated with the second reagent immediately after treating the specimen with the first reagent by removing the collected specimen to the container body, so that the treatment of a specimen with reagent can be performed quickly and efficiently.

Besides, it is preferred that the cylinder portion is composed of a rear end portion, a front end portion the front end of which is provided with a plurality of the vertical divisions, and a support portion which connects the rear end portion and the front end portion. And, in a more concrete constitution of the cylinder portion, it is preferred that: the support portion is formed in such a way that the support portion has a small diameter; the reagent-holding portion is formed as the space which is surrounded by the tube portion, the rear end portion of the cylinder portion, the support portion of the cylinder portion, and the front end portion of the cylinder portion; the front portion of the cylinder portion includes a plurality of the vertical divisions and a base portion holding a plurality of the vertical divisions; and the base portion plays a role as a stopper of the reagent-holding portion for the tube portion. Also, in another more concrete constitution of the cylinder portion, it is preferred that: the support portion is formed as a hollow part; the reagent-holding portion is formed as the space which is surrounded by the rear end portion of the cylinder portion, the hollow connection portion of the cylinder portion, and the front end portion of the cylinder portion; and a part of the hollow connection portion is provided with an opening for exhausting a reagent.

Also, in a refined container for specimen of the present invention, it is preferred that the rear end portion of the cylinder portion is provided with a stopper portion which is formed in such a way that the stopper portion restrains the movement of the cylinder portion towards the front end in the tube portion in order to keep the air-tightness of the reagent-holding portion and the restraint of the movement of the cylinder portion can be released by applying a predetermined amount or more of pressing force to the stopper portion towards the front end. As a result, it is possible to prevent a reagent from carelessly escaping to the outside of the specimen-picking rod to cause contamination at the stage where the container body is not made to contain the specimen-picking rod holding a specimen.

Also, in this case, it is preferred that: the container for specimen includes a removable outer cover which is placed on the outside of the intermediary unit on the cover-member side with the intermediary unit connecting the container body with the cover member; the intermediary unit includes a first thread portion which is provided on the external peripheral portion of the intermediary unit on the cover-member side and can be threadedly engaged with the outer cover; the outer cover includes a second thread portion which is provided on the internal peripheral portion of the outer cover and can be threadedly engaged with the intermediary unit; and the container for specimen is provided with a restraint-releasing portion which applies a predetermined amount or more of pressing force to the stopper portion to release restraint of the movement of the cylinder portion towards the front end in the tube portion by the stopper portion when the second thread portion of the outer cover is threadedly engaged with the first thread portion of the intermediary unit. As a result, the attachment of the outer cover to the intermediary unit can more surely prevent a specimen or reagent from leaking to the outside in stirring or carrying a specimen with the specimen contained in the container body.

Also, when the restraint of the movement of the cylinder portion towards the front end in the tube portion by the stopper portion is released by threadedly engaging the second thread portion of the outer cover with the first thread portion of the intermediary unit, a user does not have to directly press the specimen-picking rod with large force because the restraint of the movement of the cylinder portion towards the front end in the tube portion by the stopper portion has been released. As a result, it is also possible to prevent a reagent from spattering because of the reaction from the press of the specimen-picking rod.

Next, embodiments of the present invention will be explained using drawings.

### First Embodiment

Fig. 1 schematically shows the constitution of a container for specimen according to a first embodiment of the present invention, and Fig. 2 shows one example of the constitution of a main part of the container for specimen which is shown in Fig. 1. The constitutions except the connection portion of the intermediary unit and the container body do not shown in Fig. 2 for convenience.

As shown in Fig. 1 (a), the container for specimen of the first embodiment comprises a container body 1, a specimen-picking rod 2 for picking a specimen, a cover member 3, and an intermediary unit 4.
The container body 1 is formed in such a way that: one end of the container body 1 is open; and the container body 1 can hold a liquid 5 for dispersing or preserving a specimen and is formed in the shape of a tube having a size and shape which make it possible to mount the container body 1 in a general-purpose apparatus for physical and chemical analysis like a general-purpose nucleic acid-extracting apparatus. A thread 1a₁ is formed in the external peripheral portion 1a of the open end of the container body 1. In addition, a convex removing portion 6 is provided to be fixed on the bottom portion of the container body 1. The specimen-picking rod 2 includes a concave picking portion 2a which is placed on the front end of the specimen-picking rod 2. The concave picking portion 2a is formed in such a way that the concave picking portion 2a can be removably fitted to the convex removing portion 6 which is provided on the bottom portion of the container body 1. The cover member 3 connects with the rear end portion 2b of the specimen-picking rod 2. In addition, a thread 3a₁ is formed in the internal peripheral portion 3a of the cover member 3.

The intermediary unit 4 includes a connection portion 4a and a penetration wall 4b. The connection portion 4a is formed in the shape of a tube in such a way that the both ends of the connection portion 4a are opened. A thread 4a₁₁ is formed in the internal peripheral portion 4a₁ of the one end of the connection portion 4a and is threadedly engaged with the thread 1a₁ which is formed in the external peripheral portion 1a of the open end of the container body 1 (refer to Fig. 2 (a) to (c)). A thread 4a₂₁ is formed in the external peripheral portion 4a₂ of the other one end of the connection portion 4a and is threadedly engaged with the thread 3a₁ which is formed in the internal peripheral portion 3a of the cover member 3. And, the intermediary unit 4 is formed in such a way that the one end of the connection portion 4a can be removably fitted to the open end of the container body 1 through the threads 4a₁₁ and the other end of the connection portion 4a can be removably fitted to the cover member 3 through the threads 4a₂₁. In addition, the container body 1, the intermediary unit 4, and the cove member 3 are formed in such a way that torque by which the one end of the connection portion 4a of the intermediary unit 4 is removably fitted to the open end of the container body 1 is larger than torque by which the other end of the connection portion 4a of the intermediary unit 4 is removably fitted to the cover member 3.

The penetration wall 4b is formed on the inside of the connection portion 4a and includes a through hole 4b₁ which is formed in the central portion of the penetration wall 4b. The through hole 4b₁ is formed in such a way that the diameter of a through hole 4b₁ is smaller than that of the specimen-picking rod 2. Also, for example, the penetration wall 4b is formed as an elastic member which is made of natural rubber, synthetic rubbers such as butadiene rubber, silicone rubber, and latex, or synthetic polymers such as silicone, polyethylene, polypropylene, elastomer, fluoride-based resin, polyamide-based resin (nylon), polyacetal-base resin, ABS resin, aromatic polyether ketone resin, and polyphenylene sulfide. And, when the specimen-picking rod 2 penetrates through the through hole 4b₁, the penetration wall 4b elastically deforms to come into close contact with the specimen-picking rod 2 through the surface of the penetration wall 4b which forms the through hole 4b₁, so that the penetration wall 4b separates a space into two divisions on the upper and lower sides. In addition, in order to be capable of easily inserting the specimen-picking rod 2b into the through hole 4b₁, the penetration wall 4b is formed in such a way that the vicinity of the through hole 4b₁ is formed in the shape of a cone to slope toward the through hole 4b₁.

An operation procedure for preparing a container as a sample tube for picking a specimen, removing the specimen to the container body, and mounting the container in a nucleic acid-extracting apparatus, and the operation effects will be explained using the container for specimen of the first embodiment with the above-described constitution.
First, in the initial state, the intermediary unit 4 is fitted to the container body 1 and the cover member 3 is fitted to the intermediary unit 4, as shown in Fig. 1 (a). In addition, the specimen-picking rod 2 penetrates through the through hole 4b₁ of the penetration wall 4b of the intermediary unit 4 and the concave picking portion 2a is fitted to the convex removing portion 6.

In the case where a specimen is picked, the cover member 3 is first rotated in the open direction to remove the specimen-picking rod 2 having a state as shown in Fig. 1 (a) from the intermediary unit 4 and the container body 1. As a result, the cover member 3 moves in the direction in which the cover member 3 is removed from the other end of the connection portion 4a of the intermediary unit 4, through the thread 3a₁ which is formed in the internal peripheral portion 3a and the thread 4a₂₁ which is formed in the external peripheral portion 4a₁ of the other end of the connection portion 4a of the intermediary unit 4. As a result of the movement of the cover member 3, the concave picking portion 2a of the specimen-picking rod 2 moves in the direction in which the concave picking portion 2a is released from the convex removing portion 6 fitting to the concave picking portion 2. After the cover member 3 is unfastened from the other end of the connection portion 4a of the intermediary unit 4, the cover member 3 is pulled in the direction in which the cover member 3 moves away from the intermediary unit 4. As a result, the specimen-picking rod 2 is pulled out of the through hole 4b₁ of the penetration wall 4b to come out of the other end of the connection portion 4a.
Next, a specimen is picked in such a way that the concave picking portion 2a on the front end of the specimen-picking rod 2 is inserted into the specimen (which is omitted in the drawing), using the specimen-picking rod 2 coming out of the other end of the connection portion 4a of the intermediary unit 4.

Next, the front end of the specimen-picking rod 2 holding the specimen is pushed into the through hole 4b₁ of the intermediary unit 4, and the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides. As a result, a surplus part of the specimen which adheres to the external side surface of the specimen-picking rod 2 is removed by the upper portion of the through hole 4b₁ in the penetration wall 4b.
Next, the thread 3a₁ which is formed in the internal peripheral portion 3a of the cover member 3 is threadedly engaged with the thread 4a₂₁ which is formed in the external peripheral portion 4a₂ of the other end portion of the connection portion 4a in the intermediary unit 4, and the cover member 3 is rotated in the close direction. As a result, the concave picking portion 2a of the specimen-picking rod 2 which penetrates through the through hole 4b₁ is fitted to the convex removing portion 6 which is provided on the bottom portion of the container body 1, the specimen held by the concave picking portion 2a is pushed out into the container body 1 by the convex removing portion 6, and the cover member 3 is fastened to the intermediary unit 4. As a result, the container body 1 can hold the picked specimen with the container body 1 in an air-tight state.

In the case where the container body 1 in which holds a specimen in an air-tight state is mounted in a nucleic acid-extracting apparatus, the intermediary unit 4 is rotated relative to the container body 1 in the direction in which the intermediary unit 4 separates away from the container body 1. As a result, the intermediary unit 4, together with the cover member 3 connecting with the specimen-picking rod 2, moves in the direction in which the intermediary unit 4 is removed from the external peripheral portion 1a of the open end of the container body 1, through the thread 1a₁ which is formed in the external peripheral portion 1a of the open end of the container body 1 and the thread 4a₁₁ which is formed in the external peripheral portion 4a₁ of the one end portion of the connection portion 4a in the intermediary unit 4. And, after the intermediary unit 4 is removed from the container body 1, the container body 1 is mounted in a nucleic acid-extracting apparatus.

Accordingly, according to the container for specimen of the first embodiment, it is possible to mount the container body in a general-purpose apparatus for physical and chemical analysis like a centrifuge as it stands without transferring a collected and preserved specimen from the container body to another container even in tests requiring the extraction of nucleic acids with a general-purpose apparatus for physical and chemical analysis like a centrifuge. In addition, it is possible to use for test a specimen the collection amount of which is quantified within a predetermined acceptable range.

### Second Embodiment

Fig. 3 is an explanatory view showing one example of the constitution of a main part of a container for specimen according to a second embodiment of the present invention, and Figs. 4 and 5 are explanatory views showing examples of variations of the main parts of the second embodiment, where the examples shown in Figs. 4 and 5 are different from each other. Besides, the constitutions except the connection portion of the intermediary unit and the container body do not shown in Figs. 3, 4, and 5 for convenience.

In the container for specimen of the second embodiment, the structure of fitting convex portions into concave portions is adopted instead of the engagement of threads in the container for specimen of the first embodiment so that the intermediary unit 4 can be removably fitted to the container body 1.
For example, in the example of the container for specimen which is shown in Fig. 3, the external peripheral portion 4a₁' of one end of the connection portion 4a in the intermediary unit 4 is provided with: an annular convex portion 4a₁₁' which is formed along the penetration direction; an annular stopper portion 4a₁₂' the diameter of which is larger than the internal peripheral portion 1a' of the open end of the container body 1; and an O-ring 4a₁₃" which is placed on the container-body-1 side of the stopper member 4a₁₂', as shown in Fig. 3 (b) and (c). In addition, the internal peripheral portion 1a' of the open end of the container body 1 is provided with an annular concave portion 1a₁' which can be fitted to the annular convex portion 4a₁₁', as shown in Fig. 3 (a) and (c).

Also, for example, in the example of the container for specimen which is shown in Fig. 4, the edge portion 4a₁₁" of one end of the connection portion 4a in the intermediary unit 4 is provided with: an annular groove 4a₁₁" which is formed so as to be capable of being fitted to the open end of the container body 1; an O-ring 4a₁₂" which is placed on the bottom of the annular groove 4a₁₁"; and a plurality of opening portions 4a₁₃" which are provided on the circumference of the connection portion 4a of the intermediary unit 4 so as to link the annular groove 4a₁₁" and the external peripheral portion 4a₁' of the one end of the connection portion 4a of the intermediary unit 4, as shown in Fig. 4 (b) and (c).
In addition, the external peripheral portion 1a of the open end of the container body 1 is provided with a plurality of catch portions 1a₂ which can be inserted into the opening portions 4a₁₃" of the connection portion 4a of the intermediary unit 4, as shown in Fig. 4 (a) and (c). The catch portions 1a₂ are formed in such a way that the catch portions 1a₂ break off at the bases when the catch portions 1a₂ are inserted into the opening portions 4a₁₃" and a predetermined amount of force is applied in the upper or lower direction.

Also, for example, in the container for specimen which is shown in Fig. 5 and is one example of variations of the container for specimen shown in Fig. 4, the catch portions 1a₂ which is provided for the container body 1 are formed in such a way that the lower side surfaces 1a₂, of the catch portions 1a₂ slope upward so that the catch portions 1a₂ come out of the opening portions 4a₁₃" when force is applied so as to move the intermediary unit 4 in the direction opposite to the direction toward the container-body-1 side with the catch portions 1a₂ inserted into the opening portions 4a₁₃".

The other constitutions of the second embodiment are approximately the same as those of the container for specimen of the first embodiment. The container for specimen of the second embodiment which is formed in such a manner makes it possible to removably fit the intermediary unit 4 to the container body 1 without rotating the one end of the connection portion 4a of the intermediary unit 4 relative to the open end of the container body 1.

For example, in the example of the container for specimen which is shown in Fig. 3, when the one end of the connection portion 4a of the intermediary unit 4 is pushed into the open end of the container body 1, as shown in Fig. 3 (d), the annular convex portion 4a₁₁' of the external peripheral portion 4a₁' of the one end of the connection portion 4a in the intermediary unit 4 slides on the internal peripheral portion 1a' of the open end of the container body 1 to move toward the bottom portion of the container body 1. And, when the annular convex portion 4a₁₁' moves to a set position, as shown in Fig. 3 (e), the annular convex portion 4a₁₁' is fitted into the annular concave portion 1a₁' and the O-ring 4a₁₃" is put between the stopper portion 4a₁₂' and the edge portion of the open end of the container body 1. As a result, the attachment of the intermediary unit 4 to the container body 1 is finished.
On the other hand, when the intermediary unit 4 is separated from the container body 1 in a state of the intermediary unit 4 shown in Fig. 3 (e), the intermediary unit 4 is pulled relative to the container body 1. As a result, the annular convex portion 4a₁₁' is released from the annular concave portion 1a₁' fitted to the annular convex portion 4a₁₁" and the annular convex portion 4a₁₁' slides on the internal peripheral portion 1a' of the open end of the container body 1 to move toward the open end of the container body 1. And then, when the one end of the connection portion 4 of the intermediary unit 4 moves to a set position, as shown in Fig. 3 (c), the intermediary unit 4 comes out of the container body 1.

Also, for example, in the example of the container for specimen which is shown in Fig. 4, as shown in Fig. 4 (d), as the intermediary unit 4 is deformed, the catch portions 1a₂ of the external peripheral surface 1 a of the open end of the container body 1 are passed through opening portions 4a₁₃" of the connection portion 4a of the intermediary unit 4 respectively in turn, and the annular groove 4a₁₁" is fitted to the open end of the container body 1. When the fitting of the annular groove 4a₁₁" to the open end of the container body 1 is finished, as shown in Fig. 4 (e), the O-ring 4a₁₂" seals the gap between the edge portion of the open end of the container body 1 and the annular groove 4a₁₁" of the intermediary unit 4. As a result, the attachment of the intermediary unit 4 to the container body 1 is finished.
On the other hand, when the intermediary unit 4 is separated from the container body 1 in a state of the intermediary unit 4 shown in Fig. 4 (e), as shown in Fig. 4 (f), a predetermined amount of force is applied to the front ends of the catch portions 1a₂ in the upper or lower direction with the catch portions 1a₂ inserted into the opening portions 4a₁₃". As a result, the catch portions 1a₂ break off at the bases. And then, the broken-off catch portions 1a₂ are eliminated, so that the intermediary unit 4 can be in a state in which the intermediary unit 4 can separate from the container body 1. Next, the intermediary unit 4 is pulled relative to the container body 1, so that the intermediary unit 4 comes out of the container body 1.

Also, for example, in the example of the container for specimen which is shown in Fig. 5, the procedure for attaching the intermediary unit 4 to the container body 1 is approximately the same as that of the example of the container for specimen shown in Fig. 4.
On the other hand, when the intermediary unit 4 is separated from the container body 1, the intermediary unit 4 is pulled relative to the container body 1. As a result, the intermediary unit 4 are pushed to be gradually widen outward with the lower sides of the opening portions 4a₁₃" abutting on the lower side surfaces 1a₂₁ of the catch portions 1a₂. And then, when the intermediary unit 4 is pulled to a predetermined position, the lower sides of the opening portions 4a₁₃" come out of the lower side surfaces 1a₂₁ of the catch portions 1a₂, so that the intermediary unit 4 can be in a state in which the intermediary unit 4 can be separated from the container body 1. And then, when the intermediary unit 4 is pulled relative to the container body 1 more, the intermediary unit 4 comes out of the container body 1.

Accordingly, according to the container for specimen of the second embodiment, it is possible to removably fit the intermediary unit 4 to the container body 1 without rotating the one end of the connection portion 4a of the intermediary unit 4 relative to the container body 1.
The other operation effects of the container for specimen of the second embodiment are the same as those of the container for specimen of the first embodiment.

### Third Embodiment

Fig. 6 is a view showing the constitution of a main part of a container for specimen according to a third embodiment of the present invention. In the container for specimen of the third embodiment, a part of the penetration wall 4b of the intermediary unit 4 includes an elastic member. In a more detailed explanation, as shown in Fig. 6, an annular groove 4b₁₁ is formed in the surface which forms the through hole 4b₁ of the penetration wall 4b, and an annular elastic member 4b₁₂ the inner diameter of which is smaller than the diameter of through hole 4b₁ is fitted into the annular groove 4b₁₁ When the specimen-picking rod 2 is made to penetrate through the through hole 4b₁, the annular elastic member 4b₁₂ is pushed by the specimen-picking rod 2 to elastically deform, so that the annular elastic member 4b₁₂ comes into close contact with the specimen-picking rod 2. The other constitutions of the container for specimen of the third embodiment are approximately the same as those of the first embodiment.

According to the container for specimen of the third embodiment, even though a part of the penetration wall 4b is made of non-elastic member, as in the first embodiment, it is possible to remove a surplus part of a specimen to quantify the specimen used for a test. In addition, it is also possible to keep the container air-tight. The other operation effects of the container for specimen of the third embodiment are approximately the same as those of the container for specimen of the first embodiment.

### Fourth Embodiment

Fig. 7 is a view showing the constitution of a main part of a container for specimen according to a fourth embodiment of the present invention. In the container for specimen of the fourth embodiment, the penetration wall 4b is provided with an elastic member 4b₂ which includes radial slits 4b₂₁ passing through the center of the through hole 4b₁ and covers the through hole 4b₁. The elastic member 4b₂ is formed in such a way that the elastic member 4b₂ deforms to come into close contact with the specimen-picking rod 2 when the specimen-picking rod 2 is inserted into the through hole 4b₁. The other constitutions of the container for specimen of the fourth embodiment are approximately the same as those of the container for specimen of the first embodiment.

According to the container for specimen of the fourth embodiment, even though a part of the penetration wall 4b is made of non-elastic member, as in the first embodiment, it is possible to remove a surplus part of a specimen to quantify the specimen used for a test and it is also possible to keep the container air-tight, like the container for specimen of the third embodiment. The other operation effects of the container for specimen of the fourth embodiment are approximately the same as those of the container for specimen of the first embodiment.

### Fifth Embodiment

Fig. 8 shows the constitution of a container for specimen according to a fifth embodiment of the present invention, and Fig. 9 is an explanatory view showing examples of the formation of a cross sectional shape of a groove which is formed on the penetration wall in the container for specimen of the fifth embodiment.

In the container for specimen of the fifth embodiment, a plurality of annular grooves 4b₁₁' are formed on the surface forming the through hole 4b₁ of the penetration wall 4b of the intermediary unit 4 and along the penetration direction, so that a plurality of fins are to be formed on the surface forming the through hole 4b₁ of the penetration wall 4b. The other constitutions of the container for specimen of the fifth embodiment are approximately the same as those of the container for specimen of the first embodiment.

According to the container for specimen of the fifth embodiment which is formed in such a manner, a plurality of the annular grooves 4b₁₁' are provided on the surface forming the through hole 4b₁ of the penetration wall 4b, so that the vicinity of the through hole 4b₁ becomes easy to elastically deform. The other constitutions and operation effects of the container for specimen of the fifth embodiment are approximately the same as those of the container for specimen of the first embodiment.
Besides, even if the cross sectional shapes of a plurality of a plurality of the annular grooves which are formed on the surface forming the through hole 4b₁ of the penetration wall 4b are formed in the shape of any one of the letter "V" as shown in Fig. 9 (b) and an arc as shown in Fig. 9 (c) other than the annular grooves 4b₁₁' having the shape of the letter "U" as shown in Figs. 8 and 9 (a), the same operation effects can be obtained in the container for specimen of the fifth embodiment.

### Sixth Embodiment

Fig. 10 is a view showing the constitution of a container for specimen according to a sixth embodiment of the present invention. The container for specimen of the sixth embodiment is formed in such a way that: the intermediary unit 4 connects with the convex removing portion 6 through a connection means 7; and the convex removing portion 6 which is explained in the container for specimen of the first embodiment also can be separated from the container body 1 with the convex removing portion 6 kept fitted to the concave picking portion 2a of the specimen-picking rod 2 at the same time when the intermediary unit 4 is removed form the container body 1.
As shown in Fig. 10 (a), the connection means 7 is composed of an engaging member 7a and a frame member 7b. The engaging member 7a is fixed on the inside of the connection portion 4a of the intermediary unit 4. As shown in Fig. 10 (b), the frame member 7b is formed in the shape of a tube so as to be capable of being contained inside the container body 1. In addition, the frame member 7b includes three cut-out portions 7b₁₁ on the bottom portion 7b₁ of the frame member 7b. And, the frame member 7b holds the lower end 6a of the convex removing portion 6 at the center portion of the bottom portion 7b₁. In addition, the frame member 7b is formed in such a way that the upper portion 7b₂ of the frame member 7b can be engaged with the engaging member 7a. The other constitutions of the container for specimen of the sixth embodiment are approximately the same as those of the container for specimen of the first embodiment.

According to the container for specimen of the sixth embodiment which is formed in such a manner, when the intermediary unit 4 is removed from the container body 1, the convex removing portion 6, together with the intermediary unit 4, can be removed from the container body 1 through the frame member 7b. The other operation effects of the container for specimen of the sixth embodiment are approximately the same as those of the container for specimen of the first embodiment.

Besides, the container for specimen in the example shown in Fig. 10 is provided with the connection means 7 that is composed of the engaging member 7a and the frame member 7b. However, if the convex removing portion 6, together with the intermediary unit 4, can be separated from the container body 1 without scooping up liquid and a specimen that are contained in the container body 1, the container for specimen may have any constitution. For example, the intermediary unit 4 may be integratedly connected with the convex removing portion 6 with a set member intervening between the intermediary unit 4 and the convex removing portion 6.

### Seventh Embodiment

Fig. 11 is a fragmentary explanatory view of a main part in one example of the container for specimen according to a seventh embodiment of the present invention, and Fig. 12 is a fragmentary explanatory view showing a main part in another example of the container for specimen of the seventh embodiment.

The container for specimen of the seventh embodiment is formed in such a way that the specimen-picking rod 2 includes a path which links the concave picking portion 2a and the external peripheral surface of the specimen-picking rod 2 and a specimen passes through the path to be extruded to the outside when the convex removing portion 6 is inserted into the concave picking portion 2a. In a more detailed explanation, the specimen-picking rod 2 includes a plurality of slits 2c which link the lateral side of the concave picking portion 2a and the external peripheral surface of the specimen-picking rod 2 in the example of the container for specimen shown in Fig. 11. Also, in the example of the container for specimen shown in Fig. 12, the specimen-picking rod 2 includes a plurality of extrusion holes 2c' which link the bottom surface of the concave picking portion 2a and the external peripheral surface of the specimen-picking rod 2. The other constitutions of the container for specimen of the seventh embodiment are approximately the same as those of the container for specimen of the first embodiment.

According to the container for specimen of the seventh embodiment which is formed in such a manner, in the example of the container for specimen shown in Fig. 11, a specimen passes through the slits 2c to be extruded to the outside when the convex removing portion 6 is inserted into the concave picking portion 2a, so that the specimen that is quantitatively collected by the concave picking portion 2a becomes easy to remove to the container body 1 through the convex removing portion 6. Also, in the example of the container for specimen shown in Fig. 12, a specimen passes through the extrusion holes 2c' to be extruded to the outside when the convex removing portion 6 is inserted into the concave picking portion 2, so that the specimen that is quantitatively collected by the concave picking portion 2a becomes easy to remove to the container body 1 through the convex removing portion 6.
The other operation effects of the container for specimen of the seventh embodiment are approximately the same as those of the container for specimen of the first embodiment.

Besides, in order to remove impurities in a stool, such as fibrous substance and large solid body having a grain diameter, for example, as shown in Fig. 11 (d) to (k), the shapes of the slits 2c in the front end of the specimen-picking rod 2 may be given a large number of indentations (refer to Fig. 11 (d) and (e)) or a large number of narrows (refer to Fig. 11 (f) and (g)), or a division plate 2c₁ (refer to Fig. 11 (h) and (i)) or a large number of snags 2c₂ (refer to Fig. 11 (j) and (k)) may be provided in the slits 2c, so that the paths are provided with parts the diameters which are smaller than that of each of the normal flow paths shown in Fig. 11 (a) and (c). As a result, a large number of impurities can be removed to the extent that the impurities do not affect test processes.

### Eighth Embodiment

Fig. 13 is a fragmentary explanatory view showing a main part in one example of a container for specimen according to an eighth embodiment of the present invention and Fig. 14 is a fragmentary explanatory view showing a main part in another example of the container for specimen of the eighth embodiment.
The container for specimen of the eighth embodiment is different from the containers for specimen of the first to seventh embodiments in the constitution of the picking portion of the specimen-picking rod 2. In a more detailed explanation, the specimen-picking rod 2 of the example shown in Fig. 13 includes a picking portion 2a' which is provided with a groove 2a₁' that is formed in the shape of a lattice and on the external peripheral surface of the front end of the specimen-picking rod 2. Also, the specimen-picking rod 2 of the example shown in Fig. 14 includes a picking portion 2a" which is placed at the front end of the specimen-picking rod 2 and is shaped like a brush.
And, the container for specimen of the eighth embodiment is formed on the premise that a specimen which is picked by the picking portion 2a' or 2a" of the specimen-picking rod 2 is dispersed or suspended in liquid 5 of the container body 1 to be removed to the container body 1, so that a convex removing portion is not provided on the bottom of the container body 1. The other constitutions of the container for specimen of the eighth embodiment are approximately the same as those of the container for specimen of the first embodiment.

In the container for specimen of the eighth embodiment which is formed in such a manner, when a specimen to be picked is a gel-like and soft substance, the gel-like specimen can be made to easily adhere to the picking portion 2a' which is provided with the groove 2a₁' that is formed on the external peripheral surface of the front end of the specimen-picking rod 2 in the example shown in Fig. 13, so that the gel-like specimen is easy to quantitatively pick. In addition, the gel-like specimen is easily dispersed or suspended in the liquid 5 of the container body 1, so that the gel-like specimen hardly remains on the picking portion 2a' even though the lattice-shaped grooves 2a₁' are used for picking the gel-like specimen. Accordingly, the specimen can be quantitatively removed to the container body 1. Besides, because a surplus part of the specimen which has adhered to the lateral surface except the lattice-shaped groove in the specimen-picking rod 2 is removed when the specimen-picking rod 2 passes through the through hole of the penetration wall, there is no obstacle to quantifying an amount of a specimen that is removed to the container body 1. However, it cannot be said that adhesion force of a gel-like and soft specimen to the lattice-shaped groove 2a₁' is strong. Accordingly, in the case where the picking portion 2a' is formed with the lattice-shaped groove 2a₁' that are provided on the external peripheral surface of the front end of the specimen-picking rod 2 for the purpose of picking a gel-like specimen, it is preferred that the diameters of the through hole 4b₁ of the penetration wall 4b and the specimen-picking rod 2 are designed to the extent that the specimen-picking rod 2 slides on the through hole 4b₁ without deforming the penetration wall 4b. Also, in the example of the container for specimen shown in Fig. 14, it is possible to pick a specimen having some degree of viscosity, for example, like mucosa by the brash-shaped picking portion 2a".

### Ninth Embodiment

Fig. 15 is a fragmentary explanatory view showing a main part in a container for specimen according to a ninth embodiment of the present invention.
The container for specimen of the ninth embodiment is different from the containers for specimen of the first to eighth embodiments in the constitution of specimen-picking rod, and the specimen-picking rod of the ninth embodiment is provided with a mechanism for extruding to the outside a specimen that is contained inside the specimen-picking rod. In a more detailed explanation of the container for specimen of the ninth embodiment, the specimen-picking rod 2 includes an absorbing portion 2a"', a tube member 2b"', and a rod member 2c"'. The absorbing portion 2a"' is made of spongy material. The both ends of the tube member 2b"' are opened and the front end of the tube member 2b"' connects with the absorbing portion 2a"'. The rod member 2c"' is formed in such a way that: the rod member 2c"' has a diameter that is as large as the rod member 2c"' can move inside the tube member 2b"'; and the front end portion 2c, "' of the rod member 2c"' can push the absorbing portion 2a"'. In addition, the other end of the rod member 2c"' is connected with the cover member 3. The other constitutions of the container for specimen of the ninth embodiment are approximately the same as those of the container for specimen of the first embodiment.

According to the container for specimen of the ninth embodiment which is formed in such a manner, for example, it is possible to collect specimens which are high in water and have viscosity, such as blood and sputum, by the absorbing portion 2a"' of the specimen-picking rod 2 which is made of spongy material. Also, when the collected specimen is put into and removed to the container body 1, the specimen-picking rod 2 the absorbing portion 2a"' of which absorbs the specimen is made to penetrate through the through hole 4b₁ of the intermediary unit 4 to be inserted into the container body 1. In this case, the container for specimen is formed in such a way that the front end portion 2c₁"' of the rod member 2c"' pushes the absorbing portion 2a"' when the cover member which is not shown in the drawing is fitted to the one end of the connection portion of the intermediary unit. As a result, the absorbing portion 2a"' is sandwiched between the front end portion 2c₁"' of the rod member 2c"' and the bottom of the container body 1 to be elastically deformed, so that the absorbed specimen is expelled from the absorbing portion 2a"' into the container body 1. As a result, it is possible to quantitatively remove the collected specimen to the container body.

### Tenth Embodiment

Fig. 16 is an explanatory view schematically showing the constitution of a container for specimen according to a tenth embodiment of the present invention, and Fig. 17 is an explanatory view showing the constitution of the cover member of the container for specimen shown in Fig. 16.

As shown in Fig. 16, the container for specimen of the tenth embodiment is formed in such a way that the outer diameter of the cover member 3 is larger than that of the connection portion 4a of the intermediary unit 4 and the outer diameter of the connection portion 4a of the intermediary unit 4 is larger than that of the container body 1. In addition, as shown in Fig. 17 (c), the cover member 3 includes a grasping portion which is formed in the shape of a D-cut shape so that the outside portion of the cover member 3 can be easily grasped. In the example shown in Fig. 17 (c), the D-cut shapes are given in two parts of the cover member 3 which are opposite to each other. Besides, the D-cut shape may be given in one part of the outside portion of the cover member 3. In addition, a fitting groove 3b into which the vicinity of the rear end 2b of the specimen-picking rod 2 is inserted is formed in the middle of the inside of the cover member 3. The vicinity of the rear end 2b is fitted into the fitting groove 3b, so that the specimen-picking rod 2 is fixed to the cover member 3. The other constitutions of the container for specimen of the tenth embodiment are approximately the same as those of the container for specimen of the first embodiment.

According to the container for specimen of the tenth embodiment which is formed in such a manner, the outside portion of the cover member 3 includes the D-cut shaped grasping portions, so that the cover member 3 becomes easy to rotate. In addition, the outer diameter of the cover member 3 is larger than that of the connection portion 4a of the intermediary unit 4 and the outer diameter of the connection portion 4a of the intermediary unit 4 is larger than that of the container body 1, so that force necessary for removing the cover member 3 from the intermediary unit 4 can be made to become weaker than the minimum force necessary for removing the intermediary unit 4 from the container body 1. As a result, the container for specimen can have the action in which the intermediary unit 4, together with the cover member 3, does not come out of the container body 1 when the cover member 3 is removed from the intermediary unit 4. Accordingly, although the intermediary unit 4 cannot be released from the threaded engagement of the intermediary unit 4 with the container body 1 unless force that is stronger than force necessary for removing the cover member 3 from the intermediary unit 4 is applied to the intermediary unit 4, the outer diameter of the connection portion 4a of the intermediary unit 4 is larger than that of the container body 1, so that the intermediary unit 4 can be easily removed from the container body 1.
Besides, when the connection portion 4a of the intermediary unit 4 and the cover member 3 are formed in such a way that the material of the connection portion 4a is different from that of the cover member 3, the container for specimen can have the action of keeping the container air-tight and the cover member 3 becomes easy to removably fit to the intermediary unit 4. Besides, when the container body 1 and the connection portion 4a of the intermediary unit 4 are formed in such a way that the material of the container body 1 is different from that of the connection portion 4a, the container for specimen can have the action of keeping the container air-tight and the intermediary unit 4 becomes easy to removably fit to the container body 1.

### Eleventh Embodiment

Fig. 18 is an explanatory view showing the constitution of a container for specimen according to an eleventh embodiment of the present invention, Fig. 19 is an explanatory view showing the constitutions of the cover member and specimen-picking rod in the container for specimen shown in Fig. 18, Fig. 20 is a side view of the cover member and specimen-picking portion, Fig. 21 is a view showing the constitution of the intermediary unit, and Fig. 22 is a view showing the constitution of the container body.

As shown in Fig. 18, the container for specimen of the eleventh embodiment comprises a container body 1, a specimen-picking rod 2 for picking a specimen, a cover member 3, and an intermediary unit 4. As shown in Figs. 18 to 22, the container body 1 is formed in such a way that: one end of the container body 1 is open; the container body 1 can hold a liquid for dispersing or preserving a specimen; and the container body 1 is formed in the shape of a tube having a size and shape which make it possible to mount the container body 1 in a general-purpose apparatus for physical and chemical analysis like a general-purpose nucleic acid-extracting apparatus. A thread 1b₁ is formed in the internal peripheral portion 1b of the open end of the container body 1. As shown in Figs. 18 to 20, the cover member 3 connects with the rear end portion 2b of the specimen-picking rod 2. In addition, a thread 3a₁ is formed in the internal peripheral portion 3a of the cover member 3.

As shown in Figs. 18 to 21, the intermediary unit 4 includes a connection portion 4a and a penetration wall 4b. The connection portion 4a is formed in the shape of a tube the both ends of which are opened. A thread 4a₁₁ is formed in the external peripheral portion 4a₁ of the middle part of the connection portion 4a and is threadedly engaged with the thread 1b₁ which is formed in the internal peripheral portion 1b of the open end of the container body 1 (refer to Figs. 18 and 21). A thread 4a₂₁ is formed in the external peripheral portion 4a₂ of the upper end of the connection portion 4a and is threadedly engaged with the thread 3a₁ which is formed in the internal peripheral portion 3a of the cover member 3. And, the intermediary unit 4 is formed in such a way that the middle part of the connection portion 4a can be removably fitted to the open end of the container body 1 through the threads 4a₁₁ and the upper end of the connection portion 4a can be removably fitted to the cover member 3 through the threads 4a₂₁. In addition, the container body 1, the intermediary unit 4, and the cove member 3 are formed in such a way that torque by which the middle part of the connection portion 4a of the intermediary unit 4 is fitted to or removed from the open end of the container body 1 is larger than torque by which the upper end of the connection portion 4a of the intermediary unit 4 is fitted to or removed from the cover member 3.

The penetration wall 4b is formed on the inside of the connection portion 4a and includes a through hole 4b₁ which is formed in the central portion of the penetration wall 4b. The through hole 4b₁ is formed in such a way that the diameter of a through hole 4b₁ is smaller than that of the specimen-picking rod 2. Also, for example, the penetration wall 4b is formed as an elastic member which is made of natural rubber, synthetic rubbers such as butadiene rubber, silicone rubber, and latex, or synthetic polymers such as silicone, polyethylene, polypropylene, elastomer, fluoride-based resin, polyamide-based resin (nylon), polyacetal-base resin, ABS resin, aromatic polyether ketone resin, and polyphenylene sulfide. And, when the specimen-picking rod 2 penetrates through the through hole 4b₁, the penetration wall 4b elastically deforms to come into close contact with the specimen-picking rod 2 through the surface of the penetration wall 4b which forms the through hole 4b₁, and the penetration wall 4b separates a space into two divisions on the upper and lower sides. In addition, in order to be capable of easily inserting the specimen-picking rod 2 into the through hole 4b₁, the penetration wall 4b is formed in such a way that the vicinity of the through hole 4b₁ is formed in the shape of a cone to slope toward the through hole 4b₁.

In this case, as a characteristic constitution of the container for specimen of the eleventh embodiment, the specimen-picking rod 2 includes a forked portion 2c which is placed on the opposite side to the rear end 2b connecting with the cover member 3. The forked portion 2c is formed in such a way that elastic force keeps the forked portion 2c open, and the forked portion 2c can be opened and closed on the front end of the specimen-picking rod 2. The elasticity of the material which the forked portion 2c is made of is used for the elasticity of the forked portion 2c of the specimen-picking rod 2. Besides, the forked portion 2c may be made to have elasticity by forming a hinge joint in the forked portion 2c and biasing the forked portion 2c by a spring in the direction of the forked portion 2c opening. Also, as shown in Figs. 19 and 20, the forked portion 2c includes at least one specimen-holding portion 2c₁ which is provided with: a specimen-introducing inlet 2c₁₁ that is formed on the side on which one branching portion of the forked portion 2c touches the other branching portion of the forked portion 2c when the forked portion is closed (or, on the inside); and a specimen-exhausting outlet 2c₁₂ that is formed on the side opposite to the side on which the specimen-introducing inlet 2c₁₁ is formed (or, on the outside). (In the example shown in Figs. 19 and 20, the forked portion 2c includes two specimen-holding portions 2c₁ which are placed in the branching portions of the forked portion 2c respectively.) The specimen-holding portion 2c₁ is designed with a volume which makes the specimen-holding portion 2c₁ capable of holding a desired and predetermined amount of a specimen. And, specimen-holding portion 2c₁ holds a desired and predetermined amount of a fragmented specimen in a state of the forked portion 2c closed.
Besides, at least one specimen-holding portion 2c₁ may be provided for one of the branching portions of the forked portion 2c.
Also, and the specimen-exhausting outlet 2c₁₂ may be formed in the same shape as the specimen-introducing inlet 2c₁₁, or in the shape and/or size different from the shape and/or size of specimen-introducing inlet 2c₁₁ in accordance with kinds of specimens to be collected.

In addition, an annular member 7 is fitted to the outer circumference of the specimen-picking rod 2. The annular member 7 can move along the longitudinal direction of the specimen-picking rod 2. The annular member 7 is formed in such a way that the inner diameter of the annular member 7 is large enough that the annular member 7 is moved towards the front end by a predetermined distance to make the forked portion 2c close and is moved towards the rear end by a predetermined distance to release the forked portion 2c from the closed state. In addition, the specimen-picking rod 2 includes a projection 2d which is placed at a predetermined position that is nearer to the rear side than the forked portion 2c that is in an open state. The projection 2d comes into contact with a part of the internal peripheral surface of the annular member 7 to hold the annular member 7. The projection 2d functions as an annular member-fixing means for fixing the annular member 7.
Also, the through hole 4b₁ is formed with a size as large as the specimen-picking rod 2 can slide on the through hole 4b₁ to penetrate through the through hole 4b₁ with the forked portion 2c closed.

An operation procedure for preparing a container as a sample tube for picking a specimen using the container for specimen of the eleventh embodiment which is formed in such a manner, removing the specimen to the container body, and mounting the container in a nucleic acid-extracting apparatus, and the operation effects will be explained.
When the annular member 7 is moved towards the rear end by a predetermined distance, the forked portion 2c of the specimen-picking rod 2 is released from the annular member 7 that restrains the forked portion 2c from opening, and the front end of the forked portion 2c opens towards the outside by the elasticity of the forked portion 2c. In the initial state in a stage before picking a specimen, the intermediary unit 4 is fitted to the container body 1 and the cover member 3 is fitted to the intermediary unit 4, as shown in Fig. 18 (a). In addition, the specimen-picking rod 2 penetrates through the through hole 4b₁ of the penetration wall 4b of the intermediary unit 4 and the container for specimen is sealed up. The annular member 7 is pushed up through the upper-side surface of the penetration wall 4b up to a position at which the annular member 7 comes in contact with the projection 2d that is provided at a set position on the rear-end-2b side of the specimen-picking rod 2. And then, the position of the annular member 7 is fixed by the projection 2d coming in contact with a part of the internal surface of the annular member 7. At this point, the forked portion 2c is in an open state.

In the case where a specimen is picked, a user first holds the cover member 3 and rotates the cover member 3 in the direction of the cover member 3 opening in order to remove the specimen-picking rod 2 having a state as shown in Fig. 18 (a) from the intermediary unit 4 and the container body 1. As a result, the cover member 3 moves in the direction in which the cover member 3 is removed from the other end of the connection portion 4a of the intermediary unit 4 through the thread 3a₁ which is formed in the internal peripheral portion 3a and the thread 4a₂₁ which is formed in the external peripheral portion 4a₂ of the other end portion of the connection portion 4a in the intermediary unit 4. After the cover member 3 is unfastened from the other end of the connection portion 4a of the intermediary unit 4, the cover member 3 is pulled in the direction in which the cover member 3 moves away from the intermediary unit 4. As a result, the specimen-picking rod 2 is pulled out of the through hole 4b₁ of the penetration wall 4b to come out of the other end of the connection portion 4a. In this case, when the specimen-picking rod 2 passes through the through hole 4b₁, the forked portion 2c is subjected to inward force from the through hole 4b₁ to become closed from the open state. When the specimen-picking rod 2 is completely pulled out, the elasticity of the forked portion 2c makes the forked portion 2c open again. At this point, the position of the annular member 7 is fixed through the projection 2d, so that the forked portion 2c is kept open.

Next, the user holds the cover member 3 and approximates the specimen-picking rod 2 to a position at which a specimen can be pinched by the forked portion 2c. Next, the cover member 3 is held by one hand of the user, and the annular member 7 is pinched by the other hand of the user to be moved towards the front end. At this point, the annular member 7 is released from the projection 2d by which the position of the annular member 7 is fixed. The annular member 7 is moved towards the front end by a predetermined distance, so that the forked portion 2c is closed and a specimen is made to pass through the specimen-introducing inlet 2c₁₁ into the specimen-holding portion 2c₁ with the specimen fragmented. Also, a surplus part of the specimen exceeding a predetermined amount of the specimen sticks out of the specimen-exhausting outlet 2c₁₂.

After picking the specimen in such a manner, the user pushes the specimen-picking rod 2 into the through hole 4b₁ of the intermediary unit 4 to insert the specimen-picking rod 2 into the through hole 4b₁ with the forked portion 2c closed, so that the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides. Besides, at the point in time of the beginning of the insertion of the specimen-picking rod 2 into the through hole 4b₁, the annular member 7 gives the forked portion 2c inward force that restrains force making the forked portion 2c open, so that the forked portion 2c is kept close.
After pushing the specimen-picking rod 2 inserted into the through hole 4b₁ until the annular member 7 meets the surface on the upper side of the penetration wall 4b, the user further continues to insert the specimen-picking rod 2 into the through hole 4b₁, so that the annular portion 7 is pushed up by the surface of the upper side in the penetration wall 4b. As a result, the elasticity of the forked portion 2c makes the forked portion 2c open in preservation liquid in the container body 1. The specimen which is contained by the specimen-holding portion 2c₁ is dispersed in the preservation liquid in the container body 1.

Now, when a specimen is picked by the specimen-picking rod 2, a surplus part of the specimen exceeding a predetermined amount of the specimen spills from the specimen-exhausting outlet 2c₁₂ and adheres to the circumference. However, in the container for specimen of the eleventh embodiment, when the forked portion 2c passes through the through hole 4b₁, the surplus part of the specimen which has spilled from the specimen-exhausting outlet 2c₁₂ and adhered to the forked portion 2c is scrubbed away to remain on the surface of the upper side of the penetration wall 4b. As a result, only part of the quantified specimen is contained in the preservation liquid in the container body 1.
Also, a specimen must be easily dispersed in preservation liquid. Accordingly, in the container for specimen of the eleventh embodiment, a plurality of the specimen-holding portions 2c₁ are provided for the container for specimen, so that the specimen can be fragmented to be collected. And, the fragmented specimen becomes easy to disperse in preservation liquid.

Besides, in the use of the container for specimen in the case of collecting a hard specimen having a high viscosity, it is preferred that the specimen is made to easily escape from the specimen-exhausting outlet 2c₁₂ so that the specimen is made to easily go in the specimen-holding portion 2c₁ through the specimen-introducing inlet 2c₁₁ and can be easily scooped up in picking the specimen. In order to achieve this purpose, it is preferred that the specimen-exhausting outlet 2c₁₂ is formed in such a way that the specimen-exhausting outlet 2c₁₂ has the same size as that of the specimen-introducing inlet 2c₁₁ or a size which is larger than that of the specimen-introducing inlet 2c₁₁. On the other hand, in the use of the container for specimen in the case of collecting a specimen having a low viscosity, it is preferred that the specimen is made to become incapable of easily escaping from the specimen-exhausting outlet 2c₁₂ in order to prevent the specimen from excessively going out of the specimen-holding portion 2c₁ to easily and quantitatively collect the specimen. In order to achieve this purpose, it is preferred that the specimen-exhausting outlet 2c₁₂ is formed in such a way that the specimen-exhausting outlet 2c₁₂ is smaller than the specimen-introducing inlet 2c₁₁.

Next, the thread 3a₁ which is formed in the internal peripheral portion 3a of the cover member 3 is threadedly engaged with the thread 4a₂₁ which is formed in the external peripheral portion 4a₂ of the other end portion of the connection portion 4a in the intermediary unit 4, and the user rotates the cover member 3 in the direction in which the cover member 3 is threadedly fastened to the intermediary unit 4. As a result, the cover member 3 is threadedly fastened to the intermediary unit 4 and the picked specimen is preserved in the container body 1 with the container body 1 in an air-tight state. At this point, the annular member 7 is pushed up up to the position at which the inside surface of the annular member 7 meets the projection 2d, and the position of the annular member 7 is fixed by the projection 2d.

In the case of analyzing the specimen which is contained in the preservation liquid in the container for specimen using a general-purpose apparatus for physical and chemical analysis, the intermediary unit 4 is rotated relative to the container body 1 in the direction in which the intermediary unit 4 is released from the container body 1 which is threadedly fitted to the intermediary unit 4. As a result, the intermediary unit 4 moves in the direction in which the intermediary unit 4, together with the cover member 3 connecting with the specimen-picking rod 2, is removed from the external peripheral portion 1a of the open end of the container body 1, through the thread 1b₁ which is formed in the internal peripheral portion 1a of the open end of the container body 1 and the thread 4a₁₁ which is formed in the external peripheral portion 4a₁ of the one end portion of the connection portion 4a in the intermediary unit 4. As a result, only the container body 1 which holds the specimen that is dispersed in the preservation liquid is removed from the container for specimen. Next, the removed container body 1 is mounted in an apparatus for physical and chemical analysis (which is not shown in the drawings). Accordingly, the cover member 3 and the intermediary unit 4 can be easily removed from the container body 1 without deteriorating the quantitative capability by mixing the surplus part of the specimen which is scrubbed away by the intermediary unit 4.

Accordingly, according to the container for specimen of the eleventh embodiment, it is possible to quantitatively pick a specimen the amount of which is sufficient for test, and it is possible to mount the container body in a general-purpose apparatus for physical and chemical analysis as it stands without transferring a collected and preserved specimen from the container body to another container even in a test requiring the extraction of nucleic acids with a general-purpose apparatus for physical and chemical analysis like a centrifuge. In addition, it is possible to use for test a specimen that is quantified within a predetermined acceptable range and has a sufficient picking amount.

Also, according to the container for specimen of the eleventh embodiment, a specimen is pinched by the forked portion to be picked. Accordingly, for example, in the case of collecting a stool which is excreted on a sheet floating on water in a bowl, there is no situation in which the sheet sinks so that the stool is made to come in contact with water in the bowl or in which the stool rolls down into water in the bowl, and the specimen-picking rod is easy to insert into a specimen like stool without applying large force to the specimen-picking rod. In addition, such specimen-picking rod makes it easy to collect a sufficient amount of a stool sample to detect a cancer cell or the like even though the stool is hard.

### Twelfth Embodiment

Fig. 23 is an explanatory view showing the constitution of a container for specimen according to a twelfth embodiment of the present invention, Figs. 24 and 25 are an explanatory view showing the constitutions of the cover member and specimen-picking rod in the container for specimen shown in Fig. 23, Fig. 26 is a view in which the cover member and specimen-picking rod shown in Fig. 24 (b) are viewed from the lower side. Fig. 27 is an explanatory view showing one example of variations of the annular portion-fixing means in the cover member and specimen-picking rod of the container for specimen according to the twelfth embodiment. Besides, the components of the container for specimen of the twelfth embodiment which are the same as those of the container for specimen of the eleventh embodiment are given the same reference numerals in the drawings, and the detailed explanations for the components are omitted.

The container for specimen of the twelfth embodiment is an example of variations of the container for specimen of the eleventh embodiment with regard to the forked portion of the specimen-picking rod. In the container for specimen of the twelfth embodiment, the specimen-picking rod 2 includes a forked portion 2c' which is placed on the opposite side to the rear end 2b connecting with the cover member 3. The forked portion 2c' is formed in such a way that: elastic force keeps the forked portion 2c' open; and the forked portion 2c' can be opened and closed on the front end of the specimen-picking rod 2. The elasticity of the material which the forked portion 2c' is made of is used for the elasticity of the forked portion 2c' of the specimen-picking rod 2. Besides, the forked portion 2c' may be capable of opening by forming a hinge joint in the forked portion 2c' and biasing the forked portion 2c' by a spring in the direction of the forked portion 2c' opening.

Also, instead of the specimen-holding portion 2c₁ which is shown in Fig. 19 and includes the specimen-introducing inlet 2c₁₁ and the specimen-exhausting outlet 2c₁₂, as shown in Fig. 25, the forked portion 2c' includes a groove 2c₂' and a shield portion 2c₁'. The groove 2c₂' is formed by placing at least two thin plate-shaped plane portions 2c₂₁' in the front end of one branching portion of the forked portion 2c' and along the direction of the forked portion 2c' opening or closing, and at least one groove 2c₂' is provided for the forked portion 2c'. (Besides, two grooves 2c₂' are provided for the forked portion 2c' in the example shown in Fig. 25 (b).) As shown in Fig. 26, the thin plate-shaped plane potions 2c₂₁' become thinner and thinner towards the side on which the one branching portion of the forked portion 2c' touches the other branching portion of the forked portion 2c' when the forked portion 2c' is closed. The shield portion 2c₁' is placed in the front end of the other branching portion of the forked portion 2c' and is provided with a thin plate-shaped plane portion 2c₁₁' that protrudes towards the the-one-branching-portion side so that the thin plate-shaped plane portion 2c₁₁' covers the opening side 2c₂₂' of the groove 2c₂' along the direction of the forked portion 2c' opening or closing when the forked portion 2c' is closed. The other constitutions of the twelfth embodiment are approximately the same as those of the eleventh embodiment.

According to the container for specimen of the twelfth embodiment which is formed in such a manner, when the forked portion 2c' is closed so that the shield portion 2c₁' covers the opening side 2c₂₂' of the groove 2c₂' along the direction of the forked portion 2c' opening or closing, the opening portion of the groove 2c₂' on the side on which the one branching portion of the forked portion 2c' touches the other branching portion of the forked portion 2c' in closing the forked portion 2c' functions as the specimen-introducing inlet in the container for specimen of the eleventh embodiment, the opening portion of the groove 2c₂' on the side opposite to the side on which the one branching portion of the forked portion 2c' touches the other branching portion of the forked portion 2c' in closing the forked portion 2c' functions as the specimen-exhausting outlet in the container for specimen of the first embodiment, and a space which is surrounded by the groove 2c₂' and shield portion 2c₁' functions as the specimen-holding portion in the container for specimen of the eleventh embodiment. As a result, a collected specimen can be fragmented into an enough size to easily disperse the specimen in liquid of the container body 1. In addition, a surplus part of the specimen can be exhausted from the specimen-exhausting outlet of the groove 2c₂' on the side opposite to the side on which the one branching portion of the forked portion 2c' touches the other branching portion of the forked portion 2c' in closing the forked portion 2c', so that the specimen can be quantitatively collected and be preserved in the container body 1 by inserting the specimen-picking rod 2 into the through hole 4b₁ with the forked portion 2c' closed.

Also, the thin plate-shaped plane potions 2c₂₁' which form the groove 2c₂' are formed in such a way that the thin plate-shaped plane potions 2c₂₁' become thinner and thinner towards the side on which the one branching portion of the forked portion 2c' touches the other branching portion of the forked portion 2c' when the forked portion 2c' is closed. As a result, when the forked portion 2c' is closed, a picked specimen becomes easy to introduce into the groove 2c₂' from the opening portion of the groove 2c₂' on the side on which the one branching portion of the forked portion 2c' touches the other branching portion of the forked portion 2c' in closing the forked portion 2c' toward the opening portion of the groove 2c₂' on the side opposite to the side on which the one branching portion of the forked portion 2c' touches the other branching portion of the forked portion 2c' in closing the forked portion 2c'.

Also, the shield portion 2c₁' is provided with a thin plate-shaped plane portion 2c₁₁' that protrudes towards the one branching portion including the groove 2c₂' so that the thin plate-shaped plane portion 2c₁₁' covers the opening side 2c₂₂' of the groove 2c₂' along the direction of the forked portion 2c' opening or closing. As a result, when a specimen is pinched to be picked by closing the forked portion 2c', the specimen can be scooped up with the bottom portion of the specimen supported by the shield portion 2c₁'. Accordingly, a specimen like stool becomes easy to pick more surely in the case of collecting a stool which is excreted on a sheet floating on water in a bowl.

In addition, when the forked portion 2c' is opened in the container body, the opening side 2c₂₂' of the groove 2c₂' along the direction of the forked portion 2c' opening or closing is not covered by the shield portion 2c₁', so that it is possible to make a large area with which a specimen like stool picked by the groove 2c₂' comes in contact with liquid in the container body 1. As a result, a specimen picked by the specimen-picking rod 2 becomes yet easier to disperse in liquid in the container body 1.

The other operation effects of the container for specimen of the twelfth embodiment are approximately the same as those of the container for specimen of the eleventh embodiment. Besides, as shown as a variation example in Fig. 27, the position of the annular portion 7 may be fixed in such a way that: a flange 8 for fixing the specimen-picking rod 2 to the cover member 3 is made to protrude toward the specimen-picking rod 2 instead of providing the projection 6; and the external peripheral surface of the flange 8 is made to slidably come in contact with the internal peripheral surface of the annular member 7 so that the position of the annular member 7 is fixed. Also, this constitution is applicable to the container for specimen of the eleventh embodiment.

### Thirteenth Embodiment

Fig 28 is an explanatory view showing the constitution of a main part of a container for specimen according to a thirteenth embodiment of the present invention, Fig. 29 is a view in which the cover member and specimen-picking rod shown in Fig. 28 are viewed from the lower side. Besides, the components of the container for specimen of the thirteenth embodiment which are the same as those of the containers for specimen of the eleventh and twelfth embodiments are given the same reference numerals in the drawings, and the detailed explanations for the components are omitted.

The container for specimen of the thirteenth embodiment is formed in such a way that the forked portion 2c" of the specimen-picking rod 2 combines the constitution of the forked portion 2c of the container body for specimen of the eleventh embodiment shown in Figs. 19 and 20 with the constitution of the forked portion 2c' of the container body for specimen of the twelfth embodiment shown in Figs. 24 to 26.

In a more detailed explanation, as shown in Fig. 28, the forked portion 2c" includes: at least one groove 2c₂' which is formed by placing at least two thin plate-shaped plane portions 2c₂₁' in the front end of one branching portion of the forked portion 2c" and along the direction of the forked portion 2c" opening or closing; a specimen-holding portion 2c₁ which is placed in the other branching portion of the forked portion 2c" and is provided with a specimen-introducing inlet 2c₁₁ that is formed on the side on which the one branching portion of the forked portion 2c" touches the other branching portion of the forked portion 2c" in closing the forked portion 2c" and a specimen-exhausting outlet 2c₁₂ that is formed on the side opposite to the side on which the specimen-introducing inlet 2c₁₁ is formed; and a shield portion 2c₁' which is placed in the front end of the other branching portion of the forked portion 2c" and is provided with a thin plate-shaped plane portion 2c₁₁' that protrudes towards the the-one-branching-portion side so that the thin plate-shaped plane portion 2c₁₁' covers the opening side 2c₂₂' of the groove 2c₂' along the direction of the forked portion 2c" opening or closing when the forked portion 2c" is closed.

Also, as shown in Fig. 29, in the at least one groove 2c₂', at least one thin plate-shaped plane portion 2c₂₁₁' which forms the groove 2c₂' protrudes toward the the-other-branching-portion side so that the thin plate-shaped plane portion 2c₂₁₁' is contained by the specimen-holding portion 2c₁ when the forked portion 2c" is closed. Also, the thin plate-shaped plane potions 2c₂₁' are formed in such a way that the thin plate-shaped plane potions 2c₂₁' become thinner and thinner towards the side on which the one branching portion of the forked portion 2c" touches the other branching portion of the forked portion 2c" when the forked portion 2c" is closed.
The other constitutions of the thirteenth embodiment are approximately the same as those of the eleventh and twelfth embodiments.

According to the container for specimen of the thirteenth embodiment which is formed in such a manner, the forked portion 2c" of the specimen-picking rod 2 combines: the constitution of the forked portion of the specimen-picking rod in the above-described container body for specimen of the eleventh embodiment which is provided with the specimen-holding portion; and the constitution of the forked portion of the specimen-picking rod in the above-described container body for specimen of the twelfth embodiment which is provided with the groove and the shield portion, so that the same effects as those are obtained in the eleventh and twelfth embodiments respectively can be obtained in the container for specimen of the thirteenth embodiment.

In addition, the thin plate-shaped plane portions 2c₂₁₁' are made to go in the specimen-holding portion 2c₁ to be contained by the specimen-holding portion 2c₁, so that a fragmented specimen can be fragmented yet more and the specimen which is picked by the specimen-picking rod 2 becomes yet easier to disperse in liquid of the container body 1. In addition, the above constitution matter of the thin plate-shaped plane portions 2c₂₁' contained by the specimen-holding portion 2c₁ is coupled with the constitution matter of the thin plate-shaped plane potions 2c₂₁' becoming thinner and thinner towards the side on which the one branching portion of the forked portion 2c" touches the other branching portion of the forked portion 2c" when the forked portion 2c" is closed, and a specimen which is fragmented by making the specimen-holding portion hold the specimen becomes yet easier to fragment, so that the specimen which is picked by the specimen-picking rod 2 becomes yet easier to disperse in liquid of the container body 1. The other operation effects of the container for specimen of the thirteenth embodiment are approximately the same as those of the container for specimen of the eleventh and twelfth embodiments.

### Fourteenth Embodiment

Fig. 30 is an explanatory view showing the constitution of a container for specimen according to a fourteenth embodiment of the present invention, Fig. 31 is an explanatory view showing the constitutions of the cover member and specimen-picking rod in the container for specimen shown in Fig. 30, Fig. 32 is an explanatory view showing the constitutions of main parts of the cover member and specimen-picking rod shown in Fig. 31, Fig. 33 is a sectional view of the cover member and specimen-picking rod shown in Fig. 31, Fig. 34 is an explanatory view showing the constitution of a main part of the specimen-picking rod of the fourteenth embodiment, and Fig. 35 is a view in which the cover member and specimen picking rod of the fourteenth embodiment are viewed from the lower side. Besides, the components of the container for specimen of the fourteenth embodiment which are the same as those of the containers for specimen of the eleventh to thirteenth embodiments are given the same reference numerals, and the detailed explanations for the components are omitted.

In the container for specimen of the fourteenth embodiment, as shown in Fig. 31 (a), the rear end 2b of the specimen-picking rod 2 is provided with a flange 2b₁. Also, as shown in Figs. 32 (b) and 32 (c), the specimen-picking rod 2 includes a pair of guide grooves 2e which are provided for each of the branching portions of the forked portion 2c approximately along the longitudinal direction of the specimen-picking rod 2 and on the side on which the one branching portion of the forked portion 2c touches the other branching portion of the forked portion 2c when the forked portion 2c is closed. As shown in Figs. 34 (b) and 34 (d), the pair of the guide grooves 2e includes a groove 2e₁ with a T-shaped cross section and a second groove 2e₂ which continues to the one edge of the T-shaped groove 2e₁. In addition, as shown in Figs. 34 (a) and 34 (c), the pair of the guide grooves 2e is provided in such a way that the guide grooves 2e incline relative to the longitudinal direction of the specimen-picking rod 2 so that the T-shaped grooves 2e₁ and the bottom surfaces 2e₂₁ of the second grooves 2e₂ become shallower and shallower towards the base (the upper end) of the forked portion 2c.

Also, the external peripheral portion of the upper portion of each of the branching portions of the forked portion 2c is provided with a shaft 2f. In addition, as shown in Fig. 33 (a) and (b), the trunk portion 2bc of the specimen-picking rod 2 which runs from the forked portion 2c to the rear end 2b is formed in a shape of a cylinder the diameter of which is larger than that of a below-described male screw, and the trunk portion 2bc has a hollow structure. As shown in Fig. 32 (a), the lower portion of the trunk portion 2bc is provided with an open and close supporting portion 2f which supports the forked portion 2c in such a way that the forked portion 2c can open and close. The open and close supporting portion 2f is composed of a pair of shaft holes through which the shafts 2g that are provided for the forked portion 2c are passed.

As shown in Fig. 31, the cover member 3 includes an annular holding portion 3b by which the flange 2b₁ at the rear end 2b is rotatably held. In addition, the cover member 3 includes: a shaft-shaped male screw 3c which is fixed in the middle of the cover member 3 and runs towards the front end of the specimen-picking rod 2 with the male screw 3c having a predetermined length; a female screw 3d into which the male screw 3c is screwed and the diameter of which has a size by which the second grooves 2e₂ of the both guide grooves 2e can hold the female screw 3d when the male screw 3c is threadedly engaged with female screw 3d at a predetermined position (P1) in the male screw 3c (refer to Fig. 34 (c)) and the forked portion 2c is closed, as shown in Fig. 34 (c) and 34 (d); and T-shaped portions 3d₁ which are placed on the external peripheral surface of the female screw 3d with the T-shaped portions 3d₁ opposite to each other and are freely fitted to the T-shaped grooves 2e₁ of the guide grooves 2e of the forked portion 2c respectively, as shown in Fig. 32 (d). In Fig. 32 (d), the female screw 3d is formed in such a way that the radius of the female screw 3d is approximately as large as the diameter of the second grooves 2e₂ at the predetermined position (P1).

And, in the container for specimen of the fourteenth embodiment, the forked portion 2c is made to open and close by rotating the cover member 3 relative to the specimen-picking rod 2, as described below. Besides, the rotation direction of the cover member 3 in closing the forked portion 2c corresponds with the rotation direction in which the cover member 3 is unfastened from (the thread 4a₂₁ of) the external peripheral portion 4a₂ of the other end of the connection portion 4a of the intermediary unit 4 which is shown in Fig. 30. The other constitutions of the container for specimen of the fourteenth embodiment are approximately the same as those of the containers for specimen of the eleventh to thirteenth embodiments.

Besides, a procedure for fitting the specimen-picking rod 2 to the cover member 3 in the container for specimen of the fourteenth embodiment is as follows for example.
First, the male screw 3c which is fixed to the cover member 3 is passed into the trunk portion 2bc from the flange-2b₁-of-the-rear-end-2b side. Next, the annular holding portion 3b is fixed to the cover member 3 in such a way that the flange 2b₁ is surrounded by the annular holding portion 3d so that the trunk portion 2bc can freely rotate about the male screw 3c. Next, the female screw 3d is threadedly engaged with the male screw 3c. Next, the T-shaped portions 3d₁ of the female screw 3d are fitted into the grooves 2e₁ having T-shaped cross section from the end sides of the bases of a pair of the branching portions of the forked portion 2c, respectively. Next, the base of the forked portion 2c is inserted into the inside of the trunk portion 2bc, the shafts 2g which are provided for the branching portions of the forked portion 2c respectively are passed into the shafts holes of the open and close supporting portions 2f that are provided on the lower portion of the trunk portion 2bc, and the forked portion 2c are fitted to the trunk portion 2bc in such a way that the forked portion 2c can be opened and closed relative to the trunk portion 2bc. As a result, the specimen-picking rod 2 is rotatably fitted to the cover member 3 and the forked portion 2c can be opened and closed by rotating the cover member 3 with the trunk portion 2bc held.

In the container for specimen of the fourteenth embodiment which is formed in such a manner, the cover member 3 is rotated relative to the specimen-picking rod 2, so that the male screw 3 which is fixed to the cover member 3 rotates. At this point, the T-shape portions 3d₁ are freely fitted to the T-shaped grooves 2e₁ that are provided for the guide grooves 2e of the forked portion 2c, so that the rotation of the female screw 3d is restrained. Accordingly, the female screw 3d is given force in the direction in which the female screw 3d moves along the axial direction of the male screw 3c by the threaded engagement of the female and male screws 3d and 3c. And, as a part of the external peripheral portion of the female screw 3d slides on the second grooves 2e₂, or as parts of the T-shaped portions 3d₁ slide in the T-shaped grooves 2e₁ at the same time when the part of the external peripheral portion of the female screw 3d slides on the second grooves 2e₂, the female screw 3d is guided by the guide grooves 2e. At this point, as shown in Fig. 34 (a) and 34 (c), the pair of the guide grooves 2e is provided in such a way that the guide grooves 2e incline relative to the longitudinal direction of the specimen-picking rod 2 so that the T-shaped grooves 2e₁ and the bottom surfaces 2e₂₁ of the second grooves 2e₂ become shallower and shallower towards the base (the upper end) of the forked portion 2c.

As a result, when the cover member 3 is rotated in the direction of the forked portion 2c opening, the female screw 3c moves toward the base (the upper end) of the forced portion 2c. At this point, the T-shaped grooves 2e₁ and the bottom surfaces 2e₂₁ of the second grooves 2e₂ become shallower and shallower, so that a part of the external peripheral surface of the female screw 3c pushes the bottom surfaces 2e₂₁ of the second grooves 2e₂, or parts of the T-shaped portions 3d₁ push the bottom surfaces 2e₁₁ of the T-shaped grooves 2e₁ while the part of the external peripheral surfaces of the female screw 3d push the bottom surfaces 2e₂ of the second grooves 2e₂. As a result, the forked portion 2c opens more and more.

On the other hand, when the cover member 3 is rotated in the direction of the forked portion 2c closing, the female screw 3d moves toward the front end of the forced portion 2c. At this point, the T-shaped grooves 2e₁ and the bottom surfaces 2e₂₁ of the second grooves 2e₂ become deeper and deeper, so that force with which the part of the external peripheral surface of the female screw 3c pushes the bottom surfaces 2e₂₁ of the second grooves 2e₂ is weakened, and, in addition, a part of the T-shaped portion 3d₁ pushes the internal surfaces 2e₁₂ of the T-shaped grooves 2e₁. As a result, the branching portions of the forked portion 2c pivot so that the forked portion 2c is closed, and then the forked portion 2c is closed when the female screw 3c is located at the predetermined position (P1) of the male screw 3c.

As described above, the forked portion 2c opens and closes by the rotation of the cover member 3. Besides, in the example shown in Figs. 30 to 34, the screws are formed in such a way that the male screw 3c makes the forked portion 2c open in the clockwise rotation and the male screw 3c makes the forked portion 2c close in the counterclockwise rotation, and the screws have the same thread direction as that of the thread 3a₁ in the cover member 3 which is shown in Fig. 30 and threadedly is engaged with (the thread 4a₂₁ of) the external portion 4a₁ of the other end of the connection portion 4a of the intermediary unit 4.

Accordingly, according to the container for specimen of the fourteenth embodiment, the cover member 3 can be rotated relative to the specimen-picking rod 2. As a result, the container for specimen of the fourteenth embodiment operates like the containers for specimen of the eleventh to thirteenth embodiments. In the case where the cover member 3 is first fitted to the container for specimen, the cover member 3 is first rotated in the direction of the forked portion 2c closing (or, in the counterclockwise direction) while the trunk portion 2bc (or, the portion running from the base of the forked portion 2c to the rear end 2b) of the specimen-picking rod 2 is held, and the female screw 3d is made to move up to a predetermined position (P1) on the the-front-end-of-the-male-screw-3c side. As a result, the forked portion 2c becomes closed.

Next, the specimen-picking rod 2 is pushed into the through hole 4b₁ of the intermediary unit 4 shown in Fig. 13 to be inserted into the through hole 4b₁, with the forked portion 2a closed. As a result, the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the cover member 3 is rotated in the direction in which the cover member 3 is threadedly attached to the intermediary unit 4 (or, in the clockwise direction, that is to say, in the direction of the forked portion 2c opening). As a result, the cover member 3 is threadedly attached to the intermediary unit 4. At this point, the trunk portion 2bc of the specimen-picking rod 2 is squeezed by the penetration wall 4b of the intermediary unit 4, so that the specimen-picking rod 2, together with the cover member 3, cannot be rotated. As a result, the female screw 3d moves away from the front end of the male screw 3c, and the forked portion 2c is opened in accordance with the movement distance of the female screw 3d. Accordingly, in an air-tight state of the container for specimen before picking a specimen, the forked portion 2a of the specimen-picking rod 2 is in an open state, as shown in Fig. 30.

In the case of picking a specimen, the cover member 3 is rotated in the direction in which the cover member 3 is released from the threaded attachment of the intermediary unit 4 to the cover member 3 (or, in the counterclockwise direction, that is to say, in the direction of the forked portion 2c closing), so that the thread 3a₁ which is formed in the internal peripheral portion 3a is released from the threaded engagement of the thread 3a₁ with the thread 4a₂₁ which is formed in the external peripheral portion 4a₂ of the other end of the connection portion 4a in the intermediary unit 4. At this point, the female screw 3d is located at a predetermined position (P1) in the vicinity of the front end of the male screw 3c (refer to Fig. 34 (c)), and the forked portion 2c is in a close state.

In picking a specimen, a user rotates the cover member 3 in the direction of the forked portion 2c opening while holding the trunk portion 2bc of the specimen-picking rod 2, so that the female screw 3d moves away from the front end of the male screw 3c, and the forked portion 2c is opened in accordance with the movement distance of the female screw 3d. Next, the user holds the cover member 3 and approximates the specimen-picking rod 2 to a position at which a specimen can be pinched by the forked portion 2c. Next, the user rotates the cover member 3 in the direction of the forked portion 2c closing while holding the trunk portion 2bc of the specimen-picking rod 2. As a result, when the female screw 3d moves towards the vicinity of the front end of the male screw 3c and to the predetermined position (P1), the forked portion 2c is in a close state, and the specimen is held by the specimen-holding portion that is surrounded by the groove 2c₂' and the shield portion 2c₁', with the specimen fragmented.

After picking the specimen, the user pushes the specimen-picking rod 2 into the through hole of the intermediary unit 4 to insert the specimen-picking rod 2 into the through hole 4b₁ with the forked portion 2c closed, so that the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the user rotates the cover member 3 in the direction in which the cover member 3 is threadedly attached to the intermediary unit 4 (or, in the clockwise direction, that is to say, in the direction of the forked portion 2c opening). As a result, the cover member 3 is threadedly attached to the intermediary unit 4 and the picked specimen is preserved in the container body 1 with the container body 1 in an air-tight state. At this point, the trunk portion 2bc of the specimen-picking rod 2 is squeezed by the penetration wall 4b of the intermediary unit 4, so that the specimen-picking rod 2, together with the cover member 3, cannot be rotated. As a result, the female screw 3d moves away from the front end of the male screw 3c, the forked portion 2c is opened in accordance with the movement distance of the female screw 3d, and the picked specimen is dispersed in liquid in the container body 1.

According to the container for specimen of the fourteenth embodiment, the rotation of the cover member 3 makes it possible to adjust the forked portion 2c to a desired state of the forked portion 2c opening or closing in accordance with the sizes of specimens to be picked. The other effects of the container for specimen of the fourteenth embodiment are approximately the same as those of the containers for specimen of the eleventh to thirteenth embodiments.

Besides, in the example shown in Figs. 30 to 35, the male screw 3c, the female screw 3d, the T-shaped portion 3d₁, the guide groove 2e, the T-shaped groove 2e₁, the flange 2b₁, the holding portion 3b, and so on are provided for the container for specimen and the forked portion 2c can be opened and closed by rotating the cover member 3 relative to the specimen-picking rod 2. However, the constitution of the container for specimen of the fourteenth embodiment is not limited to the example shown in Figs. 30 to 35, and the container for specimen of the fourteenth embodiment may have any constitutions which make it possible to open and close the forked portion 2c by rotating the cover member 3 relative to the specimen-picking rod 2. Also, in the example shown in Figs. 30 to 35, the front end of the forked portion 2c of the specimen picking rod 2 is formed in the same manner as that of the thirteenth embodiment. However, the front end of the forked portion 2c of the specimen picking rod 2 may be formed in the same manner as that of the eleventh or twelfth embodiment.

### Fifteenth Embodiment

Fig. 36 is an explanatory view showing the constitution of a container for specimen according to the fifteenth embodiment of the present invention, Fig. 37 is an explanatory view showing the constitutions of the cover member and specimen-picking rod, Fig. 38 is a sectional view of the cover member and specimen-picking rod, and Fig. 39 is an enlarged view of the connection region of the forked portion in the sectional view shown in Fig. 38. Fig. 40 is a view in which the cover member and specimen-picking rod are viewed from the lower side. Besides, the components of the container for specimen of the fifteenth embodiment which are the same as those of the containers for specimen of the eleventh to thirteenth embodiments are given the same reference numerals, and the detailed explanations for the components are omitted.

In the container for specimen of the fifteenth embodiment, as shown in Fig. 36 (a), the specimen-picking rod 2 consists of two members 2-1 and 2-2. The two members 2-1 and 2-2 are connected with each other through at least one headed pin-shaped connection member 2-3 at the middle portions of the two members 2-1 and 2-2, the two members 2-1 and 2-2 can open and close with the shaft 2-3b of the headed pin-shaped connection member 2-3 as a fulcrum, and the portions 2-1a and 2-2a which run from the front ends of the two members 2-1 and 2-2 to the fulcrum (the shaft 2-3b) form a forked portion. Besides, because the constitution of the front end of the forked portion 2-1a and 2-2a is approximately the same as that of the front end of the forked portion 2a in the container for specimen of the fourteenth embodiment, the detailed explanation for the forked portion 2-1a and 2-2a in the fifteenth embodiment is omitted here.

As shown in Fig. 38 (a), the first member 2-1 of the two members includes: a first nick portion 2-1₁ which runs from the rear end to the vicinity of the fulcrum; and a shaft hole 2-1₂ which is placed at the position of the fulcrum. As shown in Fig. 38 (b), the second member 2-2 of the two members includes: a second nick portion 2-2₁ which runs from the rear end to the vicinity of the fulcrum and into which the portion of the first member 2-1 running from the rear end of the first member 2-1 to the vicinity of the fulcrum can be slidably inserted; and a shaft hole 2-2₂ which is placed at the position of the fulcrum.

As shown in Fig. 40, the cover member 3 includes a shaft-shaped supporting member 3e and a groove 3f. The shaft-shaped supporting member 3e is fixed in the middle of the cover member 3, has a proper diameter and length so that the supporting portion 3e can be inserted into the first nick portion 2-1₁ of the first member 2-1 as shown in Fig. 38 (a), and is formed in the shape of a shaft including an annular groove 3e₁ that can hold the head of the headed pin-shaped connection member 2-3 connecting the two members 2-1 and 2-2 in the circumferential direction. As shown in Figs. 36 and 37, the groove 3f is formed in the internal surface of the cover member 3 in such a way that the rear ends 2-1b and 2-2b of the two members 2-1 and 2-2 are slidably fitted to the groove 3f, and the groove 3f is formed in the shape of a swirl converging to the center of the cover member 3, as shown in Fig. 40.

As shown in Fig. 39, the headed pin-shaped connection member 2-3 is composed of a male screw 2-3₁ and a female screw 2-3₂. The male screw 2-3₁ includes a head 2-3₁₁, a shaft portion 2-3b, and a thread portion 2-3₁₂. The thread portion 2-3₁₂ is formed so that the thread portion 2-3₁₂ can be thrededly engaged with the female screw 2-3₂.
The female screw 2-3₂ forms the head of the headed pin-shaped connection member 2-3 when the female screw 2-3₂ is thrededly engaged with the thread portion 2-3₁₂ of the male screw 2-3₁.

And, in the container for specimen of the fifteenth embodiment, the forked portion 2-1a and 2-2a are opened by rotating the cover member 3 relative to the specimen-picking rod 2, as described below.
Besides, as shown in Fig. 40, the swirl direction of the groove 3f is provided in such a way that the groove 3f swirls counterclockwise and heads toward the center of the cover member 3 when the cover member 3 is viewed from the back side of the cover member 3, and the rotation direction of the cover member 3 in closing the forked portion 2-1a and 2-2a corresponds to the rotation direction of the cover member 3 in unfastening the cover member 3 from (the thread portion 4a₂₁ of) the external peripheral portion 4a₂ of the other end of the connection portion 4a of the intermediary unit 4 shown in Fig. 36. The other constitutions of the container for specimen of the fifteenth embodiment are approximately the same as those of the containers for specimen of the eleventh to thirteenth embodiments.

Besides, a procedure for fitting the specimen-picking rod 2 to the cover member 3 in the container for specimen of the fifteenth embodiment is as follows for example. First, the two female screws 2-3₂ are axisymmetrically fitted into the annular groove 3e₁ of the shaft-shaped supporting portion 3e that is fixed to the cover member 3. Next, the first nick portion 2-1₁ of the first member 2-1 is fitted to the shaft-shaped supporting portion 3e, the rear end 2-1b is fitted into the groove 3f, and, in addition, the position of the shaft hole 2-1₂ is adjusted so that the shaft hole 2-1₂ is located on the same axis as that of the female screw 2-3₂. Next, the second nick portion 2-2₁ of the second member 2-2 is fitted to the part of the first member 2-1 which runs from the rear end to the vicinity of the fulcrum, the rear end 2-2b is fitted into the groove 3f, and, in addition, the position of the shaft hole 2-2₂ is adjusted so that the shaft hole 2-2₂ is located on the same axis as that of the shaft hole 2-1₂ and the female screw 2-3₂. Next, the male screws 2-3₁ are inserted into the shaft holes 2-2₂, and the thread portions 2-3₁₂ are thrededly engaged with the female screws 2-3₂. As a result, the specimen-picking rod 2 is rotatably fitted to the cover member 3, and the forked portion 2-1a and 2-2a can be opened by rotating the cover member 3 while the trunk portion 2bc (the vicinity of the fulcrum of the specimen-picking rod 2) is held.

In the container for specimen of the fifteenth embodiment which is formed in such a way, when the cover member 3 is rotated relative to the specimen-picking rod 2, the rear ends 2-1b and 2-2b of the first and second members 2-1 and 2-2 are guided by the guide groove 3f which is formed in the shape of a swirl, respectively, so that the rear ends 2-1b and 2-2b approach or move away from the center of the cover member 3. At this point, the swirl-shaped groove 3f applies forces to the rear ends 2-1b and 2-2b of the first and second members 2-1 and 2-2 in such a way that the forces are applied in the radial direction of the cover member 3 and in the directions opposite to each other. In addition, at this point, the first and second members 2-1 and 2-2 are connected with the shaft-shaped supporting portion 3e through the headed pin-shaped connection member 2-3 so that the first and second members 2-1 and 2-2 can open and close, and the annular groove 3e₁ of the shaft-shaped supporting portion 3e is formed in such a way that the annular groove 3e₁ can rotate relative to the headed pin-shaped connection member 2-3. As a result, the forked portion 2-1a and 2-2a is opened or closed by the rotation of the cover member 3 with the fulcrum (the shaft portion 2-3b of the headed pin-shaped connection member 2-3) used as a shaft.

That is to say, when the cover member 3 is rotated in the direction of the forked portion 2-1a and 2-2a opening (or, in the clockwise direction), the rear ends 2-1b and 2-2b of the first and second members 2-1 and 2-2 are guided by the groove 3f which is formed in the shape of a swirl, and the rear ends 2-1b and 2-2b counterclockwise turn around the center of the cover member 3 to move away from the center of the cover member 3. As a result, the forked portion 2-1a and 2-2a opens more and more.

On the other hand, when the cover member 3 is rotated in the direction of the forked portion 2-1a and 2-2a closing (or, in the counterclockwise direction), the rear ends 2-1b and 2-2b of the fires and second members 2-1 and 2-2 are guided by the groove 3f which is formed in the shape of a swirl, and the rear ends 2-1b and 2-2b clockwise turn around the center of the cover member 3 to approach the center of the cover member 3. As a result, the forked portion 2-1a and 2-2a closes more and more. And, when the rear ends 2-1b and 2-2b of the first and second members 2-1 and 2-2 are located at predetermined positions in the vicinity of the center of the cover member 3 in the groove 3f which is formed in the shape of a swirl, the forked portion 2-1a and 2-2a is completely closed.

As described above, the forked portion 2-1a and 2-2a is opened or closed by the rotation of the cover member 3. Besides, in the example shown in Fig. 40, the swirl direction of the groove 3f is set so that the forked portion 2-1a and 2-2a is opened by the clockwise rotation and is closed by the counterclockwise rotation, and the swirl direction of the groove 3f corresponds to the thread direction of the thread 3a₁ of the cover member 3 that is threadedly engaged with (the thread 4a₂₁ of) the external peripheral portion 4a₂ of the other end of the connection portion 4a of the intermediary unit 4 shown in Fig. 36.

Accordingly, according to the container for specimen of the fifteenth embodiment, the cover member 3 can be rotated relative to the specimen-picking rod 2, so that the container for specimen of the fifteenth embodiment operates like the containers for specimen of the eleventh to thirteenth embodiments. In the case where the cover member 3 is first fitted to the container for specimen, the cover member 3 is first rotated in the direction of the forked portion 2-1a and 2-2a closing (or, in the counterclockwise direction) while the vicinity of the fulcrum in the specimen-picking rod 2 (or, the vicinity of the headed pin-shaped connection member 2-3) is held, so that the rear ends 2-1b and 2-2b of the first and second members 2-1 and 2-2 are located in a predetermined place in the vicinity of the center of the cover member 3 in the groove 3f which is formed in the shape of a swirl. As a result, the forked portion 2-1a and 2-2a is in a close state.

Next, the specimen-picking rod 2 is pushed into the through hole 4b₁ of the intermediary unit 4 shown in Fig. 36 and is inserted into the through hole 4b₁, with the forked portion 2-1a and 2-2a closed, so that the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the cover member 3 is rotated in the direction in which the cover member 3 is threadedly attached to the intermediary unit 4 (or, in the clockwise direction, that is to say, in the direction of the forked portion 2-1a and 2-2a opening). As a result, the cover member 3 is threadedly attached to the intermediary unit 4. At this point, the vicinity of fulcrum of the specimen-picking rod 2 is squeezed by the penetration wall 4b of the intermediary unit 4, so that the specimen-picking rod 2, together with the cover member 3, cannot be rotated. As a result, the rear ends 2-1b and 2-2b of the first and second members 2-1 and 2-2 are guided by the groove 3f which is formed in the shape of a swirl, the rear ends 2-1b and 2-2b counterclockwise turn around the center of the cover member 3 to move away form the center of the cover member 3, and the forked portion 2-1a and 2-2a is opened in accordance with the movement distances of the rear ends 2-1b and 2-2b. As described above, in a state in which the container for specimen is in an air-tight state before picking a specimen, the forked portion 2-1a and 2-2a of the specimen-picking rod 2 is in an open state, as shown in Fig. 36 (a).

In the case of picking a specimen, the cover member 3 is rotated in the direction in which the cover member 3 is released from the threaded attachment of the cover member 3 to the intermediary unit 4 (or, in the counterclockwise direction, that is to say, in the direction of the forked portion 2-1a and 2-2a closing), so that the thread 3a₁ which is formed in the internal peripheral portion 3a is released from the threaded engagement of the thread 3a₁ with the thread 4a₂₁ which is formed in the external peripheral portion 4a₂ of the other end of the connection portion 4a in the intermediary unit 4. At this point, the rear ends 2-1b and 2-2b of the first and second members 2-1 and 2-2 are located in a predetermined place in the vicinity of the center of the cover member 3 in the groove 3f which is formed in the shape of a swirl, and the forked portion 2-1a and 2-2a is in a close state.

In picking a specimen, a user rotates the cover member 3 in the direction of the forked portion 2-1a and 2-2a opening while holding the vicinity of the fulcrum in the specimen-picking rod 2. As a result, the rear ends 2-1b and 2-2b of the first and second members 2-1 and 2-2 are guided by the swirl-shaped groove 3f to move away from the vicinity of the center of the cover member 3, and the forked portion 2-1a and 2-2a is opened in accordance with the movement distances of the rear ends 2-1b and 2-2b. Next, the user holds the vicinity of the fulcrum in the specimen-picking rod 2 and approximates the specimen-picking rod 2 to a position at which a specimen can be pinched by the forked portion 2-1 a and 2-2a. Next, the user rotates the cover member 3 in the direction of the forked portion 2-1a and 2-2a closing while holding the vicinity of the fulcrum in the specimen-picking rod 2. As a result, the rear ends 2-1b and 2-2b of the first and second members 2-1 and 2-2 are guided by the swirl-shaped groove 3f to move to the vicinity of the center of the cover member 3, the forked portion 2-1a and 2-2a is in a close state when the rear ends 2-1b and 2-2b move to predetermined positions, and the specimen is held by the specimen-holding portion that is surrounded by the groove 2a₂' and the shield portion 2a₁', with the specimen fragmented.

After picking the specimen, the user pushes the specimen-picking rod 2 into the through hole 4b₁ of the intermediary unit 4 to insert the specimen-picking rod 2 into the through hole 4b₁ with the forked portion 2-1a and 2-2a closed, so that the specimen-picking rod 2 is made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the user rotates the cover member 3 in the direction in which the cover member 3 is threadedly attached to the intermediary unit 4 (or, in the clockwise direction, that is to say, in the direction of the forked portion 2-1a and 2-2a opening). As a result, the cover member 3 is threadedly attached to the intermediary unit 4, and the picked specimen is held by the container body 1 with the container body 1 air-tight. At this point, the vicinity of fulcrum of the specimen-picking rod 2 is squeezed by the penetration wall 4b of the intermediary unit 4, so that the specimen-picking rod 2, together with the cover member 3, cannot be rotated. As a result, the rear ends 2-1b and 2-2b of the first and second members 2-1 and 2-2 move away from the vicinity of the center of the cover member 3 in the groove 3f which is formed in the shape of a swirl, the forked portion 2-1a and 2-2a is opened in accordance with the movement distances of the rear ends 2-1b and 2-2b, and the picked specimen is dispersed in liquid in the container body 1.

According to the container for specimen of the fifteenth embodiment, the rotation of the cover member 3 makes it possible to adjust the forked portion 2-1a and 2-2a to a desired state of the forked portion 2-1a and 2-2a opening and closing in accordance with the sizes of specimens to be picked. The other operation effects of the container for specimen of the fifteenth embodiment are approximately the same as those of the containers for specimen of the eleventh to thirteenth embodiments.

Besides, in the example shown in Figs. 36 to 40, the first member 2-1, the first nick portion 2-1₁, the shaft hole 2-1₂, the second member 2-2, the second nick portion 2-2₁, the shaft hole 2-2₂, the headed pin-shaped connection member 2-3, the shaft-shaped supporting portion 3e, the annular groove 3e₁, the swirl-shaped groove 3f and so on are provided for the container for specimen and the forked portion 2-1a and 2-2a can be opened and closed by rotating the cover member 3 relative to the specimen-picking rod 2. However, the constitution of the container for specimen of the fifteenth embodiment is not limited to the example shown in Figs. 36 to 40, and the container for specimen of the fifteenth embodiment may have any constitutions which make it possible to open and close the forked portion 2-1a and 2-2a by rotating the cover member 3 relative to the specimen-picking rod 2. Also, in the example shown in Figs. 36 to 40, the front end of the forked portion 2-1a and 2-2a of the specimen picking rod 2 is formed in the same manner as that of the thirteenth embodiment. However, the front end of the forked portion 2-1a and 2-2a of the specimen picking rod 2 may be formed in the same manner as that of the eleventh or twelfth embodiment.

### Sixteenth Embodiment

Fig. 41 is an explanatory view showing the constitution of a container for specimen according to the sixteenth embodiment of the present invention, Fig. 42 is an explanatory view showing the constitutions of the cover member and specimen-picking rod in the container for specimen shown in Fig. 41, and Fig. 43 is a sectional view of the cover member and specimen-picking rod. Besides, the components of the containers for specimen of the sixteenth embodiment which are the same as those of the containers for specimen of the eleventh to thirteenth embodiments are given the same reference numerals, and the detailed explanations for the components are omitted.

In the container for specimen of the sixteenth embodiment, the internal surface 3g of the cover member 3 which is provided with the swirl-shaped groove 3f is formed in the shape of a cone. The other constitutions of the containers for specimen of the sixteenth embodiment are approximately the same as those of the container for specimen of the fifteenth embodiment.

According to the container for specimen of the sixteenth embodiment, the internal surface 3g of the cover member 3 is formed in the shape of a cone. As a result, the opening and closing operation of the forked portion 2-1a and 2-2a which is related to the rotation of the cover member 3 can be made to become all the more slow because the radius of the back surface of the cover member 3, or the length of the groove 3f which is provided in the shape of a swirl running from the center to the edge becomes large. Accordingly, a specimen becomes easy to collect. The other operation effects of the container for specimen of the sixteenth embodiment are approximately the same as those of the container for specimen of the fifteenth embodiment.

### Seventeenth Embodiment

Fig. 44 is an explanatory view schematically showing the constitutions of the cover member and specimen-picking rod in a container for specimen according to the seventeenth embodiment of the present invention. Besides, the components of the containers for specimen of the seventeenth embodiment which are the same as those of the containers for specimen of the eleventh to thirteenth embodiments are given the same reference numerals, and the detailed explanations for the components are omitted.

In the container for specimen of the seventeenth embodiment, as shown in Fig. 44 (a), 44 (b), and 44(e), the internal surface 3g of the cover member 3 is formed in the shape of a cone. In addition, the internal surface 3g is provided with a groove 3h which: is formed in the shape of a spiral converging to the center of the cover member 3; and has a T-shaped cross section. Also, as shown in Fig. 44 (a), the specimen-picking rod 2 is composed of two members 2-1' and 2-2'. The two members 2-1' and 2-2' are connected with each other through at least one headed pin-shaped connection member 2-3' at the middle portions of the two members 2-1' and 2-2', so that the specimen-picking rod 2 can open and close with the shaft of the headed pin-shaped connection member 2-3' as a fulcrum (where the shaft of the headed pin-shaped connection member 2-3' is not shown in Fig. 44). And, the portions 2-1a' and 2-2a' that run from the front ends of the two members 2-1a' and 2-2a' to the fulcrum (the shaft of the headed pin-shaped connection member 2-3') respectively form a forked portion. Besides, because the constitution of the front end of the forked portion 2-1a' and 2-2a' is approximately the same as that of the front end of the forked portion 2c in the container for specimen of the fourteenth embodiment, the detailed explanation for the constitution of the front end of the forked portion 2-1a' and 2-2a' is omitted here.

As shown in Fig. 44 (c) and 44(d), the two members 2-1' and 2-2' are formed in the shapes that enable the two members 2-1' and 2-2' to overlap with each other over the range from the rear end to the vicinity of the fulcrum (or, the vicinity of the shaft of the headed pin-shaped connection member 2-3'), and the inside surfaces of the forked portion 2-1a' and 2-2a' are formed in the shapes that enable the inside surfaces of the forked portion 2-1 a' and 2-2a' to abut each other. Also, the rear ends of the two members 2-1' and 2-2' are provided with T-shaped portions 2-1b' and 2-2b' which are freely fitted to the T-shaped groove 3h of the cover member 3, respectively. Also, the two members 2-1' and 2-2' are provided with shaft holes 2-1₁' and 2-2₁' which are located at the position of the fulcrum, respectively. And, the shaft of the headed pin-shaped connection member 2-3' which is shown in Fig. 44 (a) is fitted into the shaft holes 2-1₁' and 2-2₁'.

Besides, the back side of the shaft of the headed pin-shaped connection member 2-3' shown in Fig. 44 (a) is swaged, so that the two members 2-1' and 2-2' cannot separate from the shaft of the headed pin-shaped connection member 2-3'. Also, the rotation direction of the cover member 3 in closing the forked portion 2-1a' and 2-2a' corresponds to the rotation direction of the cover member 3 in unfastening the cover member 3 from the other end of the connection portion of the intermediary unit (which is not shown in Fig. 44). The other constitutions of the container for specimen of the seventeenth embodiment are approximately the same as those of the containers for specimen of the first to third embodiments.

In the container for specimen of the seventeenth embodiment which is formed in such a way, when the cover member 3 is rotated relative to the specimen-picking rod 2, the T-shaped portions 2-1b' and 2-2b' which are provided for the two members 2-1' and 2-2' respectively are guided by the T-shaped groove 3h. At this point, the T-shaped groove 3h applies forces to the T-shaped portion 2-1b' of the first member 2-1' and the T-shaped portion 2-2b' of the second member 2-2' in such a way that the forces are applied in the radial direction of the cover member 3 and in the directions opposite to each other. In addition, at this point, the first and second members 2-1' and 2-2' are connected with each other through the headed pin-shaped connection member 2-3' so that the first and second members 2-1' and 2-2' can pivot. As a result, the forked portion 2-1a' and 2-2a' is opened or closed by the rotation of the cover member 3 with the fulcrum (the shaft portion 2-3b of the headed pin-shaped connection member 2-3') used as a shaft.

That is to say, when the cover member 3 is rotated in the direction of the forked portion 2-1a' and 2-2a' opening (or, in the clockwise direction), the T-shaped portions 2-1b' and 2-2b' are guided by the T-shaped groove 3h to move away from the center of the cover member 3. As a result, the forked portion 2-1a' and 2-2a' opens more and more. On the other hand, when the cover member 3 is rotated in the direction of the forked portion 2-1a' and 2-2a' closing (or, in the counterclockwise direction), the T-shaped portions 2-1b' and 2-2b' are guided by the T-shaped groove 3h to approach the vicinity of the center of the cover member 3. As a result, the forked portion 2-1a' and 2-2a' closes more and more. And, when the T-shaped portions 2-1b' and 2-2b' of the first and second members 2-1' and 2-2' are located in a predetermined place in the vicinity of the center of the cover member 3 through the T-shaped groove 3h, the forked portion 2-1a' and 2-2a' is completely closed.

As described above, the forked portion 2-1a' and 2-2a' is opened or closed by the rotation of the cover member 3. Besides, in the example shown in Fig. 44 (b), the spiral direction of the T-shaped groove 3h is set so that the forked portion 2-1a' and 2-2a' is opened by the clockwise rotation and is closed by the counterclockwise rotation, and the spiral direction of the T-shaped groove 3h corresponds to the thread direction of the thread 3a₁ of the cover member 3 that is threadedly engaged with (the thread 4a₂₁ of the external peripheral portion 4a₂ of) the other end of the connection portion 4a of the intermediary unit 4 shown in Fig. 21. Besides, because the intermediary unit of the container for specimen of the seventeenth embodiment is the same as the intermediary unit 4 of the container for specimen of the eleventh embodiment which is shown in Fig. 21, the intermediary unit is not shown in Fig. 44.

Accordingly, according to the container for specimen of the seventeenth embodiment, the cover member 3 can be rotated relative to the specimen-picking rod 2, so that the container for specimen of the seventeenth embodiment operates like the containers for specimen of the eleventh to thirteenth embodiments. That is to say, in the case where the cover member 3 is first fitted to the container for specimen, the cover member 3 is first rotated in the direction of the forked portion 2-1a' and 2-2a' closing (or, in the counterclockwise direction) while the vicinity of the fulcrum in the specimen-picking rod 2 (or, the vicinity of the headed pin-shaped connection member 2-3) is held, so that the T-shaped portions 2-1b' and 2-2b' of the first and second members 2-1' and 2-2' are located in a predetermined place in the vicinity of the center of the cover member 3 in the T-shaped groove 3h. As a result, the forked portion 2-1a' and 2-2a' is in a close state.

Next, the specimen-picking rod 2 is pushed into the through hole of the intermediary unit which is omitted in Fig. 44 and is inserted into the through hole, with the forked portion 2-1a' and 2-2a' closed, so that the specimen-picking rod 2 is made to penetrate through the through hole. At this point, the penetration wall comes into close contact with the side surface of the specimen-picking rod 2 through the through hole with the penetration wall elastically deforming, and the penetration wall separates a space into two divisions on the upper and lower sides.

Next, the thread 3a₁ which is formed in the internal peripheral portion 3a of the cover member 3 is threadedly engaged with the thread which is formed in the external peripheral portion of the other end of the connection portion in the intermediary unit which is omitted in Fig. 44, and the cover member 3 is rotated in the direction in which the cover member 3 is threadedly attached to the intermediary unit (or, in the clockwise direction, that is to say, in the direction of the forked portion 2-1a' and 2-2a' opening). As a result, the cover member 3 is threadedly attached to the intermediary unit which is omitted in Fig. 44. At this point, the vicinity of fulcrum of the specimen-picking rod 2 is squeezed by the penetration wall of the intermediary unit which is omitted in Fig. 44, so that the specimen-picking rod 2, together with the cover member 3, cannot be rotated. As a result, the T-shaped portions 2-1b' and 2-2b' of the first and second members 2-1' and 2-2' are guided by the T-shaped groove 3h, and the T-shaped portions 2-1b' and 2-2b' counterclockwise turn around the center of the cover member 3 to move away form the center of the cover member 3, and the forked portion 2-1a' and 2-2a' is opened in accordance with the movement distances of the T-shaped portions 2-1b' and 2-2b'. As described above, when the container for specimen is in an air-tight state before picking a specimen, the forked portion 2-1a' and 2-2a' of the specimen-picking rod 2 is in an open state.

In the case of picking a specimen, the cover member 3 is rotated in the direction in which the cover member 3 is released from the threaded attachment of the cover member 3 to the intermediary unit (or, in the counterclockwise direction, that is to say, in the direction of the forked portion 2-1a' and 2-2a' closing), so that the thread 3a₁ which is formed in the internal peripheral portion 3a is released from the threaded engagement of the thread 3a₁ with the thread which is formed in the external peripheral portion of the other end of the connection portion in the intermediary unit which is omitted in Fig. 44. At this point, the T-shaped portions 2-1b' and 2-2b' of the first and second members 2-1' and 2-2' are located in a predetermined place in the vicinity of the center of the cover member 3 in the T-shaped groove 3h, and the forked portion 2-1a' and 2-2a' is in a close state.

In picking a specimen, a user rotates the cover member 3 in the direction of the forked portion 2-1a' and 2-2a' opening while holding the vicinity of the fulcrum in the specimen-picking rod 2. As a result, the T-shaped portions 2-1b' and 2-2b' of the first and second members 2-1' and 2-2' are guided by the T-shaped groove 3h to move away from the vicinity of the center of the cover member 3, and the forked portion 2-1a' and 2-2a' is opened in accordance with the movement distances of the T-shaped portions 2-1b' and 2-2b'. Next, the user holds the vicinity of the fulcrum in the specimen-picking rod 2 and approximates the specimen-picking rod 2 to a position at which a specimen can be pinched by the forked portion 2-1a' and 2-2a'. Next, the user rotates the cover member 3 in the direction of the forked portion 2-1a' and 2-2a' closing while holding the vicinity of the fulcrum in the specimen-picking rod 2. As a result, the T-shaped portions 2-1b' and 2-2b' of the first and second members 2-1' and 2-2' are guided by the T-shaped groove 3h to move towards the vicinity of the center of the cover member 3, the forked portion 2-1a' and 2-2a' is in a close state when the T-shaped portions 2-1b and 2-2b move to predetermined positions, and the specimen is held by the specimen-holding portion that is surrounded by the groove 2a₂' and the shield portion 2a₁', with the specimen fragmented.

After picking the specimen, the user pushes the specimen-picking rod 2 into the through hole of the intermediary unit which is omitted in Fig. 44 to insert the specimen-picking rod 2 into the through hole, with the forked portion 2-1a' and 2-2a' closed, so that the specimen-picking rod 2 is made to penetrate through the through hole. At this point, the penetration wall comes into close contact with the side surface of the specimen-picking rod 2 through the through hole with the penetration wall elastically deforming, and the penetration wall separates a space into two divisions on the upper and lower sides.

Next, the thread 3a₁ which is formed in the internal peripheral portion 3a of the cover member 3 is threadedly engaged with the thread which is formed in the external peripheral portion of the other end of the connection portion in the intermediary unit which is omitted in Fig. 44, and the user rotates the cover member 3 in the direction in which the cover member 3 is threadedly attached to the intermediary unit 4 (or, in the clockwise direction, that is to say, in the direction of the forked portion 2-1a' and 2-2a' opening). As a result, the cover member 3 is threadedly attached to the intermediary unit which is omitted in Fig. 44, and the picked specimen is held by the container body which is omitted in Fig. 44, with the container body air-tight. At this point, the vicinity of fulcrum of the specimen-picking rod 2 is squeezed by the penetration wall of the intermediary unit which is omitted in Fig. 44, so that the specimen-picking rod 2, together with the cover member 3, cannot be rotated. As a result, the T-shaped portions 2-1b' and 2-2b' of the first and second members 2-1' and 2-2' are guided by the T-shaped groove 3h to move away from the vicinity of the center of the cover member 3, the forked portion 2-1a' and 2-2a' is opened in accordance with the movement distances of the T-shaped portions 2-1b' and 2-2b', and the picked specimen is dispersed in liquid in the container body 1.

According to the container for specimen of the seventeenth embodiment, the rotation of the cover member 3 makes it possible to adjust the forked portion 2-1a' and 2-2a' to a desired state of the forked portion 2-1a' and 2-2a' opening and closing in accordance with the size of a specimen to be picked, like the containers for specimen of the fifteenth and sixteenth embodiments. The other operation effects of the container for specimen of the seventeenth embodiment are approximately the same as those of the containers for specimen of the eleventh to thirteenth embodiments. Besides, in the example shown in Fig. 44, the front end of the forked portion 2-1a' and 2-2a' of the specimen picking rod 2 is formed in the same manner as that of the thirteenth embodiment. However, the front end of the forked portion 2-1a' and 2-2a' of the specimen picking rod 2 may be formed in the same manner as that of the eleventh or twelfth embodiment.

### Eighteenth Embodiment

Fig. 45 is a view schematically showing the constitution of a container for specimen according to the eighteenth embodiment of the present invention with the container for specimen divided into its constitution members, Fig. 46 is an explanatory view showing the constitutions of the cover member and specimen-picking rod in the container for specimen shown in Fig. 45, and Fig. 47 is view showing the constitution of the intermediary unit in the container for specimen shown in Fig. 45. Besides, the components of the containers for specimen of the eighteenth embodiment which are the same as those of the containers for specimen of the first and eleventh embodiments are given the same reference numerals, and the detailed explanations for the components are omitted.

In the container for specimen of the eighteenth embodiment, as shown in Figs. 45 and 46, the specimen-picking rod 2 includes a concave picking portion 2a which is placed in the front end of the specimen-picking rod 2. The concave picking portion 2a is formed in such a way that the diameter of the concave picking portion 2a becomes smaller and smaller towards the bottom plane of the concave picking portion 2a. In addition, the concave picking portion 2a includes a plurality of slits 2a₁ which connect the side surface of the concave picking portion 2a to the external peripheral surface of the specimen-picking rod 2.

Also, as shown in Figs. 45 and 47, the intermediary unit 4 includes a convex removing portion 4c which is placed on the bottom of the intermediary unit 4. The convex removing portion 4c is formed in such a way that the diameter of the convex removing portion 4c becomes smaller and smaller towards the top plane of the convex removing portion 4c. In addition, a plurality of knurlings 4c₁ are provided on the surface of the convex removing portion 4c.
And, the concave picking portion 2c and the convex removing portion 4c are formed in such a way that: the concave picking portion 2c and the convex removing portion 4c can be fitted to each other and be separated from each other from a state in which the convex removing portion 4c is fitted to the concave picking portion 2c; and as the convex removing portion 4c is fitted into the concave picking portion 2c, a specimen is pushed out to the outside through the slits 2a₁.

Also, the convex removing portion 4c is connected with the connection portion 4a of the intermediary unit 4 through a connection means 4d which is shown in Fig. 47 (b). The connection means 4d is composed of: an annular portion 4d₁ the diameter of which is the same as that of the connection portion 4a; and a removing portion-holding portion 4d₂ which is provided inside the annular portion 4d₁. The annular portion 4d₁ is connected with the lower end of the connection portion 4a. The beam portion 4d₂ consists of plate-shaped members that cross each other in the middle of the annular portion 4d₁ and in the shape of a cross, and the lower end of the convex removing portion 4c is fixed to the part of the beam portion 4d₂ in which the plate-shaped members cross each other. And, when the intermediary unit 4 is removed from the container body 1, the convex removing portion 4c can be also separated from the container body 1 with the convex removing portion 4c kept fitted into the concave picking portion 2a of the specimen-picking rod 2, at the same time. The other constitutions of the container for specimen of the eighteenth embodiment are approximately the same as those of the container for specimen of the eleventh embodiment.

In the container for specimen of the eighteenth embodiment which is formed in such a manner, a user sticks the front end of the specimen-picking rod 2 into a specimen in picking the specimen. As a result, the concave picking portion 2a enters the specimen. At this point, a surplus part of the specimen spills from a plurality of the slits 2a₁ which connect the side surface of the concave picking portion 2a to the external peripheral surface of the specimen-picking rod 2.

After picking the specimen, the user pushes the specimen-picking rod 2 into the through hole 4b₁ of the intermediary unit 4 and inserts the specimen-picking rod 2 into the through hole 4b₁ to make the specimen-picking rod 2 penetrate through the through hole 4b₁. At this point, the penetration wall comes into close contact with the side surface of the specimen-picking rod 2 through the through hole with the penetration wall elastically deforming, and the penetration wall separates a space into two divisions on the upper and lower sides. At this point, a surplus part of the picked specimen which exceeds a desired amount of the specimen picked by the specimen-picking rod 2 spills from the slits 2a₁ and adheres to the circumference.

However, in the container for specimen of the eighteenth embodiment, when the specimen-picking rod 2 passes through the through hole 4b₁, the surplus part of the specimen which has spilled from the slits 2a₁ and adhered to the circumference is scrubbed away to be removed to the surface of the upper side of the penetration wall 4b. As a result, only part of the quantified specimen is contained in preservation liquid in the container body 1.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the user rotates the cover member 3 in the direction in which the cover member 3 is threadedly attached to the intermediary unit 4. As a result, the cover member 3 is threadedly attached to the intermediary unit 4, and the picked specimen is held by the container body 1 with the container body 1 air-tight. At this point, the concave picking portion 2a of the specimen-picking rod 2 is fitted to the convex removing portion 4c which is provided on the bottom of the intermediary unit 4. And, the specimen which is picked by the picking portion 2a is pushed out by the convex removing portion 4c to pass through the slits 2a₁ to the outside. As a result, the picked specimen is fragmented to become easy to disperse in the preservation liquid.

In this case, the knurlings 4c₁ are further formed on the surface of the convex removing portion 4c in the container for specimen of the eighteenth embodiment. As a result, even in the case where a specimen having high viscosity is picked, the specimen can be smoothly and efficiently pushed out to pass through the slits 2a₁ to the outside.

In the case of analyzing the specimen which is contained in the preservation liquid in the container for specimen using a general-purpose apparatus for physical and chemical analysis, the intermediary unit 4 is rotated relative to the container body 1 in the direction in which the intermediary unit 4 is released from the threaded attachment of the intermediary unit 4 to the container body 1. As a result, the intermediary unit 4 moves in the direction in which the intermediary unit 4, together with the cover member 3 connecting with the specimen-picking rod 2 and the concave removing portion 4c connecting with the intermediary unit 4 through the connection means 4d, is removed from the external peripheral portion 1a of the open end of the container body 1, through the thread 1a₁ which is formed in the internal peripheral portion 1a of the open end of the container body 1 and the thread 4a₁₁ which is formed in the external peripheral portion 4a₁ of the one end of the connection portion 4a in the intermediary unit 4. As a result, only the container body 1 which holds the specimen that is dispersed in the preservation liquid is removed from the container for specimen. Next, the removed container body 1 is mounted in an apparatus for physical and chemical analysis (which is not shown in the drawings). Accordingly, the cover member 3 and the intermediary unit 4 that is connected with the convex removing portion 4c can be easily removed from the container body 1 together, without deteriorating the quantitative capability by mixing the surplus part of the specimen which is scrubbed away by the intermediary unit 4.

### Nineteenth Embodiment

Fig. 48 is a view schematically showing the constitution of a container for specimen according to the nineteenth embodiment of the present invention with the container for specimen divided into its constitution members, Fig. 49 is an explanatory view showing the constitutions of the cover member and the specimen-picking rod in the container for specimen shown in Fig. 48, and Fig. 50 is a view showing the constitution of the intermediary unit in the container for specimen shown in Fig. 48. Besides, the components of the containers for specimen of the nineteenth embodiment which are the same as those of the containers for specimen of the first and eleventh embodiments are given the same reference numerals, and the detailed explanations for the components are omitted.

In the container for specimen of the nineteenth embodiment, as shown in Figs. 48 and 49, the specimen-picking rod 2 includes a specimen-picking groove 2e which is placed on the surface of the front end of the specimen-picking rod 2 and has a thread structure. Also, as shown in Figs. 48 and 50, the intermediary unit 4 includes a cylinder-shaped specimen-pushing portion 4e which is placed on the bottom of the intermediary unit 4. The cylinder-shaped specimen-pushing portion 4e includes a specimen-pushing groove 4e₁ which is placed on the internal peripheral surface of the cylinder-shaped specimen-pushing portion 4e and has a thread structure that enables specimen-pushing groove 4e₁ to be threadedly fitted to the specimen-picking groove 2e. And, the specimen-picking groove 2e and the specimen-pushing groove 4e₁ are formed in such a way that: the specimen-picking groove 2e and the specimen-pushing groove 4e₁ can be threadedly fitted to each other and be separated from each other from a state in which the specimen-picking groove 2e is threadedly fitted to the specimen-pushing groove 4e₁; and as the specimen-picking groove 2e is threadedly fitted to the specimen-pushing groove 4e₁ more, a specimen is pushed out to the outside more.

Also, the cylinder-shaped specimen-pushing portion 4e is connected with the connection portion 4a of the intermediary unit 4 through a connection means 4d' which is shown in Fig. 50 (b). The connection means 4d' is composed of: an annular portion 4d₁' the diameter of which is the same as that of the connection portion 4a; and a beam portion 4d₂' which is provided inside the annular portion 4d₁'. The annular portion 4d₁' is connected with the lower end of the connection portion 4a. The beam portion 4d₂' consists of plate-shaped members that cross each other in the middle of the annular portion 4d₁' and in the shape of a cross, and the lower end of the cylinder-shaped specimen-pushing portion 4e is fixed to the part of the beam portion 4d₂' in which the plate-shaped members cross each other. And, when the intermediary unit 4 is removed from the container body 1, the cylinder-shaped specimen-pushing portion 4e can be also separated from the container body 1 with the cylinder-shaped specimen-pushing portion 4e kept fitted into the specimen-picking groove 2e of the specimen-picking rod 2, at the same time. The other constitutions of the container for specimen of the nineteenth embodiment are approximately the same as those of the container for specimen of the eighteenth embodiment.

In the container for specimen of the nineteenth embodiment which is formed in such a manner, a user rubs the front end of the specimen-picking rod 2 on a specimen in picking the specimen. As a result, the specimen enters the specimen-picking groove 2e. At this point, a surplus part of the specimen spills from the specimen-picking groove 2e.

After picking the specimen, the user pushes the specimen-picking rod 2 into the through hole 4b₁ of the intermediary unit 4 and inserts the specimen-picking rod 2 into the through hole 4b₁ to make the specimen-picking rod 2 penetrate through the through hole 4b₁. At this point, the penetration wall comes into close contact with the side surface of the specimen-picking rod 2 through the through hole with the penetration wall elastically deforming, and the penetration wall separates a space into two divisions on the upper and lower sides.

At this point, a surplus part of the picked specimen which exceeds a desired amount of the specimen picked by the specimen-picking rod 2 spills from the specimen-picking groove 2e and adheres to the circumference. However, in the container for specimen of the nineteenth embodiment, when the specimen-picking rod 2 passes through the through hole 4b₁, the surplus part of the specimen which has spilled from the specimen-picking groove 2e and adhered to the circumference is scrubbed away to be removed to the surface of the upper side of the penetration wall 4b. As a result, only part of the quantified specimen is contained in preservation liquid in the container body 1.

Next, the thread 3a₁ which is formed in the internal peripheral portion 3a of the cover member 3 is threadedly engaged with the thread 4a₂₁ which is formed in the external peripheral portion 4a₂ of the other end of the connection portion 4a in the intermediary unit 4, and the user rotates the cover member 3 in the direction in which the cover member 3 is threadedly attached to the intermediary unit 4. As a result, the cover member 3 is threadedly attached to the intermediary unit 4, and the picked specimen is held by the container body 1 with the container body 1 air-tight. At this point, the specimen-picking groove 2e of the specimen-picking rod 2 is fitted to the cylinder-shaped specimen-pushing portion 4e which is provided on the bottom of the intermediary unit 4. And, the specimen which is picked by the specimen-picking groove 2e is pushed out by the specimen-pushing portion 4e to the outside. As a result, the picked specimen is fragmented to become easy to disperse in the preservation liquid.

In the case of analyzing the specimen which is contained in the preservation liquid in the container for specimen using a general-purpose apparatus for physical and chemical analysis, the intermediary unit 4 is rotated relative to the container body 1 in the direction in which the intermediary unit 4 is released from the threaded attachment of the intermediary unit 4 to the container body 1. As a result, the intermediary unit 4 moves in the direction in which the intermediary unit 4, together with the cover member 3 connecting with the specimen-picking rod 2 and the cylinder-shaped specimen-pushing portion 4e connecting with the intermediary unit 4 through the connection means 4d', is removed from the external peripheral portion 1a of the open end of the container body 1, through the thread 1a₁ which is formed in the internal peripheral portion 1a of the open end of the container body 1 and the thread 4a₁₁ which is formed in the external peripheral portion 4a₁ of the one end of the connection portion 4a in the intermediary unit 4. As a result, only the container body 1 which holds the specimen that is dispersed in the preservation liquid is removed from the container for specimen. Next, the removed container body 1 is mounted in an apparatus for physical and chemical analysis (which is not shown in the drawings). Accordingly, the cover member 3 and the intermediary unit 4 that is connected with the cylinder-shaped specimen-pushing portion 4e can be easily removed from the container body 1 together, without deteriorating the quantitative capability by mixing the surplus part of the specimen which is scrubbed away by the intermediary unit 4.

### Twentieth Embodiment

Fig. 51 is an explanatory view showing the constitutions of the cover member and specimen-picking rod in a container for specimen according to the twentieth embodiment of the present invention, and Fig. 52 is an explanatory view showing the constitution of a container for specimen. Besides, the components of the container for specimen of the twentieth embodiment which have the same fundamental constitutions as those of the container for specimen of the eleventh embodiments are given the same reference numerals, and the detailed explanations for the components are omitted.

In the container for specimen of the twentieth embodiment, as shown in Fig. 51, the specimen-picking rod 2 includes a cylinder portion 2h and a piston portion 2i which can be slidably inserted into the cylinder portion 2h. The cylinder portion 2h is connected with the middle portion of the cover member 3. The piston portion 2i is composed of a front end portion 2i₁, a support portion 2i₂, and a rear end portion 2i₃. The front end portion 2i₁ is composed of a plurality of vertical divisions 2i₁ₐn (where n is an integer which is two or more) and a base portion 2i_{1b}. As shown in Fig. 51 (a) and 51 (e), a plurality of the vertical divisions 2i₁ₐn are formed so as to expand towards the outside more and more towards the front end with the vertical divisions 2i₁ₐn curving when the vertical divisions 2i₁ₐn protrude from the cylinder portion 2h towards the outside. Also, each of a plurality of the vertical divisions 2i₁ₐn is formed in such a way that the front end 2i₁ₐn' of each of a plurality of the vertical divisions 2i₁ₐn bends inward and the front ends 2i₁ₐn' of the vertical divisions 2i₁ₐn can hold a set amount of a specimen in a state of the vertical divisions 2i₁ₐn closing, as shown in Fig. 51 (b).

The support portion 2i₂ is formed in the shape of a rod having a small diameter and connects the front end portion 2i₁ with the rear end portion 2i₃. The base portion 2i_{1b} of the front end portion 2i₁ and the rear end portion 2i₃ have outer diameters which are approximately as large as the inner diameter of cylinder portion 2h, respectively. As a result, as shown in Fig. 51 (c) and 51 (d), when a plurality of the vertical divisions 2i₁ₐn are inside the cylinder portion 2h, a plurality of the vertical divisions 2i₁ₐn can slide in the cylinder portion 2h with an air-tight state kept.

A reagent-holding portion is formed by the space which is surrounded by the cylinder portion 2h, the rear end portion 2i₃ of the piston portion 2i, the support portion 2i₂ of the piston portion 2i, and (the base portion 2i_{1b} of) the front end portion 2i₁ of the piston portion 2i, and the reagent-holding portion holds a reagent for giving a specimen a predetermined treatment. And, the base portion 2i_{1b} of the front end portion 2i₁ functions as a stopper for the reagent-holding portion with respect to the cylinder portion 2h.

The rear end portion 2i₃ of the piston portion 2i is provided with a stopper portion 2i₃ₐ which is placed on the external peripheral surface of the rear end portion 2i₃. The stopper portion 2i₃ₐ is formed in such a shape that: when the stopper portion 2i₃ₐ meets the cover member 3, the stopper portion 2i₃ₐ restrains the movement of the piston portion 2i towards the front end in the cylinder portion 2h in order to keep the air-tightness of the reagent-holding portion; and the restraint of the movement of the piston portion 2i can be released by applying a predetermined amount or more of pressing force to the stopper portion towards the front end (where, in the example shown in Figs. 51 and 52, the stopper portion 2i₃ₐ is formed as a projection the sides of which have the shape of a right triangle). Also, the tail of the rear end portion 2i₃ of the piston portion 2i is provided with a head portion 2i_{3b} which is formed in the shape of a thin plate the diameter of which is larger than that of a below-described hole 3k of the cover member 3.

The cover member 3 includes a plate-shaped portion 3i, a stopper 3j, and the hole 3k into which the piston portion 2i is fitted. As shown in Fig. 52 (a), the plate-shaped portion 3i has a diameter that enables the plate-shaped portion 3i to cover the opening of the other end of the connection portion 4a of the intermediary unit 4. The stopper 3j has a diameter that enables the stopper 3j to be fitted into the inside of the other end of the connection portion 4a of the intermediary unit 4. In addition, the stopper 3j includes an annular projection 3j₁. The hole 3k is formed in the middle of the cover member 3 and the diameter of the hole 3k is approximately as large as the inner diameter of the cylinder portion 2h of the specimen-picking rod 2.

Also, in the container for specimen of the twentieth embodiment, an annular groove 4a₃₁ is formed in the internal periphery portion 4a₃ of the other end of the connection portion 4a of the intermediary unit 4. The annular groove 4a₃₁ is formed in such a way that the annular projection 3j₁ can be fitted into the annular groove 4a₃₁ when the stopper 3j is fitted into the inside of the other end of the connection portion 4a of the intermediary unit 4.

Also, in the container for specimen of the twentieth embodiment, as shown in Fig. 52 (b), the container for specimen is provided with a removable outer cover 7 which is placed on the outside on the cover-member-3 side of the intermediary unit 4 with the intermediary unit 4 connecting the container body 1 with the cover member 3. A first thread portion 4a₂₁ is formed on the external peripheral portion 4a₂ on the cover-member-3 side (or, the the-other-end side) of the connection portion 4a of the intermediary unit 4 and can be threadedly engaged with the outer cover 7. A second thread portion 7a₁ is formed on the internal peripheral portion 7a of the outer cover 7 and can be threadedly engaged with the first thread portion 4a₂₁ of the intermediary unit 4. In addition, a restraint-releasing portion 7b is provided in the middle of the outer cover 7. As shown in Fig. 52 (c), the restraint-releasing portion 7b is formed in the shape of a tube the diameter of which is smaller that that of the head portion 2i_{3b} of the rear end portion 2i₃ of the piston portion 2i.

And, when the second thread portion 7a₁ of the outer cover 7 is threadedly engaged with the first thread portion 4a₂ of the intermediary unit 4 and then the front end of the restraint-releasing portion 7b meets the head portion 2i_{3b} of the rear end portion 2i₃ of the piston portion 2i, a predetermined amount or more of pressing force is applied to the stopper portion 2i₃ₐ, so that the restraint-releasing portion 7b releases restraint of the movement of the piston portion 2i towards the front end in the cylinder portion 2h by the stopper portion 2i₃ₐ. The other fundamental constitutions of the intermediary unit 4 and the container body 1 in the container for specimen of the twentieth embodiment are approximately the same as those of the intermediary unit and the container body in the container for specimen of the eleventh embodiment even though the intermediary unit 4 and the container body 1 of the container for specimen of the twentieth embodiment are different from those of the container for specimen of the eleventh embodiment in shape.

In the container for specimen of the twentieth embodiment which is formed in such a manner, as shown in Fig. 51 (c), the specimen-picking rod 2 is supplied to a user with a plurality of the vertical divisions 2i₁ₐn contained in the cylinder portion 2h.

In the case of picking a specimen, a user pushes the rear end portion 2i₃ of the piston portion 2i while holding the cover member 3. As a result, as shown in Fig. 51 (b), a plurality of the vertical divisions 2i₁ₐn protrude from the cylinder portion 2h towards the outside. The user pushes the rear end portion 2i₃ of the piston portion 2i yet more. As a result, as a plurality of the vertical divisions 2i₁ₐn protrude from the cylinder portion 2h towards the outside more, the vertical divisions 2i₁ₐn expand towards the outside more with the vertical divisions 2i₁ₐn curving. And, as shown in Fig. 51 (a), when the stopper portion 2i₃ₐ meets the plate-shaped portion 3i of the cover member 3, the stopper portion 2i₃ₐ restrains the movement of the piston portion 2i towards the front end. In this state, the base portion 2i_{1b} of the front end portion 2i₁ remains inside the cylinder portion 2h, so that the base portion 2i_{1b} of the front end portion 2i₁ functions as a stopper for the reagent-holding portion that is formed by the space which is surrounded by the cylinder portion 2h, the rear end portion 2i₃ of the piston portion 2i, the support portion 2i₂ of the piston portion 2i, and (the base portion 2i_{1b} of) the front end portion 2i₁ of the piston portion 2i. As a result, a reagent does not escape from the reagent-holding portion.

Next, the user holds the cover member 3 and approximates the specimen-picking rod 2 to a position at which a specimen can be pinched by a plurality of the vertical divisions 2i₁ₐn. Next, as the user holds the cover member 3 with one hand of the user, the user holds the rear end portion 2i₃ of the piston portion 2i with the other hand of the user to pull the rear end portion 2i₃ of the piston portion 2i. As a result, as shown in Fig. 51 (b), a plurality of the vertical divisions 2i₁ₐn enter the inside of the cylinder portion 2h with the vertical divisions 2i₁ₐn catching the specimen.

At this point, a plurality of the vertical divisions 2i₁ₐn are formed in such a way that the vertical divisions 2i₁ₐn expand towards the outside more and more towards the front end with the vertical divisions 2i₁ₐn curving, in the container for specimen of the twentieth embodiment. As a result, a specimen can be picked without pushing the specimen-picking rod against the specimen hard, so that it is possible to easily pick even a specimen which lies in an unstable location, like a stool which is excreted on a sheet floating on water in a bowl. In addition, it is possible to pick a specimen on the surface of a stool or the like which widely extends, so that a probability that cells on the surface of a specimen can be picked becomes high.

Also, the front ends 2i₁ₐn' of a plurality of the vertical divisions 2i₁ₐn bend inward. As a result, as shown in Fig. 51 (c), when the vertical divisions 2i₁ₐn are closed by holding the rear end portion 2i₃ of the piston portion 2i to pull the rear end portion 2i₃ of the piston portion 2i more, the inside of the vertical divisions 2i₁ₐn can be made to have a predetermined space, so that the amount of a picked specimen becomes easy to quantify into a predetermined amount. Also, even though a specimen is rather hard, the closing of a plurality of the vertical divisions 2i₁ₐn can make the inward-bending front ends 2i₁ₐn' bite into the specimen, so that the specimen can be pinched to be picked. Also, in the case where a specimen is rather hard and shaped like a small grain, the specimen can be pinched by the inward-bending front ends 2i₁ₐn' to be picked.

Also, in the specimen-picking rod 2 of the container for specimen of the twentieth embodiment, the specimen-picking rod 2 is formed in such a way that the piston portion 2i can slide with the air-tight state kept when a plurality of the vertical divisions 2i₁ₐn are in the cylinder portion 2h. Accordingly, the specimen-picking rod 2 can function as a syringe. That is to say, as shown in Fig. 51 (d), when the rear end portion 2i₃ of the piston portion 2i is pulled more so that a plurality of the vertical divisions 2i₁ₐn which are in a close state are located on the inside of the cylinder portion 2h beyond the end of the cylinder portion 2h, a specimen having a low viscosity can be sucked into the cylinder portion. As a result, it is possible to pick specimens which have various hardness and viscosity, such as liquid specimen together with hard and soft specimens.

After picking the specimen, the user pushes the front end of the cylinder 2h into the through hole 4b₁ of the intermediary unit 4 connecting with the container body 1 while holding the cover member 3, with a plurality of the vertical divisions 2i₁ₐn contained in the cylinder portion 2h as shown in Fig. 51 (c) and 51 (d), and the specimen-picking rod 2 is inserted into the through hole 4b₁ to be made to penetrate through the through hole 4b₁. At this point, the penetration wall 4b comes into close contact with the side surface of the specimen-picking rod 2 through the through hole 4b₁ with the penetration wall 4b elastically deforming, and the penetration wall 4b separates a space into two divisions on the upper and lower sides.

Next, the stopper 3j of the cover member 3 is fitted into the inside of the other end of the connection portion 4a of the intermediary unit 4, and the annular projection 3j₁ is fitted to the annular groove 4a₃. As a result, the cover member 3 is connected with the intermediary unit 4 to be fixed to the intermediary unit 4.

Next, as shown in Fig. 52 (a), the rear end portion 2i₃ of the piston portion 2i is pushed into the cylinder portion 2h until the stopper portion 2i₃) meets the plate-shaped portion 3i of the cover member 3. As a result, a plurality of the vertical divisions 2i₁ₐn protrude from the cylinder portion 2h to the outside to expand towards the outside with the vertical divisions 2i₁ₐn curving. Accordingly, the picked specimen can be removed to the container body 1.

Next, as shown in Fig. 52 (b), the second thread portion 7a₁ of the outer cover 7 is threadedly engaged with the first thread portion 4a₂₁ of the intermediary unit 4. And then, when the front end of the restraint-releasing portion 7b meets the head portion 2i_{3b} of the rear end portion 2i₃ of the piston portion 2i, a predetermined amount or more of pressing force is applied to the stopper portion 2i₃ₐ, so that the restraint-releasing portion 7b releases restraint of the movement of the piston portion 2i towards the front end in the cylinder portion 2h by the stopper portion 2i₃ₐ. And then, the rear end portion 2i₃ of the piston portion 2i is pushed into the cylinder portion 2h yet more, so that the base portion 2i_{1b} of the front end portion 2i₁ of the piston portion 2i comes out of the cylinder portion 2h, and a reagent escapes from the reagent-holding portion to enter the container body 1. As a result, the specimen mixes with the reagent in the container body 1 and is dispersed in preservation liquid of the container body 1. Also, the closing of the intermediary unit 4 by the outer cover 7 makes it possible to prevent a specimen and reagent from leaking to the outside when the specimen and reagent in the container body 1 are stirred or when the container for specimen is carried.

Besides, a first reagent may be contained in the container body 1 in advance, and a second reagent may be contained in the reagent-holding portion of the specimen-picking rod 2. As a result, when a specimen which is picked by the specimen-picking rod is removed to the container body, the specimen can be treated with the second reagent immediately after treating the specimen with the first reagent. As a result, the treatment of a specimen with reagent can be performed quickly and efficiently. Accordingly, tumor markers in a specimen, cancer cells dissociated in a specimen, and so on can be preserved with the tumor markers, the cancer cells, and so on in a stable state.

### Twenty-First Embodiment

Fig. 53 is an explanatory view showing the constitutions of the cover member and specimen-picking rod in a container for specimen according to the twenty-first embodiment of the present invention. Besides, the components of the container for specimen of the twenty-first embodiment which have the same constitutions as the components of the specimen-picking rod in the container for specimen of the twentieth embodiments are given the same reference numerals, and the detailed explanations for the components are omitted.

The container for specimen of the twenty-first embodiment is one example of variations of the container for specimen of the twentieth embodiment. As shown in Fig. 53 (a), in the specimen-picking rod 2, the support portion 2i₂' which connects the front end portion 2i₁ with the rear end portion 2i₃ in the piston portion 2i is formed as a hollow part, and the outer diameter of the support portion 2i₂' is as large as the outer diameters of the front end portion 2i₁ and the rear end portion 2i₃. And, the reagent-holding portion is formed by the space which is surrounded by the rear end portion 2i₃ of the piston portion 2i, the hollow support portion 2i₂' of the piston portion 2i, and the front end portion 2i₁ of the piston portion 2i. In addition, the hollow support portion 2i₂' is provided with an opening 2i₂ₐ' for exhausting a reagent. As shown in Fig. 53 (c), the opening 2i₂ₐ' is located inside the cylinder portion 2h when the stopper portion 2i₃ₐ restrains the movement of the piston portion 2i towards the front end in the cylinder portion 2h. Also, as shown in Fig. 53 (d), the opening 2i₂ₐ' is located outside the cylinder portion 2h when the rear end portion 2i₃ of the piston portion 2i is pushed into the cylinder portion 2h in a state in which the restraint of the movement of the piston portion 2i towards the front end in the cylinder portion 2h is released by the stopper portion 2i₃ₐ. The other constitutions of the container for specimen of the twenty-first embodiment are approximately the same as those of the container for specimen of the twentieth embodiment.

In the container for specimen of the twenty-first embodiment which is formed in such a manner, when the stopper portion 2i₃ₐ is released and the piston portion 2i is pushed into the cylinder portion 2h yet more, the opening 2i₂ₐ' moves out of the cylinder portion 2h to the outside as shown in Fig. 53(d), and a reagent escapes from the reagent-holding portion. Besides, the other operations for picking a specimen, removing the picked specimen to the inside of the container body, and making a reagent escape from the reagent-holding portion are approximately the same as those for the container for specimen of the twentieth embodiment.

According to the container for specimen of the twenty-first embodiment, an opening part in the reagent-holding portion can be restricted to the opening 2i₂ₐ' in a hollow 2i₂' of the hollow support portion, and, in addition, the reagent-holding portion can be composed of only the piston portion 2i without using the cylinder portion 2h. And, because the opening part of the reagent-holding portion is regular regardless of the size of the reagent-holding portion, it is hard for a reagent to leak from the reagent-holding portion. Accordingly, the space which forms the reagent-holding portion can be made to become wide on the rear-end-portion-2i₃ side, and the volume of a reagent can be increased. The other operation effects of the container for specimen of the twenty-first embodiment are approximately the same as those of the container for specimen of the twentieth embodiment.

The several embodiments for a container for specimen of the present invention were explained hereinbefore. However, a container for specimen of the present invention is not limited to the constitutions of the above-described embodiments. For example, in the above-described embodiments, the connection portion 4a of the intermediary unit 4, the cover member 3, and container body 1 may be made of elastic material in such a way that: the elastic modulus of the connection portion 4a of the intermediary unit 4 is smaller than those of the penetration wall 4b and the cover member 3 and is larger than that of the container body 1; and the elastic modulus of the cover member 3 is smaller than that of the penetration wall 4b. As a result, the cover member 3 is more flexible than the intermediary unit 4, so that the cover member 3 is easy to deform and to attach to or detach from the intermediary unit 4. Also, the intermediary unit 4 is more flexible than the container body 1, so that the intermediary unit 4 is easy to deform and to attach to or detach from the container body 1.

As a result, in picking a specimen, the cover member 3 which connects with the specimen-picking rod 2 becomes easy to detach from the intermediary unit 4 with the intermediary unit 4 kept fitted to the container body 1. Also, after picking the specimen, when the specimen-picking rod 2 is made to penetrate through the penetration wall 4b of the intermediary unit 4 to be inserted into the container body 1, the deformation of the intermediary unit 4 can be restrained to the utmost. In addition, also when the cover member 3 is fitted to the intermediary unit 4, the deformation of the intermediary unit 4 can be restrained. Furthermore, the intermediary unit 4 becomes easy to detach from the container body 1.

Or, the connection portion 4a of the intermediary unit 4, the cover member 3, and container body 1 may be made of elastic material in such a way that the elastic modulus of the connection portion 4a of the intermediary unit 4 is smaller than that of the penetration wall 4b and is larger than those of the cover member 3 and the container body 1. Also in this case, the cover member 3 which connects with the specimen-picking rod 2 becomes easy to detach from the intermediary unit 4 with the intermediary unit 4 kept fitted to the container body 1. Also, after picking the specimen, when the specimen-picking rod 2 is made to penetrate through the penetration wall 4b of the intermediary unit 4 to be inserted into the container body 1, the deformation of the intermediary unit 4 can be restrained to the utmost. In addition, when the cover member 3 is fitted to the intermediary unit 4, the deformation of the cover member 3 can be restrained. Furthermore, the intermediary unit 4 becomes easy to detach from the container body 1.

In addition, as described in the first embodiment, in a container for specimen in which the cover member 3 and the intermediary unit 4 can be removably fitted to each other through threads, the container for specimen may be formed in such a way that torque by which the other end of the connection portion 4a of the intermediary unit 4 is screwed into the cover member 3 is smaller than torque by which the open end of the container body 1 is screwed into the one end of the connection portion 4a of the intermediary unit 4. As a result, when the cover member 3 connecting with the specimen-picking rod 2 is removed from the intermediary unit 4 in order to pick a specimen, it is possible to prevent the intermediary unit 4 from coming out of the container body 1.

In addition, the open and close directions in rotating the intermediary unit 4 in a predetermined direction through the threads which are formed in the internal peripheral portion of the one end of the connection portion 4a of the intermediary unit 4 and in the external peripheral portion of the open end of the container body 1 may be oppose to the open and close directions in rotating the intermediary unit 4 in a predetermined direction through the threads which are formed in the external peripheral portion of the other end of the connection portion 4a of the intermediary unit 4 and in the internal peripheral portion of the cover member 3, respectively. As a result, for example, the rotation direction in unfastening the cover member 3 from the intermediary unit 4 for the purpose of picking a specimen corresponds to the rotation direction in fastening the intermediary unit 4 to the container body 1. Also, the rotation direction in fastening the cover member 3 to the intermediary unit 4 after picking the specimen corresponds to the rotation direction in unfastening the intermediary unit 4 from the container body 1.
Accordingly, by merely changing the rotation direction of the cover member 3, the cover member 3 can be removed from the intermediary unit 4 with the intermediary unit 4 kept fitted to the container body 1 when a specimen is picked, while the intermediary unit 4 can be removed from the container body 1 with the cover member 3 kept fitted to the intermediary unit 4 when the test of the picked specimen is performed using a general-purpose test apparatus after removing the picked specimen to the container body 1.

Furthermore, it is more preferred that the cover member 3 includes a rectangle-shaped holding portion (which is not shown in the drawings). Even in such a manner, the rectangle-shaped holding portion makes it possible to easily rotate the cover member 3, so that the cover member 3 becomes easy to fasten to and unfasten from the intermediary unit 4. In addition, as described above, in the case where the open and close directions in rotating the intermediary unit 4 in a predetermined direction through the threads which are formed in the internal peripheral portion of the one end of the connection portion 4a of the intermediary unit 4 and in the external peripheral portion of the open end of the container body 1 are oppose to the open and close directions in rotating the intermediary unit 4 in a predetermined direction through the threads which are formed in the external peripheral portion of the other end of the connection portion 4a of the intermediary unit 4 and in the internal peripheral portion of the cover member 3, respectively, the rectangle-shaped holding portion makes it easy to perform: the operation of removing the cover member 3 from the intermediary unit 4 with the intermediary unit 4 kept fitted to the container body 1 in picking a specimen; and the operation of removing the intermediary unit 4 from the container body 1 with the cover member 3 kept fitted to the intermediary unit 4 in performing the test of the picked specimen using a general-purpose test apparatus after removing the picked specimen to the container body 1.

The several embodiments for a container for specimen of the present invention were explained hereinbefore. However, a container for specimen of the present invention is not limited to the constitutions of the above-described embodiments and can have various variations. For example, in the above-described embodiments, the connection portion 4a of the intermediary unit 4, the cover member 3, and container body 1 may be made of elastic material in such a way that: the elastic modulus of the connection portion 4a of the intermediary unit 4 is smaller than those of the penetration wall 4b and the cover member 3 and is larger than that of the container body 1; and the elastic modulus of the cover member 3 is smaller than that of the penetration wall 4b. As a result, the cover member 3 is more flexible than the intermediary unit 4, so that the cover member 3 is easy to deform and to attach to or detach from the intermediary unit 4. Also, the intermediary unit 4 is more flexible than the container body 1, so that the intermediary unit 4 is easy to deform and to attach to or detach from the container body 1. Accordingly, the cover member 3 which connects with the specimen-picking rod 2 becomes easy to unfasten from the intermediary unit 4 with the intermediary unit 4 kept fitted to the container body 1 when a specimen is picked. Also, when the specimen-picking rod 2 is made to penetrate through the penetration wall 4b of the intermediary unit 4 to be inserted into the container body 1 after picking the specimen, the deformation of the intermediary unit 4 can be restrained to the utmost. In addition, also when the cover member 3 is fastened to the intermediary unit 4, the deformation of the intermediary unit 4 can be restrained. Furthermore, the intermediary unit 4 becomes easy to unfasten from the container body 1.

Or, the connection portion 4a of the intermediary unit 4, the cover member 3, and container body 1 may be made of elastic material in such a way that the elastic modulus of the connection portion 4a of the intermediary unit 4 is smaller than that of the penetration wall 4b and is larger than those of the cover member 3 and the container body 1. Also in this case, the cover member 3 which connects with the specimen-picking rod 2 becomes easy to detach from the intermediary unit 4 with the intermediary unit 4 kept fitted to the container body 1. Also, after picking the specimen, when the specimen-picking rod 2 is made to penetrate through the penetration wall 4b of the intermediary unit 4 to be inserted into the container body 1, the deformation of the intermediary unit 4 can be restrained to the utmost. In addition, when the cover member 3 is fitted to the intermediary unit 4, the deformation of the cover member 3 can be restrained. Furthermore, the intermediary unit 4 becomes easy to detach from the container body 1.

In addition, as described in the first and eleventh embodiments, in a container for specimen in which the cover member 3 and the intermediary unit 4 can be removably fitted to each other through threads, the container for specimen may be formed in such a way that torque by which the other end of the connection portion 4a of the intermediary unit 4 is screwed into the cover member 3 is smaller than torque by which the open end of the container body 1 is screwed into the one end of the connection portion 4a of the intermediary unit 4. As a result, when the cover member 3 connecting with the specimen-picking rod 2 is unfastend from the intermediary unit 4 in order to pick a specimen, it is possible to prevent the intermediary unit 4 from coming out of the container body 1.

In addition, the specimen-picking rod 2 may be formed integratedly with the cover member 3. Or, the cover member 3 may be formed in such a way that the cover member 3 is provided with the fitting groove 3b as in the tenth embodiment which is shown in Fig. 16 and is furthermore provided with a thread, a concave-convex portion, or the like for example so that the specimen-picking rod 2 can be removably fitted to the cover member 3. Such a constitution of specimen-picking rod 2 capable of being removably fitted to the cover member 3 makes it possible to fit to the cover member 3 specimen-picking rods that are made in a suitable form for picking a specimen in accordance with the shape, hardness, and viscosity of the specimen. As a result, the collection of a specimen can be optimized.

In addition, the open and close directions in rotating the intermediary unit 4 in a predetermined direction through the threads which are formed in the internal peripheral portion of the one end of the connection portion 4a of the intermediary unit 4 and in the external peripheral portion of the open end of the container body 1 may be oppose to the open and close directions in rotating the intermediary unit 4 in a predetermined direction through the threads which are formed in the external peripheral portion of the other end of the connection portion 4a of the intermediary unit 4 and in the internal peripheral portion of the cover member 3, respectively. As a result, for example, the rotation direction in unfastening the cover member 3 from the intermediary unit 4 for the purpose of picking a specimen corresponds to the rotation direction in fastening the intermediary unit 4 to the container body 1. Also, the rotation direction in fastening the cover member 3 to the intermediary unit 4 after picking the specimen corresponds to the rotation direction in unfastening the intermediary unit 4 from the container body 1.
Accordingly, by merely changing the rotation direction of the cover member 3, the cover member 3 can be removed from the intermediary unit 4 with the intermediary unit 4 kept fitted to the container body 1 when a specimen is picked, while the intermediary unit 4 can be removed from the container body 1 with the cover member 3 kept fitted to the intermediary unit 4 when the test of the picked specimen is performed using a general-purpose test apparatus after removing the picked specimen to the container body 1.

Furthermore, it is more preferred that the cover member 3 includes a rectangle-shaped holding portion (which is not shown in the drawings). Even in this case, the rectangle-shaped holding portion makes it possible to easily rotate the cover member 3, so that the cover member 3 becomes easy to fasten to and unfasten from the intermediary unit 4. In addition, as described above, in the case where the open and close directions in rotating the intermediary unit 4 in a predetermined direction through the threads which are formed in the internal peripheral portion of the one end of the connection portion 4a of the intermediary unit 4 and in the external peripheral portion of the open end of the container body 1 are oppose to the open and close directions in rotating the intermediary unit 4 in a predetermined direction through the threads which are formed in the external peripheral portion of the other end of the connection portion 4a of the intermediary unit 4 and in the internal peripheral portion of the cover member 3, respectively, the rectangle-shaped holding portion makes it easy to perform: the operation of removing the cover member 3 from the intermediary unit 4 with the intermediary unit 4 kept fitted to the container body 1 in picking a specimen; and the operation of removing the intermediary unit 4 from the container body 1 with the cover member 3 kept fitted to the intermediary unit 4 in performing the test of the picked specimen using a general-purpose test apparatus after removing the picked specimen to the container body 1.

With respect to another matters, materials used for the members that form the container for specimen of each of the embodiments of the present invention may be as follows. It is preferred that a synthetic resin material which is used for the cover member 3 has a good tribological property for detaching the cover member 3 in order to improve easy attachment and detachment of the cover member 3 and air-tightness. For example, it is more preferred that a synthetic resin material for the cover member 3 is a fluorinated resin or the like. Also, it is more preferred that a material which is used for the cover member 3 is more flexible than a material which is used for the intermediary unit 4. Or, plastics the density and hardness of which are higher than those of a material for the intermediary unit 4 may be used for the cover member 3.

The container body 1 is required to have resistance to organic solvents such as alcohol as liquid and buffer solutions. Accordingly, it is more preferred that a material for the container body 1 is polyolefin resin which is typified by polypropylene, polyethylene, polymethylpentene, and so on. However, in the case where solutions to be contained in the container body 1 are limited to water-based solutions, synthetic resins which can be machined into a container, such as polystyrene resin, ABS resin, and acrylic resin may be used as a material for the container body 1

A shape and/or material of the specimen-picking rod 2 vary with specimens to be picked. In the case where a specimen to be picked is a stool, it is more preferred that materials such as polyethylene having a high density, polycarbonate, polypropylene, polyacetal, and so on are used for the specimen-picking rod 2. Also, the specimen-picking rod 2 may have the same material as that of the cover member 3.

It is preferred that materials for the connection portion 4a that forms the outer frame of the intermediary unit 4 include: polyolefin resin which is typified by polypropylene, polyethylene, and so on; and materials which can be used for the container body, such as butadiene-styrene resin. In addition, it is more preferred that the connection portion 4a of the intermediary unit 4 is made of material that is more flexible than that of the container body. It is preferred that the elasticity of materials for the penetration wall 4b of the intermediary unit 4 is higher than that of plastic because plastic is used as materials for the specimen-picking rod 2; and, for example, materials for the penetration wall 4b of the intermediary unit 4 include natural rubber, and synthetic rubbers such as isoprene rubber, styrene-butadiene rubber, butadiene rubber, chloroprene-acrylo rubber, nitrile butadiene rubber, ethylene-plopylene rubber, silicone, fluoro rubber, chlorosulfated polyethylene rubber, chlorinated polyethylene, and polysulfide rubber.

Because the above-described rubber materials have resistance to organic solvents such as alcohol, it is desired that one of the above-described rubber materials is used. In particular, it is more preferred that fluoro rubber or butyl rubber is used because fluoro rubber and butyl rubber have high resistance to main agents and are easy to give a machining process. Also, in the case where the penetration wall 4b of the intermediary unit 4 is provided with a groove so that the penetration wall 4b is made to have the shape of a fin to have close contact capability, the same materials as those of the connection portion 4a forming the outer frame of the intermediary unit 4 may be used for the penetration wall 4b instead of the above-described rubber materials. For example, when polymethylpentene is used as a material of the container body 1, it is preferred that polypropylene or the like is used as a material of the intermediary unit 4 and polyethylene or fluorine resin is used as a material of the cover member 3.

With respect to another matters, the shape of the bottom side of the container body 1 may be the shape of a round bottom or flat bottom. However, in the case where the container body 1 is mounted in a centrifuge, it is desired that the container body 1 has a round bottom. Also, a container for specimen may be formed so as to have any combination of the characteristic constitutions in the above-described embodiments.

Next, examples of a test in which a specimen is picked and/or preserved by a container for specimen of the present invention and then the container is mounted in a general-purpose apparatus for physical and chemical analysis to extract nucleic acids will be explained using the drawings.

### Test Example 1

Fig. 54 is an explanatory view showing a treatment process which includes collection and/or preservation of an artificial stool with a container for specimen of the present invention, the attachment of the container for specimen to a centrifuge, and extraction of nucleic acids using the centrifuge.

A container for specimen which is used in the test example 1 has approximately the same constitution as that of the container for specimen of the tenth embodiment which is shown in Fig. 16. Beside, the constitution of the specimen-picking rod 2 for the test example 1 is approximately the same as that of the specimen-picking rod in the container for specimen of the seventh embodiment which is shown in Fig. 11. That is to say, the specimen-picking rod 2 for the test example 1 includes a plurality of slits 2c which link the concave picking portion 2a and the external peripheral surface of the specimen-picking rod 2, and a picked specimen passes through a plurality of the slits 2c to be extruded to the outside when the convex removing portion 6 is inserted into the concave picking portion 2a.

The container for specimen which is formed in such a manner was used in the test example 1 and a process which included the picking of a specimen, the remove of the picked specimen to the container body, and the attachment of the container body to a nucleic acid-extracting apparatus was performed. Artificial stools were used as a specimen. The artificial stools were made in such a way that: MKN45 cells were immediately added to each of stools from eight healthy persons to be mixed in each of the stools; next, the mixed stools were weighed and stools of 2g or more were collected from the mixed stools, respectively, and the collected stools were shaped so as to finally have a weight of 2g; and the shaped stools were kept in a freezer. The MKN45 cells were prepared so that the number of the MKN45 cells that were contained in each of the 2g stools was 3 × 10⁶. MKN45 cells originate from gastric cancer. However, because MKN45 cells highly express COX2 genes like colorectal cancer cells, MKN45 cells were used as a model of colorectal cancer cells. The picking amount of the artificial stool was fixed at 0.5g. Also, a KUBOTA 5930 universal refrigerated centrifuge was used as a general-purpose nucleic acid-extracting apparatus for extracting nucleic acids.

First, from the initial state of the container for specimen which is shown in Fig. 16 (a), the cover member 3 was rotated in the open direction, so that the thread 3a₁ which is formed in the internal peripheral portion 3a was released from the threaded engagement of the thread 3a₁ with the thread 4a₂₁ which is formed in the external peripheral portion 4a₂ of the other end of the connection portion 4a in the intermediary unit 4. After cover member 3 was unfastened from the other end of the connection portion 4a of the intermediary unit 4, the cover member 3 was pulled in the direction in which the cover member 3 moved away from the intermediary unit 4, and then the specimen-picking rod 2 was pulled out of the through hole 4b₁ of the penetration wall 4b to be removed from the other end of the connection portion 4a (refer to Fig. 16 (b)). And, as shown in Fig. 54 (a), an artificial stool E was picked by the specimen-picking rod 2 in such a way that the artificial stool E was contained in the concave picking portion 2a that was provided in the front end of the specimen-picking rod 2.

Next, the front end of the specimen-picking rod 2 that holds the picked artificial stool E was pushed into the through hole 4b₁ of the intermediary unit 4 so that the specimen-picking rod 2 was made to penetrate through the through hole 4b₁. At this point, the penetration wall 4b came into close contacted with the side surface of the specimen-picking rod 2 through the through hole 4b₁ with the penetration wall 4b elastically deforming, and the penetration wall 4b separated a space into two divisions on the upper and lower sides. As a result, as shown in Fig. 54 (b), a surplus part of the picked artificial stool E which adhered to the side surface of the specimen-picking rod 2 was removed to the upper portion of the through hole 4b₁ of the penetration wall 4b.

Next, the thread 3a₁ which was formed in the internal peripheral portion 3a of the cover member 3 was threadedly engaged with the thread 4a₂₁ which was formed in the external peripheral portion 4a₂ of the other end of the connection portion 4a in the intermediary unit 4, and the cover member 3 was rotated in the close direction. As a result, as the concave picking portion 2a of the specimen-picking rod 2 was fitted to the convex removing portion 6 which was provided on the bottom of the container body 1, the picked artificial stool E which was held by the concave picking portion 2a was pushed out by the convex removing portion 6 to pass through a plurality of the slits 2c into the container body 1. In addition, as shown in Fig. 16 (a), the cover member 3 was threadedly fastened to the intermediary unit 4. As a result, the picked artificial stool E was contained in the container body 1 with the container body 1 air-tight. As shown in Fig. 54 (c), the artificial stool E which was pushed out into the container body 1 was dispersed in liquid 5 (which is a preservation liquid).

And then, the container body 1 which contained the artificial stool E in an air-tight state was mounted in the nucleic acid-extracting apparatus (which is the refrigerated centrifuge). At this point, the intermediary unit 4 was rotated relative to the container body 1 in the open direction, so that the thread 1a₁ which is formed in the external peripheral portion 1 a of the open end of the container body 1 was released from the threaded engagement of the thread 1a₁ with the thread 4a₁₁ which is formed in the external peripheral portion 4a₁ of the one end of the connection portion 4a in the intermediary unit 4. Next, as shown in Fig. 54 (d), the intermediary unit 4 was removed from the external peripheral portion 1a of the open end of the container body 1 with the intermediary unit 4 integrated with the cover member 3 that connected with the specimen-picking rod 2. In this case, the reason why the intermediary unit 4, together with the cover member 3, was removed from the container body 1 is as follows: If the intermediary unit 4 is kept fastened to the container body 1, the penetration wall 4a narrows the path to the container body 1 and, in addition, the intermediary unit 4 lengthens the distance from the mouth of the container to the container body 1, so that after centrifuging the solution that contains the artificial stool through the centrifuge, aspiration nozzles for removing supernates on the solution, such as pipette become hard to insert into the container body 1. After removing the intermediary unit 4 from the container body 1, the container body 1 was mounted in a bucket 8 of the refrigerated centrifuge as shown in Fig. 54 (e), and the container body 1 was made to move with centrifugal motion.

After centrifuging the solution that contained the artificial stool E through the refrigerated centrifuge in such a manner, supernates in the container body 1 were removed from the solution. And then, a nucleic acid extraction reagent (ISOGEN, TRIzol, or the like) was added to the solution, the nucleic acid extraction reagent was sufficiently stirred together with the specimen, and the solution was centrifuged through the refrigerated centrifuge again. After centrifuging the solution again, supernates in the container body 1 were removed to a new tube, chloroform was added to the supernates, and the supernates were further centrifuged through the refrigerated centrifuge. After centrifuging the supernates, the aqueous phase in the tube was removed to another tube, so that desired nucleic acids were extracted. Fig. 56 shows the results of the analysis of the nucleic acids by electrophoresis.

Electrophoresis was performed as follows: A part of the extracted nucleic acids was collected, and the collected part of the nucleic acids was made to dissolve in a lysis buffer in which formalin and formamide were mixed. Also, a 0.02M MOPS buffer solution containing 0.5µg / ml EtBr was used as a running buffer. The samples were set in a prepared agarose gel, and electrophoresis was performed with a constant voltage of 100 V for about 30 minutes. After performing electrophoresis, the nucleic acids were exposed to an UV lamp, and the nucleic acids that emitted light were photographed. As a result, as shown in Fig. 56, the bands of the nucleic acids which were peculiar to the artificial stool could be identified.

### Test Example 2

By performing the same procedure as that of the test example 1, the intermediary unit 4 was removed from the container body 1 which contained the artificial stool E in an air-tight state. And then, the container body 1 was mounted in a MagNALyser (registered trademark) which was made by The Roche Co. and was a general-purpose apparatus for physical and chemical analysis, and cells in the specimen were automatically homogenized and were centrifuged. After the centrifugation, supernates in the container body 1 were removed, a nucleic acid extraction reagent (ISOGEN, TRIzol, or the like) was added to the specimen, the nucleic acid extraction reagent was sufficiently stirred together with the specimen, and the specimen was centrifuged. After the centrifugation, supernates in the container body 1 were removed to a new tube, chloroform was added to the supernates, and the supernates were centrifuged. After the centrifugation, the aqueous phase in the tube was removed to another tube, so that desired nucleic acids were extracted.

A part of the extracted nucleic acids was collected, and the collected part of the nucleic acids was made to dissolve in a lysis buffer in which formalin and formamide were mixed. Also, a 0.02M MOPS buffer solution containing 0.5µg / ml EtBr was used as a running buffer. The samples were set in a prepared agarose gel, and electrophoresis was performed with a constant voltage of 100 V for about 30 minutes. After performing electrophoresis, the nucleic acids were exposed to an UV lamp, and the nucleic acids that emitted light were photographed. As a result, as shown in Fig. 57, the bands of the nucleic acids which were peculiar to the artificial stool were identified.

### Test Example 3

Fig. 55 is an explanatory view showing a treatment process which includes collection and/or preservation of an artificial stool with a container for specimen the constitution of which is approximately the same as that of the container for specimen of the eleventh embodiment that is shown in Figs. 18 to 22, the attachment of the container for specimen to a centrifuge, and extraction of nucleic acids using the centrifuge.

The container for specimen having the above-described constitution was used in this test example and a process which included the picking of a specimen, the remove of the picked specimen to the container body, and the attachment of the container body to a nucleic acid-extracting apparatus was performed. Artificial stools were used as a specimen. The artificial stools were made in such a way that: MKN45 cells were immediately added to each of stools from eight healthy persons to be mixed in each of the stools; next, the mixed stools were weighed and stools of 2g or more were collected form the mixed stools, and the collected stools were shaped so as to finally have a weight of 2g; and the shaped stools were kept in a freezer. The MKN45 cells were prepared so that the number of the MKN45 cells that were contained in each of the 2g stools was 3 × 10⁶. MKN45 cells originate from gastric cancer. However, because MKN45 cells highly express COX2 genes like colorectal cancer cells, MKN45 cells were used as a model of colorectal cancer cells. The picking amount of the artificial stool was fixed at 0.5g. Also, a KUBOTA 5930 universal refrigerated centrifuge was used as a general-purpose nucleic acid-extracting apparatus for extracting nucleic acids.

First, from the initial state of the container for specimen which is shown in Fig. 18 (a), the cover member 3 was rotated in the direction of the cover member 3 opening, so that the thread 3a₁ which was formed in the internal peripheral portion 3a was released from the threaded engagement of the thread 3a₁ with the thread 4a₂ which was formed in the external peripheral portion 4a₂ of the other end of the connection portion 4a in the intermediary unit 4. After cover member 3 was unfastened from the other end of the connection portion 4a of the intermediary unit 4, the cover member 3 was pulled in the direction in which the cover member 3 moved away from the intermediary unit 4, and then the specimen-picking rod 2 was pulled out of the through hole 4b₁ of the penetration wall 4b to be removed from the other end of the connection portion 4a. When the specimen-picking rod 2 passes through the through hole 4b₁, the forked portion 2a is subjected to inward force from the through hole 4b₁ to become closed from the open state. When the specimen-picking rod 2 was completely pulled out, the elasticity of the forked portion 2 made the forked portion 2a open again. At this point, the position of the annular member 7 was fixed through the projection 2d, so that the forked portion 2 was kept open.

Next, the cover member 3 which was in a state shown in Fig. 55 (a) was held and the specimen-picking rod 2 was approximated to a position at which the artificial stool E could be pinched by the forked portion 2c. Next, the cover member 3 was held by one hand of a user, and the annular member 7 was pinched by the other hand of the user to be moved towards the front end by a predetermined distance as shown in Fig. 55 (b), so that the forked portion 2c was closed and the artificial stool E was made to pass through the specimen-introducing inlet 2c₁₁ into the specimen-holding portion 2c₁ with the artificial stool E fragmented. In such a manner, the artificial stool E was collected. In this case, a surplus part of the artificial stool E exceeding a predetermined amount of the artificial stool E stuck out of the specimen-exhausting outlet 2c₁₂.

Next, as shown in Fig. 55 (c), the front end of the specimen-picking rod 2 which held the picked artificial stool E was pushed into the through hole 4b₁ of the intermediary unit 4 to be made to penetrate through the through hole 4b₁, with the forked portion 2c closed. At this point, the penetration wall 4b came into close contact with the side surface of the specimen-picking rod 2 through the through hole 4b₁ with the penetration wall 4b elastically deforming, and the penetration wall 4b separated a space into two divisions on the upper and lower sides. As a result, a surplus part of the artificial stool E which had adhered to the side surface of the specimen-picking rod 2 was removed to the upper portion of the through hole 4b₁ of the penetration wall 4b. Besides, at the point in time of the beginning of the insertion of the specimen-picking rod 2 into the through hole 4b₁, the annular member 7 gave the forked portion 2c inward force that restrains force making the forked portion 2c open, so that the forked portion 2c was kept close.

After pushing the specimen-picking rod 2 that had been inserted into the through hole 4b₁ until the annular member 7 met the surface on the upper side of the penetration wall 4b, as shown in Fig. 55 (d), the specimen-picking rod 2 was further successively inserted into the through hole 4b₁, so that the annular portion 7 was pushed up by the surface of the upper side in the penetration wall 4b. As a result, the elasticity of the forked portion 2c made the forked portion 2c open in preservation liquid in the container body 1. The artificial stool E which was contained by the specimen-holding portion 2c₁ was dispersed in the preservation liquid in the container body 1.

And then, the container body 1 which contained the artificial stool E in an air-tight state was mounted in the nucleic acid-extracting apparatus (which is the refrigerated centrifuge). At this point, the intermediary unit 4 was rotated relative to the container body 1 in the direction in which the intermediary unit 4 moved away from the container body 1, so that the thread 1a₁ which is formed in the external peripheral portion 1a of the open end of the container body 1 was released from the threaded engagement of the thread 1a₁, with the thread 4a₁₁ which is formed in the external peripheral portion 4a₁ of the one end of the connection portion 4a in the intermediary unit 4. Next, as shown in Fig. 55 (e), the intermediary unit 4 was removed from the external peripheral portion 1a of the open end of the container body 1 with the intermediary unit 4 integrated with the cover member 3 that connected with the specimen-picking rod 2.

In this case, the reason why the intermediary unit 4, together with the cover member 3, was removed from the container body 1 is as follows: If the intermediary unit 4 is kept fastened to the container body 1, the penetration wall 4b narrows the path to the container body 1 and, in addition, the intermediary unit 4 lengthens the distance from the mouth of the container to the container body 1, so that after centrifuging the solution that contains the artificial stool E through the centrifuge, aspiration nozzles for removing supernates on the solution, such as pipette become hard to insert into the container body 1. After removing the intermediary unit 4 from the container body 1, the container body 1 was mounted in a bucket 8 of the refrigerated centrifuge, as shown in Fig. 55 (f), and the solution was centrifuged.

After centrifuging the solution that contained the artificial stool E through the refrigerated centrifuge, supernates in the container body 1 were removed from the solution. And then, a nucleic acid extraction reagent (ISOGEN, TRIzol, or the like) was added to the solution, the nucleic acid extraction reagent was sufficiently stirred together with the specimen, and the solution was centrifuged through the refrigerated centrifuge again. After centrifuging the solution again, supernates in the container body 1 were removed to a new tube, chloroform was added to the supernates, and the supernates were further centrifuged through the refrigerated centrifuge. After centrifuging the supernates, the aqueous phase in the tube was removed to another tube, so that desired nucleic acids were extracted. Fig. 56 shows the results of the analysis of the nucleic acids by electrophoresis.

Electrophoresis was performed as follows: A part of the extracted nucleic acids was collected, and the collected part of the nucleic acids was made to dissolve in a lysis buffer in which formalin and formamide were mixed. Also, a 0.02M MOPS buffer solution containing 0.5µg / ml EtBr was used as a running buffer. The samples were set in a prepared agarose gel, and electrophoresis was performed with a constant voltage of 100 V for about 30 minutes. After performing electrophoresis, the nucleic acids were exposed to an UV lamp, and the nucleic acids that emitted light were photographed. As a result, as shown in Fig. 56, the bands of the nucleic acids which were peculiar to the artificial stool were identified.

### Test Example 4

By performing the same procedure as that of the test example 3, the intermediary unit 4 was removed from the container body 1 which contained the artificial stool E in an air-tight state. And then, the container body 1 was mounted in a MagNALyser (registered trademark) which was made by The Roche Co. and was a general-purpose apparatus for physical and chemical analysis, and cells in the specimen were automatically homogenized and were centrifuged. After the centrifugation, supernates in the container body 1 were removed, a nucleic acid extraction reagent (ISOGEN, TRIzol, or the like) was added to the specimen, the nucleic acid extraction reagent was sufficiently stirred together with the specimen, and the specimen was centrifuged. After the centrifugation, supernates in the container body 1 were removed to a new tube, chloroform was added to the supernates, and the supernates were centrifuged. After the centrifugation, the aqueous phase in the tube was removed to another tube, so that desired nucleic acids were extracted.

A part of the extracted nucleic acids was collected, and the collected part of the nucleic acids was made to dissolve in a lysis buffer in which formalin and formamide were mixed. Also, a 0.02M MOPS buffer solution containing 0.5µg / ml EtBr was used as a running buffer. The samples were set in a prepared agarose gel, and electrophoresis was performed with a constant voltage of 100 V for about 30 minutes. After performing electrophoresis, the nucleic acids were exposed to an UV lamp, and the nucleic acids that emitted light were photographed. As a result, as shown in Fig. 57, the bands of the nucleic acids which were peculiar to the artificial stool could be identified.

The containers for specimen of the present invention were explained hereinbefore. However, a container for specimen of the present invention is not limited to the characteristics which are described in claims, and a container for specimen of the present invention have also the following characteristics.

(1) A container for specimen, characterized in that: the container for specimen comprises a container body one end of which is open and which can hold a liquid for dispersing or preserving a specimen and is formed in the shape of a tube, a specimen-picking rod for picking a specimen, a cover member which connects with the rear end portion of the specimen-picking rod, and an intermediary unit which connects the container body with the cover member; the intermediary unit includes a connection portion the both ends of which are open, the one end of which can be removably fitted to the open end of the container body, the other end of which can be removably fitted to the cover member, and which is formed in the shape of a tube, and a penetration wall which is provided on the inside of the connection portion, includes a through hole that can be penetrated by the specimen-picking rod and which separates a space into two divisions on the upper and lower sides in the state of the specimen-picking rod penetrating through the through hole; the specimen-picking rod includes a concave picking portion in the front end of the specimen-picking rod; the intermediary unit includes a convex removing portion which is placed on the bottom of the intermediary unit and can be inserted into the concave picking portion; and the concave picking portion and the convex removing portion are formed in such a way that the concave picking portion and the convex removing portion can be fitted to each other and be separated from each other from the state of the concave picking portion fitted to the convex removing portion, wherein a plurality of knurlings are formed on the surface of the convex removing portion

(2) A container for specimen according to the above (1), characterized in that the container for specimen includes a plurality of slits which link the lateral side of the concave picking portion and the external peripheral surface of the specimen-picking rod.

(3) A container for specimen according to the above (1) or (2), characterized in that the container for specimen includes a connection means for connecting the intermediary unit with the convex picking portion.

(4) A container for specimen, characterized in that: the container for specimen comprises a container body one end of which is open and which can hold a liquid for dispersing or preserving a specimen and is formed in the shape of a tube, a specimen-picking rod for picking a specimen, a cover member which connects with the rear end portion of the specimen-picking rod, and an intermediary unit which connects the container body with the cover member; and the intermediary unit includes a connection portion the both ends of which are open, the one end of which can be removably fitted to the open end of the container body, the other end of which can be removably fitted to the cover member, and which is formed in the shape of a tube, and a penetration wall which is provided on the inside of the connection portion, includes a through hole that can be penetrated by the specimen-picking rod and which separates a space into two divisions on the upper and lower sides in the state of the specimen-picking rod penetrating through the through hole, wherein the specimen-picking rod includes a specimen-picking groove which is placed on the surface of the front end of the specimen-picking rod and has a thread structure.

(5) A container for specimen according to the above (4), characterized in that the container for specimen includes a cylinder-shaped specimen-pushing portion the internal surface of which is provided with a thread structure that makes it possible to threadedly engage the specimen-pushing portion with the specimen-picking groove, and which is fixed on the bottom of the intermediary unit.

### Industrial Applicability

A container for specimen of the present invention is applicable to fields which require extraction of nucleic acids from a picked specimen using a general-purpose apparatus for physical and chemical analysis like a centrifuge and analysis of the extracted nucleic acids, such as genetic test with stool for example.

## Claims

1. A container for specimen, **characterized in that** the container for specimen comprises
a container body one end of which is open, and which can hold a liquid for dispersing or preserving a specimen and is formed in the shape of a tube so that the container body can be mounted in a general-purpose apparatus for physical and chemical analysis,
a specimen-picking rod for picking a specimen,
a cover member which connects with the rear end portion of the specimen-picking rod, and
an intermediary unit which connects the container body with the cover member,
wherein the intermediary unit includes
a connection portion the both ends of which are open, the one end of which can be removably fitted to the open end of the container body, the other end of which can be removably fitted to the cover member, and which is formed in the shape of a tube, and
a penetration wall which is provided on the inside of the connection portion, includes a through hole that can be penetrated by the specimen-picking rod, and separates a space into two divisions on the upper and lower sides in the state of the specimen-picking rod penetrating through the through hole.

2. A container for specimen according to claim 1, **characterized in that** the specimen-picking rod includes a separation portion which can open and close at least in the front end of the specimen-picking rod and round almost one part of the specimen-picking rod and which holds a set amount of specimen when the separation portion is closed, and the specimen-picking rod slides on the through hole to penetrate through the through hole while the separation portion is closed.

3. A container for specimen according to claim 1, **characterized in that** the specimen-picking rod includes a forked portion which can open and close in at least the front end of the specimen-picking rod and holds a set amount of a specimen with the specimen fragmented when the fork portion is closed, and the specimen-picking rod slides on the through hole to penetrate through the through hole while the fork portion is closed.

4. A container for specimen according to one of claims 1 to 3, **characterized in that** the penetration wall is made of elastic material.

5. A container for specimen according to one of claims 1 to 3, **characterized in that** the penetration wall is provided with an elastic member in the vicinity of the through hole.

6. A container for specimen according to claim 5, **characterized in that** the penetration wall includes an annular groove which is formed on the surface forming the through hole, and an annular elastic member which is fitted to the annular groove and the inside diameter of which is smaller than the diameter of the through hole.

7. A container for specimen according to claim 5, **characterized in that** the penetration wall is provided with an elastic member which is given a radial slash that passes through the center of the through hole, covers the through hole, and deforms to come into close contact with the specimen-picking rod when the specimen-picking rod is inserted into the through hole.

8. A container for specimen according to claim 1, **characterized in that** the penetration wall is provided with a plurality of annular grooves which are formed on the surface forming the through hole and along the direction of the penetration.

9. A container for specimen according to claim 8, **characterized in that** the cross sectional shapes of a plurality of the annular grooves which are formed on the surface forming the through hole of the penetration wall are formed in the shape of one of the letter "U", the lette "V", and an arc.

10. A container for specimen according to one of claims 1, and 3 to 9,
**characterized in that** the specimen-picking rod includes a concave picking portion in the front end of the specimen-picking rod and the container body includes a convex removing portion which is placed on the bottom of the container body and can be inserted into the concave picking portion.

11. A container for specimen according to claim 10, **characterized in that** the concave picking portion and the convex removing portion are formed in such a way that the concave picking portion and the convex removing portion can be fitted to each other and be separated from each other from the state of the concave picking portion fitted to the convex removing portion.

12. A container for specimen according to claim 10 or 11, **characterized in that** the specimen-picking rod includes a path which links the concave picking portion and the external peripheral surface of the specimen-picking rod and a specimen passes through the path to be extruded to the outside when the convex removing portion is inserted into the concave picking portion.

13. A container for specimen according to claim 12, **characterized in that** the specimen-picking rod includes a plurality of slits which link the lateral side of the concave picking portion and the external peripheral surface of the specimen-picking rod.

14. A container for specimen according to claim 12, **characterized in that** the specimen-picking rod includes a plurality of extrusion holes which link the bottom surface of the concave picking portion and the external peripheral surface of the specimen-picking rod.

15. A container for specimen according to claim 1, **characterized in that** the specimen-picking rod includes a picking portion which is placed on the external peripheral surface of the front end of the specimen-picking rod and is composed of a lattice-shaped groove.

16. A container for specimen according to claim 1, **characterized in that** the specimen-picking rod includes a picking portion which is placed at the front end of the specimen-picking rod and is shaped like a brush.

17. A container for specimen according to claim 1, **characterized in that** the specimen-picking rod includes
an absorbing portion which is placed on the front end of the specimen-picking rod and is spongy,
a tube member which connects with the absorbing portion, and
a rod member which moves inside the tube member, the front end portion of which can push the absorbing portion, and the other end of which connects with the cover member.

18. A container for specimen according to claim 3, **characterized in that** at least one branching portion of the forked portion includes at least one specimen-holding portion which is provided with a specimen-introducing inlet that is formed on the side on which the one branching portion touches the other branching portion of the forked portion when the forked portion is closed and a specimen-exhausting outlet that is formed on the side opposite to the side on which the specimen-introducing inlet is formed.

19. A container for specimen according to claim 3, **characterized in that**
the front end of one branching portion of the forked portion includes at least one groove which is formed by placing at least two thin plate-shaped plane portions along the direction of the forked portion opening or closing, and
the front end of the other branching portion of the forked portion includes a shield portion which is composed of a thin plate-shaped plane portion that protrudes toward the one-branching-portion side so that the shield portion covers the opening side of the groove along the direction of the forked portion opening or closing when the forked portion is closed.

20. A container for specimen according to claim 3, **characterized in that**
the front end of one branching portion of the forked portion includes at least one groove which is formed by placing at least two thin plate-shaped plane portions along the direction of the forked portion opening or closing,
the other branching portion of the forked portion includes a specimen-holding portion which is provided with a specimen-introducing inlet that is formed on the side on which the one branching portion touches the other branching portion of the forked portion when the forked portion is closed and a specimen-exhausting outlet that is formed on the side opposite to the side on which the specimen-introducing inlet is formed, and
the front end of the other branching portion of the forked portion includes a shield portion which is composed of a thin plate-shaped plane portion that protrudes toward the one-branching-portion side so that the shield portion covers the opening side of the groove along the direction of the forked portion opening or closing when the forked portion is closed.

21. A container for specimen according to claim 21, **characterized in that**, in the at least one groove, at least one of the thin plate-shaped plane portions which form the groove protrudes toward the the-other-branching-portion side so as to enter the specimen-holding portion when the forked portion is closed.

22. A container for specimen according to one of claims 19 to 21, **characterized in that** the thin plate-shaped plane portions which form the groove become thinner and thinner towards the side on which the one branching portion of the forked portion touches the other branching portion of the forked portion when the forked portion is closed.

23. A container for specimen according to one of claims 3, and 18 to 22, **characterized in that**
the forked portion of the specimen-picking rod is formed so as to be opened with elastic force biased, and
the specimen-picking rod is provided with an annular member which is placed on the outer circumference of the specimen-picking rod, can move in the longitudinal direction, is moved towards the front end by a predetermined distance to make the forked portion close, and is moved towards the rear end by a predetermined distance to release the forked portion that is in a closed state.

24. A container for specimen according to claim 23, **characterized in that** the specimen-picking rod includes an annular member-fixing means for fixing the annular member at a predetermined position at which the forked portion is opened.

25. A container for specimen according to one of claims 3, and 18 to 22, **characterized in that**
the specimen-picking rod includes a flange at the rear end of the specimen-picking rod, the trunk portion of the specimen-picking rod which runs from the base of the forked portion to the rear end is formed as a hollow part, and, in addition, the specimen-picking rod includes a pair of guide grooves including a groove with a T-shaped cross section which is provided for each of the branching portions approximately along the longitudinal direction of the specimen-picking rod on the side on which the one branching portion of the forked portion touches the other branching portion of the forked portion in closing the forked portion and a second groove which continues to the one edge of the T-shaped groove,
the cover member includes a holding portion by which the flange at the rear end of the specimen-picking rod is rotatably held, a shaft-shaped male screw which is fixed in the middle of the cover member and runs towards the front end of the specimen-picking rod with the male screw having a predetermined length, a female screw into which the male screw is screwed and the diameter of which has a size that enables the second grooves of the both guide grooves to hold the female screw when the male screw is screwed into the female screw up to a predetermined position in the male screw and the forked portion is closed, and T-shaped portions which are provided on the external peripheral surface of the female screw with the T-shaped portions opposite to each other and are freely fitted to the T-shaped grooves of the guide grooves in the forked portion respectively, and
the rotation of the cover member relative to the specimen-picking rod makes the forked portion open and close.

26. A container for specimen according to claim 25, **characterized in that** the pair of the guide grooves is provided so as to incline relative to the longitudinal direction of the specimen-picking rod so that the T-shaped grooves and the bottom surfaces of the second grooves become shallower and shallower towards the base of the forked portion.

27. A container for specimen according to one of claims 3, and 18 to 22,
**characterized in that**
the specimen-picking rod is connected with at least one headed pin-shaped connection member at the middle portion of the specimen-picking rod and can open and close with the shaft of the headed pin-shaped connection member as a fulcrum, and the portion of the specimen-picking rod which runs from the front end of the specimen-picking rod to the fulcrum is composed of two members which form the forked portion,
a first member of the two members includes a first nick portion which runs from the rear end to the vicinity of the fulcrum,
a second member of the two members includes a second nick portion which runs from the rear end to the vicinity of the fulcrum and into which the portion of the first member running from the rear end of the first member to the vicinity of the fulcrum can be slidably fitted,
the cover member includes
a shaft-shaped supporting member which is fixed in the middle of the cover member, has a diameter and a length that enable the supporting portion to be slidably fitted into the first nick portion of the first member, and includes an annular groove that can hold the head of the headed pin-shaped connection member connecting the two members in the circumferential direction, and
a swirl groove which is formed in the internal surface of the cover member in such a way that the rear ends of the two members are slidably fitted to the swirl groove and is formed in the shape of a swirl converging to the center of the cover member.

28. A container for specimen according to one of claims 10 to 14, **characterized in that** the container for specimen includes a connection means which connects the intermediary unit with the convex picking portion.

29. A container for specimen according to claim 28, **characterized in that**
the connection means includes
an engaging member which is fixed on the inside of the connection portion, and
a frame member which is formed in the shape of a tube so as to be capable of being held inside the container body, the bottom portion of which the convex picking portion is fixed on, and the upper portion of which can engage with the engaging member.

30. A container for specimen according to one of claims 1, 4 to 17, 28, and 29, **characterized in that** threads are formed in the internal peripheral portion of the one end of the connection portion in the intermediary unit and in the external peripheral portion of the open end of the container body, and the open end of the container body is screwed into the one end of the connection portion of the intermediary unit through the threads.

31. A container for specimen according to one of claims 1, 4 to 17, 28, and 29,
**characterized in that**
the external peripheral portion of the one end of the connection portion in the intermediary unit is provided with at least one annular convex portion which is formed along the penetration direction, an annular stopper portion the diameter of which is larger than the internal peripheral portion of the open end of the container body, and an O-ring which is placed on the container-body side of the stopper member, and
the internal peripheral portion of the open end of the container body is provided with an annular concave portion which can be fitted to the annular convex portion.

32. A container for specimen according to one of claims 1, 4 to 17, 28, and 29,
**characterized in that**
the edge portion of the one end of the connection portion in the intermediary unit is provided with an annular groove which is formed so as to be capable of being fitted to the open end of the container body, an O-ring which is placed on the bottom of the annular groove, and a plurality of opening portions which are provided on the circumference of the connection portion of the intermediary unit so as to link the annular groove and the external peripheral portion of the one end of the connection portion of the intermediary unit, and
the external peripheral portion of the open end of the container body is provided with a plurality of catch portions which can be inserted into the opening portions of the connection portion of the intermediary unit and are formed in such a way that the catch portions break off at the bases when the catch portions are inserted into the opening portions and force is applied in a set direction crossing the insertion direction.

33. A container for specimen according to one of claims 1, 4 to 17, 28, and 29,
**characterized in that**
the edge portion of the one end of the connection portion in the intermediary unit is provided with an annular groove which is formed so as to be capable of being fitted to the open end of the container body, an O-ring which is placed on the bottom of the annular groove, and a plurality of opening portions which are provided on the circumference of the connection portion of the intermediary unit so as to link the annular groove and the external peripheral portion of the one end of the connection portion of the intermediary unit, and
the external peripheral portion of the open end of the container body is provided with a plurality of catch portions which can be inserted into the opening portions of the connection portion of the intermediary unit and are formed in such a way that the catch portions have sloped surfaces so that the catch portions come out of the opening portions when force is applied so that the intermediary unit is moved in the direction opposite to the direction toward the container body with the catch portions inserted into the opening portions.

34. A container for specimen according to one of claim 4 and claims 10 to 14 which are depend on claim 4, or one of claims 28, 29, and 30 to 33, **characterized in that**
the connection portion of the intermediary unit, the cover member, and the container body are made of elastic material,
the elastic modulus of the connection portion of the intermediary unit is smaller than those of the penetration wall and the cover member and is larger than that of the container body, and
the elastic modulus of the cover member is smaller than that of the penetration wall.

35. A container for specimen according to one of claim 4 and claims 10 to 14 which are depend on claim 4, or one of claims 28, 29, and 30 to 33, **characterized in that**
the connection portion of the intermediary unit, the cover member, and the container body are made of elastic material, and
the elastic modulus of the connection portion of the intermediary unit is smaller than that of the penetration wall and is larger than those of the cover member and the container body.

36. A container for specimen according to one of claims 1, 4 to 16, 17, and 28 to 35, **characterized in that** threads are provided on the external peripheral portion of the other end of the connection portion of the intermediary unit and on the internal peripheral portion of the cover member, and the other end of the connection portion of the intermediary unit is screwed into the container body through the threads.

37. A container for specimen according to claim 36, **characterized in that** torque by which the other end of the connection portion of the intermediary unit is screwed into the cover member is smaller than torque by which the open end of the container body is screwed into the one end of the connection portion of the intermediary unit.

38. A container for specimen according to one of claims 1, 4 to 16, 17, and 28 to 37, **characterized in that** the specimen-picking rod is formed so that the specimen-picking rod can be removably fitted to the cover member.

39. A container for specimen according to one of claim 36 which is depend on claim 30, claim 37, claim 38 which is depend on claim 36 that is depend on claim 30 or on claim 37, and claim 38, **characterized in that** the threads are formed in the internal peripheral portion of the one end of the connection portion of the intermediary unit, in the external peripheral portion of the open end of the container body, in the external peripheral portion of the other end of the connection portion of the intermediary unit, and in the internal peripheral portion of the cover member respectively in such a way that the opening and closing directions in rotating the intermediary unit in a set direction through the threads that are formed in the internal peripheral portion of the one end of the connection portion of the intermediary unit and in the external peripheral portion of the open end of the container body are opposite to the opening and closing directions in rotating the intermediary unit in a set direction through the threads that are formed in the external peripheral potion of the other end of the connection portion of the intermediary unit and in the internal peripheral portion of the cover member, respectively.

40. A container for specimen according to one of claim 36 which is depend on claim 30, claim 37, claim 38 which is depend on claim 36 that is depend on claim 30 or on claim 37, and claim 39, **characterized in that** the cover member is provided with a grasping portion.

41. A container for specimen according to one of claims 1, 4 to 16, 17, and 28 to 40, **characterized in that**
the outer diameter of the cover member is larger than that of the connection portion of the intermediary unit, and
the outer diameter of the connection portion of the intermediary unit is larger than that of the container body.

42. A container for specimen according to one of claims 1, 4 to 16, 17, and 28 to 41,
**characterized in that** the cover member is different from the connection portion of the intermediary unit in material.

43. A container for specimen according to one of claims 1, 4 to 16, 17, and 28 to 42, **characterized in that** the container body is different from the connection portion of the intermediary unit in material.

44. A container for specimen according to claim 27, **characterized in that** the internal surface of the cover member which is provided with the swirl groove is formed in the shape of a cone.

45. A container for specimen according to one of claims 3, and 18 to 22,
**characterized in that**
the internal surface of the cover member is formed in the shape of a cone and is provided with a groove which is formed in the shape of a spiral converging to the center of the cover member and has a T-shaped cross section,
the specimen-picking rod is connected with at least one headed pin-shaped connection member at the middle portion of the specimen-picking rod and can open and close with the shaft of the headed pin-shaped connection member as a fulcrum and the portion of the specimen-picking rod which runs from the front end of the specimen-picking rod to the fulcrum is composed of two members which form the forked portion,
the two members are formed in the shapes that enable the two members to overlap with each other over the range from the rear end to the vicinity of the fulcrum, the inside surfaces of the forked portion are formed in the shapes that enable the inside surfaces of the forked portion to abut each other, and the rear ends of the two members are provided with T-shaped portions which are freely fitted to the T-shaped groove of the cover member,
and
the rotation of the cover member relative to the specimen-picking rod makes the forked portion open and close.

46. A container for specimen, **characterized in that**
the container for specimen comprises
a container body one end of which is open, and which can hold a liquid for dispersing or preserving a specimen and is formed in the shape of a tube,
a specimen-picking rod for picking a specimen,
a cover member which connects with the rear end portion of the specimen-picking rod, and
an intermediary unit which connects the container body with the cover member, and
the intermediary unit includes
a connection portion the both ends of which are open, the one end of which can be removably fitted to the open end of the container body, the other end of which can be removably fitted to the cover member, and which is formed in the shape of a tube, and
a penetration wall which is provided on the inside of the connection portion, includes a through hole that can be penetrated by the specimen-picking rod, and separates a space into two divisions on the upper and lower sides in the state of the specimen-picking rod penetrating through the through hole,
wherein
the specimen-picking rod includes a plurality of vertical divisions which can open and close at least on the front end of the specimen-picking rod and hold a set amount of specimen when the vertical divisions are in a closed state,
a plurality of the vertical divisions are formed in such a way that at least a part of each of the vertical divisions expands towards the outside more and more towards the front end with the parts of the vertical divisions curving, and
the specimen-picking rod slides on the through hole to penetrate through the through hole with a plurality of the vertical divisions closed.

47. A container for specimen according to claim 46, **characterized in that** the front end of each of a plurality of the vertical divisions bends inward so that the vertical divisions can hold a set amount of specimen when the vertical divisions are in a closed state.

48. A container for specimen according to claim 46 or 47, **characterized in that**
the specimen-picking rod includes a tube portion which connects with the cover member and a cylinder portion which is inserted into the tube portion, and
a plurality of the vertical divisions are placed on the front end of the cylinder portion respectively and are formed so as to expand towards the outside more and more towards the front end with the vertical divisions curving when the vertical divisions protrude from the tube portion towards the outside. (Claim 17 of the latter application)

49. A container for specimen according to one of claims 46 to 48, **characterized in that** the specimen-picking rod is formed in such a way that a plurality of the vertical divisions can slide with the secrecy kept when the vertical divisions are contained in the tube portion.

50. A container for specimen according to claim 48 or 49, **characterized in that** the specimen-picking rod includes a reagent-holding portion which is placed in the cylinder portion.

51. A container for specimen according to one of claims 48 to 50, **characterized in that** the cylinder portion is composed of a rear end portion, a front end portion the front end of which is provided with a plurality of the vertical divisions, and a support portion which connects the rear end portion and the front end portion.

52. A container for specimen according to claim 51 which is depend on claim 50,
**characterized in that**
the support portion is formed in such a way that the support portion has a small diameter,
the reagent-holding portion is formed by the space which is surrounded by the tube portion, the rear end portion of the cylinder portion, the support portion of the cylinder portion, and the front end portion of the cylinder portion,
the front end portion of the cylinder portion includes a plurality of the vertical divisions and a base portion which holds a plurality of the vertical divisions, and
the base portion plays a role as a stopper for the reagent-holding portion for the tube portion.

53. A container for specimen according to claim 51 which is depend on claim 50,
**characterized in that**
the support portion is formed as a hollow part,
the reagent-holding portion is formed by the space which is surrounded by the rear end portion of the cylinder portion, the hollow connection portion of the cylinder portion, and the front end portion of the cylinder portion, and
a part of the hollow connection portion is provided with an opening for exhausting a reagent.

54. A container for specimen according to one of claim 51 which is depend on claim 50, and claims 52 and 53, **characterized in that** the rear end portion of the cylinder portion is provided with a stopper portion which is formed in such a way that the stopper portion restrains the movement of the cylinder portion towards the front end in the tube portion in order to keep the air-tightness of the reagent-holding portion and the restraint of the movement of the cylinder portion can be released by applying a predetermined amount or more of pressing force to the stopper portion towards the front end.

55. A container for specimen according to claim 54, **characterized in that**
the container for specimen includes a removable outer cover which is placed on the outside of the intermediary unit and on the cover-member side with the intermediary unit connecting the container body with the cover member,
the intermediary unit includes a first thread portion which is provided on the external peripheral portion of the intermediary unit on the cover-member side and can be threadedly engaged with the outer cover, and
the outer cover includes a second thread portion which is provided on the internal peripheral portion of the outer cover and can be threadedly engaged with the intermediary unit, and a restraint-releasing portion which applies a predetermined amount or more of pressing force to the stopper portion to release restraint of the movement of the cylinder portion towards the front end in the tube portion by the stopper portion when the second thread portion of the outer cover is threadedly engaged with the first thread portion of the intermediary unit.
